(19)

Europäisches Patentamt

European Patent Office

Office européen des brevets

(11) **EP 4 545 584 A1**

(12) **EUROPEAN PATENT APPLICATION**
published in accordance with Art. 153(4) EPC

(43) Date of publication:
**30.04.2025  Bulletin 2025/18**

(21) Application number: **23831220.1**

(22) Date of filing: **21.06.2023**

(51) International Patent Classification (IPC):
*C08G 63/88* (2006.01)     *C07B 61/00* (2006.01)
*C07C 27/02* (2006.01)     *C07C 29/09* (2006.01)
*C07C 31/20* (2006.01)     *C07C 51/09* (2006.01)
*C07C 63/26* (2006.01)     *C08G 63/16* (2006.01)
*C08G 63/183* (2006.01)    *C08J 11/14* (2006.01)
*C08J 11/16* (2006.01)     *C08K 3/22* (2006.01)
*C08K 3/26* (2006.01)      *C08L 67/02* (2006.01)

(52) Cooperative Patent Classification (CPC):
**C07B 61/00; C07C 29/00; C07C 29/09;
C07C 31/20; C07C 51/09; C07C 63/26;
C08G 63/16; C08G 63/183; C08G 63/88;
C08J 11/14; C08J 11/16; C08K 3/22; C08K 3/26;
C08L 67/02;** Y02W 30/62

(86) International application number:
**PCT/JP2023/022870**

(87) International publication number:
**WO 2024/004774 (04.01.2024 Gazette 2024/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  27.06.2022   JP 2022102942
        15.12.2022   JP 2022200393
        15.12.2022   JP 2022200394
        04.04.2023   JP 2023060578
        27.04.2023   JP 2023072973

(71) Applicant: **Toray Industries, Inc.**
**Tokyo 103-8666 (JP)**

(72) Inventors:
• **NISHIMURA, Mihoko**
**Nagoya-shi, Aichi 455-8502 (JP)**

• **YAMASHITA, Kohei**
**Nagoya-shi, Aichi 455-8502 (JP)**
• **NAGAHARA, Takashi**
**Nagoya-shi, Aichi 455-8502 (JP)**
• **YOSHIDOMI, Shinichiro**
**Nagoya-shi, Aichi 455-8502 (JP)**
• **YOSHIKAWA, Takahiro**
**Nagoya-shi, Aichi 455-8502 (JP)**
• **KATO, Koya**
**Nagoya-shi, Aichi 455-8502 (JP)**
• **UTAZAKI, Kenichi**
**Nagoya-shi, Aichi 455-8502 (JP)**

(74) Representative: **Mewburn Ellis LLP**
**Aurora Building**
**Counterslip**
**Bristol BS1 6BX (GB)**

(54) **RECYCLED MONOMER, RECYCLED MONOMER PRODUCTION METHOD, AND RECYCLED MONOMER PRODUCTION DEVICE**

(57)     An object of a recycled monomer and a method of producing the same according to the present invention is to provide a method that can make use of the advantages of high-temperature and high-pressure water, inhibit the overreaction of a product obtained through depolymerization, and generate a recycled monomer such as a dicarboxylic acid or a diol with a high yield. In one preferable aspect of the method of producing a recycled monomer according to the present invention, i.e., the method intended to achieve the object, a polymer is a thermoplastic polyester (A), and X and X·Y·Z which is the product of X, Y, and Z satisfy the following (I) and (II) simultaneously, assuming that the melting point of the thermoplastic polyester (A) is Mp°C, as measured using

**(Cont. next page)**

EP 4 545 584 A1

a differential scanning calorimeter, that a reaction temperature is X°C, that a reaction time is Y minute(s), and that the mass ratio of water to the thermoplastic polyester (A) is Z:1.

$$(I) \ \mathrm{Mp} \leq X < 300$$

$$(II) \ 2000 \leq X \cdot Y \cdot Z \leq 20000$$

Fig. 1

## Description

Technical Field

The present invention relates to a chemical recycling technology for a polymer and is to realize both recycling of resources and a decrease in greenhouse gas emissions.

Background Art

In recent years, the problem of plastic in the ocean has acted as a trigger, attracting more attention to global environmental problems. There is a spreading recognition that it is necessary to build up a sustainable society. The global environment problems include global warming and others such as resource depletion and water shortage. Many of the problems are due to an increase in the amount of resource consumption and an increase in the greenhouse gas emissions, the increases being due to use of fossil fuel and rapid development of industry since the Industrial Revolution. Accordingly, to build up a sustainable society, a technology related to recycling fossil resources, such as plastics, and decreasing the greenhouse gas emissions is increasingly important.

There is a known technology in which thermoplastic polyesters, such as fibers, films, bottles, molded articles, or engineering plastics, which are used in large amounts in various fields are depolymerized and converted back to monomers to be recycled. Such a technology is, for example, the following: a methanolysis method in which methanol is allowed to react in the presence of a catalyst such as zinc acetate, and a methyl ester monomer is recovered; or a glycolysis method in which ethylene glycol is allowed to react in the presence of a catalyst such as sodium carbonate, and an oligomer is recovered. Commercial facilities in which polyesters are depolymerized using these technologies are already operating.

In addition, there is a method in which a thermoplastic polyester is depolymerized without use of an organic solvent such as methanol or ethylene glycol. Such a method is disclosed, in which raw material monomers-diol and dicarboxylic acid-are recovered by bringing a polyester into contact with high-temperature and high-pressure water (see, for example, Patent Literature 1 to 3). A method of depolymerization only with water needs no catalyst, and furthermore, uses no petroleum-derived organic solvent, giving an advantage of an eco-friendly recycling method.

A method is disclosed in which, in this depolymerization with high-temperature and high-pressure water, alkali is added to water, and the recovery rate of dicarboxylic acid that is a raw material monomer is enhanced (for example, Patent Literature 4 to 7). However, the depolymerization needs to be performed for a long time, or the depolymerizing temperature needs to be extremely high.

Furthermore, the plastic material is often composed of multiple polymers to provide characteristics necessary in accordance with an application. Materials composed of a single polymer are limited. To recycle a material composed of multiple polymers, a study is being made in which, before depolymerization, the material is separated into single polymers, and then, each polymer is depolymerized to regenerate a monomer. However, a material difficult to separate has been requiring development of a technology of recycling such a material.

Against the above-described background, the capability to establish a method in which multiple polymers are simultaneously depolymerized makes a pretreatment process unnecessary, and in addition, makes it possible to build up a process of recycling a material that is composed of multiple polymers, and is hitherto impossible to separate into single polymers.

Patent Literature 8 describes a depolymerizing method in which a material selected from a polyester, polyamide, and polycarbonate is hydrolyzed. However, the Literature does not mention that multiple polymers are depolymerized simultaneously.

Non-Patent Literature 1 mentions the hydrolysis of PC (polycarbonate)/PBT (polybutylene terephthalate), PC/PET (polyethylene terephthalate), and PBT/PET, but the reaction temperature was high, and it was difficult to obtain a recycled monomer with a high yield.

On the other hand, a plastic recycling apparatus is disclosed, for example, an apparatus with which an unsaturated polyester resin molded article and subcritical water are brought into contact with each other in batch treatment at a temperature of 180°C to 250°C in the range of weight ratio from 1:6 to 1:9 between the unsaturated polyester resin molded article and the subcritical water, whereby monomers are produced (see Patent Literature 9).

In addition, an apparatus and a method are disclosed, in which waste polyethylene terephthalate (PET) in a molten state is brought into contact with high-temperature and high-pressure water to be hydrolyzed continuously, whereby raw material monomers are recovered (see Patent Literature 10).

Citation List

Patent Literature

Patent Literature 1: JP2000-309663A
Patent Literature 2: JP2007-332361A
Patent Literature 3: JP06-072922A
Patent Literature 4: JP11-502870A
Patent Literature 5: JP08-302061A
Patent Literature 6: WO2007/148353A1
Patent Literature 7: WO2004/041917A1
Patent Literature 8: US4605762B
Patent Literature 9: JP2012-106244A
Patent Literature 10: JP09-077905A

Non-Patent Literature

Non-Patent Literature 1: Waste Management, 68 (2017) 24-31

Summary of Invention

Technical Problem

The thermoplastic polyester depolymerizing method disclosed in Patent Literature 1 affords ethylene glycol with a yield of 92% and terephthalic acid with a yield of 96%, which are high yields. In the polyester depolymerizing method disclosed, however, a thermoplastic polyester is allowed to react, at a high temperature of 300°C to 350°C, with an approximately 6-fold amount of water having a specific heat capacity of 4.2 kJ/kg·K and a heat of vaporization of 2,250 kJ/kg, which are very high. The method entails high energy cost in the depolymerization and the recovery of ethylene glycol from an aqueous solution of low-concentration ethylene glycol.

In addition, according to Patent Literature 2, terephthalic acid is added during the hydrolysis of polyethylene terephthalate, and ethylene glycol and terephthalic acid are obtained with a yield of 100% at 300°C in 10 minutes. However, when the reaction temperature is changed to 265°C, the yields of ethylene glycol and terephthalic acid are decreased to 56%, and the yields are further decreased without the addition of terephthalic acid. Furthermore, water is used in a large amount, approximately 9 times the amount of thermoplastic polyester, and thus, has a problem of high energy cost.

On the other hand, the polyethylene terephthalate depolymerizing method disclosed in Patent Literature 3 uses only water at a reaction temperature of 280°C, a relatively low temperature, but affords a depolymerized product with a high yield: an ethylene glycol yield of 90% and a terephthalic acid yield of 96%. However, the method takes 6 hours for the depolymerization, and cannot be said to be efficient. Furthermore, according to Patent Literature 3, waste PET is hydrogenated using a palladium/carbon catalyst at 1 wt% with respect to the waste PET in order to improve the color-tone deterioration due to coloring impurities having an unsaturated bond which are difficult to purify and remove. Actually, when the present inventors experimented depolymerization, using polyethylene terephthalate as a raw material, under the conditions the same as or similar to the conditions in the method described in Patent Literature 3 except that hydrogen was not added, the reaction mixture was colored light orange.

In light of the above-described conventional technologies, the present invention including a recycled monomer, a method of producing the recycled monomer, and the below-described first and the second preferable aspects are intended to provide a method that can make use of the advantages of high-temperature and high-pressure water, inhibit the overreaction of a product obtained through depolymerization, and generate a recycled monomer such as a dicarboxylic acid or a diol with a high yield.

In addition, the below-described third preferable aspect of the present invention is intended to provide a method that can produce a recycled monomer, particularly a dicarboxylic acid and a diol, with a high yield from a material that is composed of multiple polymers, and hitherto difficult to chemically recycle.

On the other hand, the depolymerizing apparatus disclosed in Patent Literature 9, in the form of a plastic recycling apparatus, uses a reactor of a batch type. Thus, in the case of bulky treatment, the capacity of the reactor is large because of the influence of preliminary preparation and the discharge cycle time, leading to an increase in the cost of an apparatus of producing a recycled monomer, and making it impracticable to obtain sufficient efficiency. On the other hand, in a case where the amount of subcritical water added to a resin molded article is decreased, the amount of the resin molded article treated is increased, thus enhancing the efficiency, but making it impracticable to obtain a sufficient recycled-monomer yield.

In addition, the method and the apparatus both for hydrolyzing waste PET which are disclosed in Patent Literature 10 are of a continuous type, and thus, can prevent the capacity of the reactor from being large as a batch type is, and can produce a recycled monomer with higher efficiency than a batch type. On the other hand, in a case where the amount of high-temperature and high-pressure water added to waste PET is decreased in the same manner as described above, it was impracticable to obtain a sufficient recycled-monomer yield.

In view of this, an apparatus of producing a recycled monomer according to the present invention is intended to provide an apparatus of producing a recycled monomer, the apparatus being capable of producing a recycled monomer with high efficiency and a high yield, using a continuous reactor.

Solution to Problem

To solve the above-described problems, the present invention has the following constitution.

1. A recycled monomer produced by bringing a composition containing a polymer into contact with subcritical water, wherein the recycled monomer is produced under a condition where X and X·Y·Z which is the product of X, Y, and Z satisfy the following (I) and (II) simultaneously, assuming that the melting point of the polymer in the composition is Mp °C, as measured using a differential scanning calorimeter, that a reaction temperature is X°C, that a reaction time is Y minute(s), and that the mass ratio of the water to the polymer is Z:1.

$$\text{(I) } Mp \leq X < 300$$

$$\text{(II) } 2000 \leq X \cdot Y \cdot Z \leq 20000$$

2. The recycled monomer according to 1 above, wherein the polymer is a thermoplastic polyester or a thermoplastic polyamide.

3. A recycled polymer obtained by repolymerizing the recycled monomer according to 1 above.

4. A clothing fiber, industrial fiber, film, sheet, automobile component, or electrical and electronic component produced using the recycled polymer according to 3 above.

5. A method of producing a recycled monomer, including a step of bringing a composition containing a polymer into contact with subcritical water.

6. The method of producing a recycled monomer according to 5 above, wherein the polymer is a thermoplastic polyester (A), and wherein X and X·Y·Z which is the product of X, Y, and Z satisfy the following (I) and (II) simultaneously, assuming that the melting point of the thermoplastic polyester (A) is Mp°C, as measured using a differential scanning calorimeter, that a reaction temperature is X°C, that a reaction time is Y minute(s), and that the mass ratio of water to the thermoplastic polyester (A) is Z:1.

$$\text{(I) } Mp \leq X < 300$$

$$\text{(II) } 2000 \leq X \cdot Y \cdot Z \leq 20000$$

7. The method of producing a recycled monomer according to 6 above, wherein the concentration of a diol in a reaction mixture obtained by bringing the composition containing the thermoplastic polyester (A) into contact with the subcritical water to hydrolyze the thermoplastic polyester (A) is 4.9 mass% or more, wherein the diol is derived from the thermoplastic polyester (A).

8. The method of producing a recycled monomer according to 6 above, wherein the concentration of a cyclic ether derived from the thermoplastic polyester (A) in a reaction mixture obtained by bringing the composition containing the thermoplastic polyester (A) into contact with the subcritical water to hydrolyze the thermoplastic polyester (A) is 3.0 mass% or more.

9. The method of producing a recycled monomer according to 6 above, wherein the mass ratio Z of the subcritical water to the thermoplastic polyester (A) is less than 6.

10. The method of producing a recycled monomer according to 6 above, wherein the thermoplastic polyester (A) contains, as a main component, at least one selected from polyethylene terephthalate, polypropylene terephthalate, polybutylene terephthalate, and copolymers thereof.

11. The method of producing a recycled monomer according to 6 above, wherein the thermoplastic polyester (A) is a waste containing at least a polyester.

12. The method of producing a recycled monomer according to 6 above,
wherein the recycled monomer is a dicarboxylic acid and/or a diol.

13. A method of producing a thermoplastic polyester using, as a raw material, the dicarboxylic acid and/or the diol obtained using the producing method according to 12 above.

14. A method of producing a thermoplastic polyester product, wherein the thermoplastic polyester obtained using the producing method according to 12 above is used as a raw material to produce a product containing the thermoplastic polyester in the form of a fiber, a film, or a molded article.

15. A method of producing a product of a composition containing a thermoplastic polyester, wherein the composition containing a thermoplastic polyester, obtained using the producing method according to 12 above, is used as a raw material to produce a product of the composition containing a thermoplastic polyester in the form of a fiber, a film, or a molded article.

16. The method of producing a recycled monomer according to 5 above,
wherein the polymer is the thermoplastic polyester (A), and wherein 0.01 to 4.0 parts by weight of an alkaline metal salt and/or an alkaline earth metal salt is/are blended with 100 parts by weight of the thermoplastic polyester (A).

17. The method of producing a recycled monomer according to 16 above,
wherein 100 to 1000 parts by weight of the subcritical water is blended with 100 parts by weight of the thermoplastic polyester (A).

18. The method of producing a recycled monomer according to 16 above,
wherein a temperature in the step of bringing about contact with the subcritical water is 250 to 350°C.

19. The method of producing a recycled monomer according to 16 above,
wherein a pressure in the step of bringing about contact with the subcritical water is 3 to 30 MPa.

20. The method of producing a recycled monomer according to 16 above,
wherein the alkaline metal salt and/or the alkaline earth metal salt is/are a metal hydroxide and/or a metal carbonate.

21. The method of producing a recycled monomer according to 16 above,
wherein the thermoplastic polyester (A) is at least one selected from polyethylene terephthalate, polypropylene terephthalate, polybutylene terephthalate, and copolymers thereof.

22. The method of producing a recycled monomer according to 16 above,
wherein the composition containing the thermoplastic polyester (A) contains at least one selected from antioxidants, heat-resistant agents, weather-resistant agents, mold release agents, and nucleating agents.

23. The method of producing a recycled monomer according to 16 above,
wherein the composition containing the thermoplastic polyester (A) is a waste.

24. The method of producing a recycled monomer according to 16 above,
wherein the recycled monomer is a dicarboxylic acid and/or a diol.

25. A method of producing a thermoplastic polyester using, as a raw material, a dicarboxylic acid and/or a diol obtained using the producing method according to 24 above.

26. A method of producing a composition containing a thermoplastic polyester, wherein at least one selected from antioxidants, weather-resistant agents, mold release agents, and nucleating agents is blended with the thermoplastic polyester obtained using the producing method according to 25 above.

27. A method of producing a thermoplastic polyester product, wherein the thermoplastic polyester obtained using the producing method according to 25 above is used as a raw material to produce a product containing the thermoplastic polyester in the form of a fiber, a film, or a molded article.

28. A method of producing a product of a composition containing a thermoplastic polyester, wherein the composition containing a thermoplastic polyester, obtained using the producing method according to 26 above, is used as a raw material to produce a product of the composition containing a thermoplastic polyester in the form of a fiber, a film, or a molded article.

29. The method of producing a recycled monomer according to 5 above,

   wherein the composition containing a polymer contains a polymer I and a polymer II,
   wherein the polymer I is a thermoplastic polyester (A),
   wherein the polymer II is a polymer selected from the group consisting of a thermoplastic polyester (B) having a melting point different from the melting point of the thermoplastic polyester (A), a polycarbonate, and a polyurethane, or a combination of these polymers,
   wherein the amount of the polymer II contained in the composition (the total amount of the thermoplastic polyester (B), the polycarbonate, and the polyurethane) is 1 to 100 parts by weight with respect to 100 parts by weight of the polymer I, and
   wherein the method includes a step of bringing the polymer I and the polymer II in the composition simultaneously into contact with the subcritical water of 250 to 350°C.

30. The method of producing a recycled monomer according to 5 above,

wherein the composition containing a polymer contains a polymer I and a polymer II,
wherein the polymer I is a thermoplastic polyester (A),
wherein the polymer II is a polymer selected from the group consisting of a thermoplastic polyester (B) having a melting point different from the melting point of the thermoplastic polyester (A), a polycarbonate, and a polyurethane, or a combination of these polymers,
wherein the amount of the polymer II contained in the composition (the total amount of the thermoplastic polyester (B), the polycarbonate, and the polyurethane) is 1 to 100 parts by weight with respect to 100 parts by weight of the polymer I, and
wherein the method includes a step of bringing the polymer I and the polymer II in the composition into contact with the subcritical water of 200 to 350°C to hydrolyze the polymer I and the polymer II simultaneously, under a condition where an alkaline component is blended.

31. The method of producing a recycled monomer according to 29 or 30 above, wherein the composition containing a polymer contains the composition containing the polymer I and the polymer II and/or contains a mixture of a composition containing the polymer I and a composition containing the polymer II.

32. The method of producing a recycled monomer according to 29 or 30 above, wherein 100 to 900 parts by weight of water is blended with 100 parts by weight of the polymer component in the composition containing the polymer.

33. The method of producing a recycled monomer according to 29 or 30 above, wherein a pressure in the step of bringing about contact with the subcritical water is 4.0 MPa to 30 MPa.

34. The method of producing a recycled monomer according to 29 above, wherein an alkaline component is further blended with the composition containing a polymer.

35. The method of producing a recycled monomer according to 29 or 30 above, wherein the thermoplastic polyester (A) is selected from polyethylene terephthalate, polypropylene terephthalate, polybutylene terephthalate, and copolymers thereof.

36. The method of producing a recycled monomer according to 29 or 30 above, wherein the thermoplastic polyester (B) is at least one selected from polyethylene terephthalate, polypropylene terephthalate, polybutylene terephthalate, and copolymers thereof, and has a melting point different from the melting point of the polyester (A).

37. The method of producing a recycled monomer according to 29 or 30 above, wherein a difference in the melting point between the thermoplastic polyester (A) and the thermoplastic polyester (B) is 10°C or more.

38. The method of producing a recycled monomer according to 29 or 30 above, wherein the composition containing a polymer is a waste.

39. The method of producing a recycled monomer according to 29 or 30 above, wherein the recycled monomer is a terephthalic acid and/or a diol.

40. A method of producing a thermoplastic polyester, wherein the dicarboxylic acid and/or the diol obtained using the producing method according to 29 or 30 above is/are used as a polymerization raw material to produce the thermoplastic polyester.

41. A method of producing a thermoplastic polyester product, wherein the thermoplastic polyester obtained using the producing method according to 40 above is used as a raw material to produce a thermoplastic polyester product in the form of a fiber, a film, or a molded article.

42. A method of producing a product of a composition containing a thermoplastic polyester, wherein the composition containing a thermoplastic polyester, obtained using the producing method according to 40 above, is used as a raw material to produce a product of the composition containing a thermoplastic polyester in the form of a fiber, a film, or a molded article.

43. An apparatus of producing a recycled monomer, including: means (L) for heating, pressurizing, and supplying a composition containing a polymer; means (M) for generating and supplying subcritical water; means (N) for mixing the composition containing a thermoplastic polymer and supplied from the means (L) and the subcritical water supplied from the means (M); and a continuous reactor (O) for conducting hydrolysis of the polymer.

44. The apparatus of producing a recycled monomer according to 43 above, including: a device (P) for monitoring the inside temperature of each of the upstream section and downstream section of the continuous reactor (O); and a temperature control mechanism (Q) for heating and/or cooling the continuous reactor (O);
wherein the temperature control mechanism (Q) is operated in accordance with the device (P) to control the inside temperature of the continuous reactor (O).

45. The apparatus of producing a recycled monomer according to 43 above, including a plurality of temperature control mechanisms (Q) that are installed, wherein the plurality of temperature control mechanisms (Q) are operated in accordance with the device (P).

46. The apparatus of producing a recycled monomer according to 44 or 45 above, wherein the temperature control mechanism (Q) is controlled in such a manner that the inside temperature of the downstream section of the continuous reactor (O) is 10°C or more lower than the inside temperature of the upstream section.

47. The apparatus of producing a recycled monomer according to 44 or 45 above, wherein the continuous reactor (O) is a tubular continuous reactor.

48. The apparatus of producing a recycled monomer according to 44 or 45 above, wherein the means (L) is an extruder.

49. The apparatus of producing a recycled monomer according to 44 or 45 above, wherein the means (N) is a static mixer.

50. The apparatus of producing a recycled monomer according to 44 or 45 above, wherein the composition containing a polymer is a waste of a resin molded article.

51. The apparatus of producing a recycled monomer according to 44 or 45 above, wherein the polymer is a thermoplastic polyamide or a thermoplastic polyester.

Advantageous Effects of Invention

According to the present invention of a recycled monomer, a method of producing the recycled monomer, and the below-described first preferable aspect of the present invention particularly in the depolymerization of a composition containing a thermoplastic polyester, energy consumption required for depolymerization and purification by distillation can be reduced, since the thermoplastic polyester can be depolymerized with high efficiency only through a short-time treatment with a small amount of water, and a reaction mixture containing a diol and a dicarboxylic acid at high concentrations is obtained; and thus, a method can be provided in which energy consumption required for depolymerization and distillation-based purification is small. Furthermore, the depolymerization condition at low temperature in a short time inhibits a second reaction of a diol and a dicarboxylic acid, giving, with a high yield, a diol and a dicarboxylic acid that are not colored.

In addition, the below-described second preferable aspect of the present invention makes it possible that an alkaline metal salt and/or an alkaline earth metal salt is/are added in specific amounts to hydrolyze a composition containing a thermoplastic polyester, thus inhibiting the overreaction of a product, and generating a dicarboxylic acid and/or a diol with a high yield.

Furthermore, the below-described third preferable aspect of the present invention can provide a method in which materials that are composed of multiple polymers, and hitherto difficult to chemically recycle are simultaneously hydrolyzed to produce a recycled monomer with a high yield. In particular, including a step of simultaneously hydrolyzing a composition with subcritical water in a specific temperature range makes it possible to produce a dicarboxylic acid and/or a diol with a high yield, in which the composition contains, in the respective specific amounts, a polymer I composed of a thermoplastic polyester (A) and a polymer II composed of at least one selected from a thermoplastic polyester (B) having a melting point different from the melting point of the thermoplastic polyester (A), a polycarbonate, and a polyurethane.

In addition, an apparatus of producing a recycled monomer according to the present invention can produce a recycled monomer with high efficiency and a high yield, using a continuous reactor to precisely control a reaction temperature in the upstream section of the continuous reactor and a reaction temperature in the downstream section of the continuous reactor.

Brief Description of Drawing

[FIG. 1] FIG. 1 is a schematic diagram illustrating one embodiment of an apparatus of producing a recycled monomer according to the present invention.

[FIG. 2] FIG. 2 is a schematic diagram illustrating another embodiment of an apparatus of producing a recycled monomer according to the present invention.

Description of Embodiments

The present invention will be further described in detail below.

Recycled monomer, recycled polymer, and various products produced using recycled monomer

A recycled monomer according to the present invention is a recycled monomer produced by bringing a composition containing a polymer into contact with subcritical water, wherein the recycled monomer is produced under a condition where X and $X \cdot Y \cdot Z$ which is the product of X, Y, and Z satisfy the following (I) and (II) simultaneously, assuming that the melting point of the polymer in the composition is Mp°C, as measured using a differential scanning calorimeter, that a reaction temperature is X°C, that a reaction time is Y minute(s), and that the mass ratio of the water to the polymer is Z:1.

$$(I)\ Mp \leq X < 300$$

$$(II)\ 2000 \leq X \cdot Y \cdot Z \leq 20000$$

The recycled monomer is preferably a monomer obtained by depolymerizing a polymer that is available through abundant production, and highly necessitated to be recycled. Specifically, a thermoplastic polyester monomer and a thermoplastic polyamide monomer are preferable. Examples of the former monomer include: aromatic dicarboxylic acids such as terephthalic acid, isophthalic acid, phthalic acid, 2,6-naphthalenedicarboxylic acid, 1,5-naphthalene dicarboxylic acid, bis (p-carboxyphenyl)methane, 1,4-anthracene dicarboxylic acid, 1,5-anthracenedicarboxylic acid, 1,8-anthracenedicarboxylic acid, 2,6-anthracene dicarboxylic acid, 9,10-anthracene dicarboxylic acid, 4,4'-diphenyl ether dicarboxylic acid, 5-tetrabutylphosphoniumisophthalic acid, and 5-sodiumsulfoisophthalic acid; aliphatic dicarboxylic acids such as oxalic acid, succinic acid, adipic acid, sebacic acid, azelaic acid, dodecanedioic acid, malonic acid, glutaric acid, and dimer acid; alicyclic dicarboxylic acids such as 1,3-cyclohexane dicarboxylic acid and 1,4-cyclohexane dicarboxylic acid, and ester-forming derivatives thereof; $C_{2-20}$ aliphatic or alicyclic glycols such as ethylene glycol, propylene glycol, 1,4-butane diol, neopentyl glycol, 1,5-pentane diol, 1,6-hexane diol, decamethylene glycol, cyclohexane dimethanol, cyclohexane diol, and dimer diol; long-chain glycols having a molecular weight of 200 to 100,000, such as polyethylene glycol, poly-1,3-propylene glycol, and polytetramethylene glycol; and aromatic dioxy compounds such as 4,4'-dihydroxybiphenyl, hydroquinone, t-butylhydroquinone, bisphenol A, bisphenol S, and bisphenol F, and ester-forming derivatives thereof.

Examples of the latter monomer include; amino acids such as 6-aminocaproic acid, 11-aminoundecanoic acid, 12-aminododecanoic acid, and para-aminomethylbenzoic acid; lactams such as ε-caprolactam and ω-laurolactam; aliphatic diamines such as ethylenediamine, trimethylenediamine, tetramethylenediamine, pentamethylenediamine, hexamethylenediamine, heptamethylenediamine, octamethylenediamine, nonamethylenediamine, decanediamine, undecanediamine, dodecanediamine, tridecanediamine, tetradecanediamine, pentadecanediamine, hexadecanediamine, heptadecanediamine, octadecanediamine, nonadecanediamine, eicosanediamine, 2-methyl-1,5-diamino pentane, and 2-methyl-1,8-diaminooctane; aliphatic dicarboxylic acids such as oxalic acid, succinic acid, adipic acid, suberic acid, azelaic acid, sebacic acid, and dodecanedioic acid; aromatic dicarboxylic acids such as terephthalic acid and isophthalic acid; and dialkyl esters of and dichlorides of these dicarboxylic acids.

In a recycled monomer according to the present invention, the polymer is preferably a thermoplastic polyester or a thermoplastic polyamide. The thermoplastic polyester is preferably a homopolymer or copolymer obtained by polycondensing a dicarboxylic acid or an ester-forming derivative thereof and a diol or an ester-forming derivative thereof. Specific examples include: aromatic polyester resins such as polyethylene terephthalate, polypropylene terephthalate, polybutylene terephthalate, polypropylene isophthalate, polybutylene isophthalate, polybutylene naphthalate, polypropylene isophthalate/terephthalate, polybutylene isophthalate/terephthalate, polypropylene terephthalate/naphthalate, polybutylene terephthalate/naphthalate, polybutylene terephthalate/decane dicarboxylate, polypropylene terephthalate/5-sodium sulfoisophthalate, polybutylene terephthalate/5-sodium sulfoisophthalate, polypropylene terephthalate/polyethylene glycol, polybutylene terephthalate/polyethylene glycol, polypropylene terephthalate/polytetramethylene glycol, polybutylene terephthalate/polytetramethylene glycol, polypropylene terephthalate/isophthalate/polytetramethylene glycol, polybutylene terephthalate/isophthalate/polytetramethylene glycol, polybutylene terephthalate/succinate, polypropylene terephthalate/adipate, polybutylene terephthalate/adipate, polypropylene terephthalate/sebacate, polybutylene terephthalate/sebacate, polypropylene terephthalate/isophthalate/adipate, polybutylene terephthalate/isophthalate/succinate, polybutylene terephthalate/isophthalate/adipate, and polybutylene terephthalate/isophthalate/sebacate. The thermoplastic polyester may be a mixture of two or more of these aromatic polyester resins.

On the other hand, the thermoplastic polyamide is preferably a homopolymer or copolymer obtained by polycondensing an amino acid, lactam, or dicarboxylic acid and a diamine. Specific examples include polycaproamide (polyamide 6), polyhexamethylene adipamide (polyamide 66), polytetramethylene adipamide (polyamide 46), polypentamethylene adipamide (polyamide 56), polytetramethylene sebacamide (polyamide 410), polypentamethylene sebacamide (polyamide 510), polyhexamethylene sebacamide (polyamide 610), polyhexamethylene dodecamide (polyamide 612), and copolymers thereof. The thermoplastic polyamide may be a mixture of two or more of these homopolymers and copolymers. The polymer is more preferably a thermoplastic polyester from the viewpoints of its availability due to abundant production and the availability of more source materials for recycling.

A recycled polymer according to the present invention is obtained by repolymerizing a recycled monomer according to the present invention. Obtaining a recycled polymer by repolymerizing a recycled monomer according to the present invention enables the recycled polymer to be used as a plastic material, and can contribute to recycling of resources.

A clothing fiber, industrial fiber, film, sheet, automobile component, or electrical and electronic component according to the present invention is produced, using a recycled polymer according to the present invention. The product has a value added, environmental friendliness.

Method of producing recycled monomer

A method of producing a recycled monomer according to the present invention includes a step of bringing a composition containing a polymer into contact with subcritical water. A method of producing a recycled monomer according to the present invention includes the below-described four preferable aspects. Herein, three preferable aspects of a method of producing a recycled monomer according to the present invention are simply referred to as a "first preferable aspect of the present invention", a "second preferable aspect of the present invention", and a "third preferable aspect of the present invention" respectively in some cases. In addition, a method of producing a recycled monomer described in the below-described section "[3] Apparatus of producing recycled monomer" is simply referred to as a "fourth preferable aspect of present invention" in some cases.

[2-1] first preferable aspect of the present invention

The first preferable aspect is a method of producing a recycled monomer, the method including a step of bringing a composition containing a thermoplastic polyester (A) into contact with subcritical water, wherein X and X·Y·Z which is the product of X, Y, and Z satisfy the following (I) and (II) simultaneously, assuming that the melting point of the thermoplastic polyester (A) is Mp°C, as measured using a differential scanning calorimeter, that a reaction temperature is X°C, that a reaction time is Y minute(s), and that the mass ratio of the water to the thermoplastic polyester (A) is Z:1.

$$(\text{I}) \ \text{Mp} \leq \text{X} < 300$$

$$(\text{II}) \ 2000 \leq \text{X·Y·Z} \leq 20000$$

Allowing the thermoplastic polyester (A) to be brought into contact with subcritical water to be hydrolyzed enables the thermoplastic polyester (A) to be depolymerized into a diol and/or a dicarboxylic acid that constitute(s) the thermoplastic polyester (A).

The thermoplastic polyester (A) to be used in the present invention is a homopolymer or copolymer obtained by polycondensing a dicarboxylic acid or an ester-forming derivative thereof and a diol or an ester-forming derivative thereof as main raw materials. Here, the main raw materials mean that the dicarboxylic acid or an ester-forming derivative thereof and the diol or an ester-forming derivative thereof as the constituent units in the polymer amount to 50 mol% or more. The amount is preferably 80 mol% or more, still more preferably 90 mol% or more.

Examples of the dicarboxylic acid or an ester-forming derivative thereof include: aromatic dicarboxylic acids such as terephthalic acid, isophthalic acid, phthalic acid, 2,6-naphthalenedicarboxylic acid, 1,5-naphthalene dicarboxylic acid, bis (p-carboxyphenyl)methane, 1,4-anthracene dicarboxylic acid, 1,5-anthracenedicarboxylic acid, 1,8-anthracenedicarboxylic acid, 2,6-anthracene dicarboxylic acid, 9,10-anthracene dicarboxylic acid, 4,4'-diphenyl ether dicarboxylic acid, 5-tetrabutylphosphoniumisophthalic acid, and 5-sodiumsulfoisophthalic acid; aliphatic dicarboxylic acids such as oxalic acid, succinic acid, adipic acid, sebacic acid, azelaic acid, dodecanedioic acid, malonic acid, glutaric acid, and dimer acid; alicyclic dicarboxylic acids such as 1,3-cyclohexane dicarboxylic acid and 1,4-cyclohexane dicarboxylic acid; and ester-forming derivatives thereof. Two or more kinds of these may be used.

An ester-forming derivative as referred to here is a lower alkyl ester, acid anhydride, acid halide, or the like of the dicarboxylic acid described above. As a lower alkyl ester of the dicarboxylic acid, a methyl ester, ethyl ester, hydroxyethyl ester, hydroxybutyl ester, or the like is preferably used. As an acid anhydride of the dicarboxylic acid, an anhydride of dicarboxylic acids, an anhydride of a dicarboxylic acid and an acetic acid, or the like is preferably used. As a halide of the dicarboxylic acid, an acid chloride, acid bromide, acid iodide, or the like is preferably used.

Examples of the diol or an ester-forming derivative thereof include: $C_{2-20}$ aliphatic or alicyclic glycols such as ethylene glycol, propylene glycol, 1,4-butane diol, neopentyl glycol, 1,5-pentane diol, 1,6-hexane diol, decamethylene glycol, cyclohexane dimethanol, cyclohexane diol, and dimer diol; long-chain glycols having a molecular weight of 200 to 100,000, such as polyethylene glycol, poly-1,3-propylene glycol, and polytetramethylene glycol; and aromatic dioxy compounds such as 4,4'-dihydroxybiphenyl, hydroquinone, t-butylhydroquinone, bisphenol A, bisphenol S, and bisphenol F; and ester-forming derivatives thereof. Two or more kinds of these may be used.

Examples of the homopolymer or copolymer containing, as structural units, a dicarboxylic acid or an ester-forming derivative thereof and a diol or an ester-forming derivative thereof include: aromatic polyester resins such as polyethylene terephthalate, polypropylene terephthalate, polybutylene terephthalate, polypropylene isophthalate, polybutylene isophthalate, polybutylene naphthalate, polypropylene isophthalate/terephthalate, polybutylene isophthalate/-terephthalate, polypropylene terephthalate/naphthalate, polybutylene terephthalate/naphthalate, polybutylene terephthalate/decane dicarboxylate, polypropylene terephthalate/5-sodium sulfoisophthalate, polybutylene terephthalate/5-sodium sulfoisophthalate, polypropylene terephthalate/polyethylene glycol, polybutylene terephthalate/polyethylene glycol, polypropylene terephthalate/polytetramethylene glycol, polybutylene terephthalate/polytetramethylene glycol, polypropylene terephthalate/isophthalate/polytetramethylene glycol, polybutylene terephthalate/isophthalate/polytetramethylene glycol, polybutylene terephthalate/succinate, polypropylene terephthalate/adipate, polybutylene terephthalate/adipate, polypropylene terephthalate/sebacate, polybutylene terephthalate/sebacate, polypropylene terephthalate/isophthalate/adipate, polybutylene terephthalate/isophthalate/succinate, polybutylene terephthalate/isophthalate/adipate, and polybutylene terephthalate/isophthalate/sebacate.

Here, "/" denotes a copolymer. These homopolymers and copolymers may be used singly, or used in mixture of two or more kinds thereof in arbitrary amounts. Among others, a homopolymer or copolymer, which is available through abundant production and for abundant consumption, containing a residue of an aromatic dicarboxylic acid, or an ester-forming derivative thereof, and an aliphatic diol or an ester-forming derivative thereof as main raw materials is preferable for the purposes of recycling the thermoplastic polyester (A), and promoting recycling of fossil resources. Examples of the preferable polymer include a homopolymer or copolymer obtained by polycondensing the following: at least one selected from terephthalic acid, isophthalic acid, naphthalene dicarboxylic acid, and ester-forming derivatives thereof; and at least one selected from ethylene glycol, 1,3-propylene glycol, 1,4-butane diol, and ester-forming derivatives thereof. Among these, at least one selected from the group consisting of polyethylene terephthalate, polypropylene terephthalate, polybutylene terephthalate, and copolymers thereof is particularly preferably used.

A thermoplastic polyester (A) according to the present invention may be blended with a polymerization catalyst, thermoplastic resin other than a thermoplastic polyester, dye, additive of any kind, fibrous filler, filler other than fibrous filler, or the like to the extent that the objects of the present invention are not impaired. Here, the fibrous filler may be any filler having a fibrous form. The filler other than a fibrous filler may be any of an organic filler and an inorganic filler, and is, for example, a non-fibrous filler. Two or more kinds of these may be blended in.

A thermoplastic polyester (A) according to the present invention may be a waste of a resin molded article containing at least a thermoplastic polyester. Examples of the waste of a resin molded article containing a thermoplastic polyester include thermoplastic polyester products, industrial wastes generated in production processes of a thermoplastic polyester product, spent wastes of a thermoplastic polyester product, and the like. Examples of the thermoplastic polyester product include: containers such as drink bottles and seasoning bottles; sheet products such as food trays, blister packs, partitions for food, and industrial trays; film products such as packaging films, optical functional films, magnetic tapes, and insulating materials; clothing fiber structures such as old clothes, uniforms, sports wear, and inner wear; industrial fiber structures such as curtains, carpets, nets, belts, and seats; and molded articles such as automobile components, electrical and electronic components, construction components and materials, daily necessities, household sundries, and sanitary goods. Furthermore, product scraps, pellet scraps, lumpy scraps, and the like generated in production processes of these correspond to objects as wastes. A waste of a resin molded article containing a thermoplastic polyester, together with a product composed of a thermoplastic polyester other than the thermoplastic polyester (A), may be used in a depolymerization method according to the present invention to the extent that the effects of the present invention are not impaired.

A method of depolymerizing the thermoplastic polyester (A) in the first preferable aspect of the present invention is characterized by including a step of bringing a thermoplastic polyester into contact with subcritical water, wherein, when at least the thermoplastic polyester (A) and the water are mixed under heating, X and X·Y·Z which is the product of X, Y, and Z satisfy (i) $Mp \leq X < 300$ and (ii) $2000 \leq X \cdot Y \cdot Z \leq 20000$ simultaneously, assuming that the melting point of the thermoplastic polyester is Mp°C, that the reaction temperature is X°C, that the reaction time is Y minute(s), and that the mass ratio of the water to the thermoplastic polyester is Z:1.

Here, the melting point of the thermoplastic polyester (A) in the present invention is the temperature of an endothermic peak that appears when the thermoplastic polyester (A) is cooled from a molten state to 30°C at a cooling rate of 20°C/minute, and then heated to the melting point + 40°C at a heating rate of 20°C/minute under an inert gas atmosphere, using a differential scanning calorimeter manufactured by TA Instruments, Inc. However, in a case where two or more endothermic peaks are detected, the temperature of the endothermic peak having the largest peak intensity is regarded as the melting point. The melting point of the thermoplastic polyester is not particularly limited, and a preferable range of the melting point is, for example, more than 50°C and less than 300°C. Having a melting point of more than 50°C allows the thermoplastic polyester to be solid at ordinary temperature, and hence, has excellent handleability. In addition, having a melting point of less than 300°C enables the thermoplastic polyester to be mixed in a molten state with water within a temperature region applicable for inhibiting the conversion of a depolymerized product, thus can enhance the reaction efficiency, and hence, is preferable.

Water to be used here is not particularly limited. Any water, such as tap water, ion-exchanged water, distilled water, or well water, may be used. Ion-exchanged water or distilled water is preferably used from the viewpoint of inhibiting a side reaction due to the influence of a coexistent salt.

As for the water, it could be sufficient as long as the water is heated to between Mp°C, the melting point of the thermoplastic polyester (A) and 300°C when reacted with the thermoplastic polyester (A). It is known that when brought up to a pressure of 22.1 MPa and a temperature of 374.2°C, water presents a state that is neither liquid nor gaseous. This point is referred to as the critical point of water. Hot water in the near region of the critical point, i.e., water having a temperature and a pressure that are each slightly lower than the critical point is referred to as subcritical water. Water to be use in the present invention is at less than 300°C, and corresponds to subcritical water. Although this subcritical water is water, the subcritical water is characterized by having (i) a low dielectric constant and (ii) a high ionic product. The dielectric constant and ionic product of the subcritical water depend on temperature and the partial pressure of the water, and can be controlled. Although the water is water, the water having a lower dielectric constant becomes an excellent solvent for an organic compound, and the water having a higher ionic product results in having a higher hydrogen ion concentration and a higher oxide ion concentration, hence having an excellent hydrolytic action.

In addition, it is a preferable example that the pressure of the water is higher than the saturated vapor pressure. The water may be in a liquid state or a gaseous state such as water vapor, or both of them may be used. In the field of reaction, water in a liquid state facilitates reaction better than water in a gaseous state, and hence, the pressure of the water is preferably higher than the saturated vapor pressure. In addition, the pressure of the water is not particularly limited to any upper limit, and is preferably 30 MPa. The upper limit of the pressure of the water is more preferably 25 MPa, still more preferably 22 MPa. The pressure within such a range allows the above-described ionic product of the water to have a tendency to be high, and hence, is preferable. A method of bringing the water within such a pressure range include a method of hermetically sealing a pressure container to pressurize the inside thereof. To pressurize the inside of a pressure container, for example, a method of enclosing gas in addition to the water can be used. Examples of such a gas include air, argon, and nitrogen. From the viewpoint of inhibiting a side reaction such as oxidation, nitrogen or argon is preferably used as the gas to be enclosed. In addition, introduction of high-pressure water enables the inside of a pressure container to be pressurized. In a case where high-pressure water is used, the inside of the pressure container is also enabled to be gas-free. The degree of pressurization with gas or high-pressure water is not particularly limited in being set for a pressure of interest, and is preferably 0.3 MPa or more.

The reaction temperature in a method of depolymerizing the thermoplastic polyester resin (A) in the first preferable aspect of the present invention is Mp°C or more and less than 300°C, assuming that the melting point of the thermoplastic polyester (A) is Mp°C. The reaction temperature here refers to a temperature during a given period of time when the thermoplastic polyester (A) and the water are allowed to react in a reactor with the temperature controlled. The reaction temperature may be a constant temperature or a temperature varied with time. In this regard, a temperature in the heating process until the attainment of the reaction temperature and a temperature in the cooling process after the reaction at a reaction temperature are not particularly limited, subject to being not higher than the reaction temperature. With the reaction temperature not less than the melting point of the thermoplastic polyester, the thermoplastic polyester (A) becomes molten state during reaction, and, when mixed with water, is dispersed in droplets, thus increasing the interfacial area, and making it possible to enhance the reaction efficiency. On the other hand, the reaction temperature of less than 300°C makes it possible to regulate the hot-water-solubility of a dicarboxylic acid, which catalytically acts on the conversion reaction (overreaction) of a diol. Thus, the reaction temperature enables the conversion reaction of the diol to be inhibited. The reaction temperature is preferably 290°C or less for the purpose of decreasing coloring, particularly preferably 280°C or less for the purpose of increasing the yield of the diol.

When the reaction temperature X is within the preferable range, Z is preferably less than 6, assuming that the mass ratio of the water to the thermoplastic polyester is Z:1. It is known that a diol has a large water-solubility, and well mixed with water at temperature ranging from room temperature to the reaction temperature X, and on the other hand, that the water-solubility of a dicarboxylic acid is small at room temperature, and tends to be large in hot water. For example, terephthalic acid, which is a dicarboxylic acid generated by hydrolyzing polyethylene terephthalate and polybutylene terephthalate, is hardly soluble in water, at 15 mg/L at ordinary temperature (20°C), but at a higher temperature, the water-solubility increases accordingly, affording an aqueous solution of terephthalic acid contained at 10 mass% at 250°C and at 44 mass% at 300°C. The hot-water-solubility of a dicarboxylic acid is regulated within a temperature region settable for the reaction temperature X to be preferable, and Z is made less than 6, whereby the concentration of the dicarboxylic acid with respect to the diol can be made relatively low, enabling the diol and the dicarboxylic acid to be obtained with a high yield. Considering that it is advantageous to enhance the treatment efficiency, and avoid an increase in the size of apparatus, Z is still more preferably 5 or less, particularly preferably 4 or less.

A method of depolymerizing the thermoplastic polyester (A) in the first preferable aspect of the present invention is characterized by bringing about contact under a condition where X·Y·Z which is the product of X, Y, and Z satisfies $2000 \leq X·Y·Z \leq 20000$, assuming that the reaction temperature is X°C, that the reaction time is Y minute(s), and that the mass ratio of the water to the thermoplastic polyester is Z:1. The reaction time Y here refers to a time during which the thermoplastic polyester (A) and the water are mixed in a reactor controlled at a reaction temperature X. The heating time until the attainment of the reaction temperature X and the cooling time after the reaction at the reaction temperature X are not included in Y. The value of Y is not particularly limited, subject to satisfying the range of the product of X, Y, and Z, and can be set with respect to X and Z. The product of X, Y, and Z is preferably 17,500 or less, still more preferably 15,000 or less, particularly preferably 12,500 or less. In addition, a condition can be taken for example, under which condition, the product of X, Y, and Z is preferably 2,200 or more, more preferably 2,400 or more, particularly preferably 2,600 or more. In this regard, the reaction may be performed at a plurality of temperatures. The product $X \cdot Y \cdot Z$ in this case is the total of the respective products $X \cdot Y \cdot Z$ of the reaction temperatures. The present invention relates to an energy-saving depolymerization method intended to achieve both recycling of fossil resources and a decrease in greenhouse gas emissions. Water has a specific heat capacity of 4.3 kJ/kg·K and a heat of vaporization of 2,250 kJ/kg, which are very high, compared with another organic solvent. Thus, it is important to decrease the amount of water to be used. Bringing the product of X, Y, and Z within these conditional ranges makes it possible to achieve both the generation efficiency of a dicarboxylic acid and a diol and energy saving.

Furthermore, to achieve the recovery of a diol and energy saving during purification, the concentration of a diol derived from the thermoplastic polyester (A) contained in a reaction mixture is preferably 4.9 mass% or more. The concentration of the diol is more preferably 6.0 wt% or more, still more preferably 7.0 wt% or more. That the concentration of the diol contained in the reaction mixture is within the preferable range makes it possible to decrease the amount of the reaction mixture to be treated for recovery of 1 kg of a diol, thus makes it possible to decrease the size of apparatus, and furthermore, makes it possible to reduce energy needed to heat the water during recovery. Here, the concentration of a diol derived from the thermoplastic polyester (A) contained in a reaction mixture can be determined by the absolute calibration curve method, using a gas chromatograph GC-2010 manufactured by Shimadzu Corporation. In this regard, the diol here is a diol produced by depolymerizing the thermoplastic polyester (A), not a diol added before and/or after the reaction. A method in which the concentration of a diol derived from the thermoplastic polyester (A) contained in in a reaction mixture is brought within the preferable range may be, for example, a method in which the thermoplastic polyester (A) is depolymerized in accordance with the present invention with the mass ratio Z of the water to the thermoplastic polyester (A) within the preferable range.

In a case where polybutylene terephthalate is used as the thermoplastic polyester (A), the depolymerization thereof generates butane diol as a raw material and a cyclic ether (tetrahydrofuran) from dehydration of butane diol, and the amount of the latter cyclic ether generated is larger. In a case where polybutylene terephthalate is used as the thermoplastic polyester (A), the concentration of the cyclic ether derived from the thermoplastic polyester (A) in a reaction mixture is preferably 3.0 mass% or more. The amount is more preferably 5.0 wt% or more, still more preferably 6.0 parts by weight or more. The preferable range makes it possible to restrain energy consumption during the recovery and purification of the cyclic ether.

A study has been made on the following: the generation of a diol and a dicarboxylic acid through reaction between the thermoplastic polyester and the water; the hot-water-solubility of the dicarboxylic acid; and the conversion of the diol. Consequently, with regards the above-described thermoplastic polyester other than polybutylene terephthalate, bringing X, the product of X, Y, and Z, and furthermore Z within the above-described ranges makes it possible that the conversion of a diol is inhibited, and that the yield of the dicarboxylic acid and the diol that constitute the thermoplastic polyester, particularly the diol, is significantly enhanced.

Furthermore, blending in an alkaline component makes it possible to enhance the yield of the monomer generated. Examples of the alkaline component include hydroxides, carbonates and bicarbonates of alkaline metals such as lithium, sodium, and potassium; alkaline earth metals such as calcium and ammonium and amines. In particular, at least one selected from sodium hydroxide, potassium hydroxide, and calcium hydroxide is preferable. The amount of the alkaline component to be blended in is preferably 0.01 part by weight or more and 50 parts by weight or less with respect to 100 parts by weight of the thermoplastic polyester (A). The amount is more preferably 20 parts by weight or more, still more preferably 30 parts by weight or more. On the other hand, the amount is more preferably 45 parts by weight or less, still more preferably 42 parts by weight or less. Blending in an alkaline component causes the hydrolysis of the thermoplastic polyester to generate a dicarboxylate. Through treatment with an acidic aqueous solution, a dicarboxylate can be converted into a dicarboxylic acid that is a thermoplastic polyester raw material.

For the depolymerization of the thermoplastic polyester in the first preferable aspect of the present invention, any kind of known reaction method of a batch type, continuous type, or the like can be adopted. Examples of the batch type include: an autoclave and a vertical or horizontal type reactor, which each includes a stirrer and a heating function; and a vertical or horizontal type reactor including a compression mechanism such as a cylinder in addition to a stirrer and a heating function. Examples of the continuous type include: an extruder and a tubular reactor, which each includes a heating function; a tubular reactor including a mixing mechanism such as a baffle; a line mixer; a vertical or horizontal type reactor; a vertical or horizontal type reactor including a stirrer; and a tower. In addition, the atmosphere in production is desirably a nonoxidizing atmosphere. The production is preferably performed under an inert atmosphere such as nitrogen, helium, and argon. A nitrogen atmosphere is preferable from the viewpoints of economical efficiency and easiness of handling.

A method of recovering a diol and a dicarboxylic acid generated using a method of depolymerizing a thermoplastic polyester in the first preferable aspect of the present invention is not particularly limited, and any method can be adopted. For example, in a case where the depolymerization is performed in a batch manner, a reaction mixture is distilled out together with water after the termination of the depolymerization reaction, and the reaction mixture (C) can be obtained in the form of an aqueous diol solution in which a solid dicarboxylic acid is dispersed and/or precipitated. Additionally, in a case where the depolymerization is performed continuously, the reaction mixture (C) can be obtained, with the progress of the reaction, in the form of an aqueous diol solution in which a solid dicarboxylic acid is dispersed. From the reaction mixture (C) obtained, a solid dicarboxylic acid is separated using a known method such as solid-liquid separation, and a diol is separated from water through separation by distillation, whereby a diol and a dicarboxylic acid that have a high purity can be recovered. Additionally, if the reaction mixture has any component insoluble in water, the component is separated using a known method such as solid-liquid separation under a condition where a dicarboxylic acid is dissolved. Then, the reaction mixture can be used in a step of recovering a diol and a dicarboxylic acid.

A method of obtaining a diol having an even higher purity can be a method combined with a purification method such as the following: a method in which the diol recovered is precisely distilled; a method in which the diol, with a trace amount of sodium hydroxide added, is distilled under reduced pressure; a method in which the diol is treated with activated carbon; a method in which the diol is ion-exchanged; or a method in which the diol is recrystallized. Such a method can efficiently remove impurities that are difficult to separate through separation by distillation.

A method of depolymerizing a thermoplastic polyester according to the first preferable aspect of the present invention makes it possible to obtain a diol and dicarboxylic acid that have a high purity. The diol and the dicarboxylic acid can be used as polymerization raw materials for a thermoplastic polyester in the same manner as a diol and a carboxylic acid that are produced from a petroleum-derived raw material. A diol and a dicarboxylic acid or an ester-forming derivative thereof can be used in a commonly known polycondensation reaction to produce a thermoplastic polyester (recycled polymer).

In addition, the thermoplastic polyester obtained in this manner can be processed into and utilized as various products such as clothing fibers, industrial fibers, films, sheets, bottles, injection-molded articles, and the like in the same manner as a thermoplastic polyester produced from a petroleum-derived raw material. These products are useful for agricultural materials, gardening materials, fishing materials, civil engineering and construction materials, stationery, medical supplies, automobile components, electrical and electronic components, or other applications.

[2-2] second preferable aspect of the present invention

The second preferable aspect of the present invention is a method of producing a recycled monomer, including a step of bringing a composition containing a thermoplastic polyester (A) into contact with subcritical water, wherein 0.01 to 4.0 parts by weight of an alkaline metal salt and/or an alkaline earth metal salt is blended with 100 parts by weight of the thermoplastic polyester (A) in the composition containing the thermoplastic polyester (A). In the composition containing a thermoplastic polyester (A), another component such as an additive has an influence on the depolymerization of the thermoplastic polyester (A). In the second preferable aspect of the present invention, an alkaline metal salt and/or an alkaline earth metal salt is/are blended in so that the yield of a recycled monomer obtained through depolymerization can be enhanced. In addition, the amount of the component(s) blended in is made a trace so that an increase in greenhouse gas emissions derived from an alkaline metal salt and/or an alkaline earth metal salt can be restrained.

The thermoplastic polyester (A) to be used in the second preferable aspect of the present invention is a homopolymer or copolymer obtained by polycondensing a dicarboxylic acid or an ester-forming derivative thereof and a diol or an ester-forming derivative thereof as main raw materials. Here, the main raw materials mean that the dicarboxylic acid or an ester-forming derivative thereof and the diol or an ester-forming derivative thereof as the constituent units in the polymer amount to 50 mol% or more. The amount is more preferably 80 mol% or more, still more preferably 90 mol% or more.

Examples of the dicarboxylic acid or an ester-forming derivative thereof include: aromatic dicarboxylic acids such as terephthalic acid, isophthalic acid, phthalic acid, 2,6-naphthalenedicarboxylic acid, 1,5-naphthalene dicarboxylic acid, bis (p-carboxyphenyl)methane, 1,4-anthracene dicarboxylic acid, 1,5-anthracenedicarboxylic acid, 1,8-anthracenedicarboxylic acid, 2,6-anthracene dicarboxylic acid, 9,10-anthracene dicarboxylic acid, diphenyl-4,4'-dicarboxylic acid, 4,4'-diphenyl ether dicarboxylic acid, 5-tetrabutylphosphoniumisophthalic acid, and 5-sodiumsulfoisophthalic acid; aliphatic dicarboxylic acids such as oxalic acid, succinic acid, adipic acid, sebacic acid, azelaic acid, dodecanedioic acid, malonic acid, glutaric acid, and dimer acid; alicyclic dicarboxylic acids such as 1,3-cyclohexane dicarboxylic acid and 1,4-cyclohexane dicarboxylic acid; and ester-forming derivatives thereof.

An ester-forming derivative as referred to here is a lower alkyl ester, acid anhydride, acid halide, or the like of the dicarboxylic acid described above. As a lower alkyl ester of the dicarboxylic acid, a methyl ester, ethyl ester, hydroxyethyl ester, hydroxybutyl ester, or the like is preferably used. As an acid anhydride of the dicarboxylic acid, an anhydride of dicarboxylic acids, an anhydride of a dicarboxylic acid and an acetic acid, or the like is preferably used. As a halide of the dicarboxylic acid, an acid chloride, acid bromide, acid iodide, or the like is preferably used.

Examples of the diol or an ester-forming derivative thereof include: $C_{2-20}$ aliphatic or alicyclic glycols such as ethylene glycol, propylene glycol, 1,4-butane diol, neopentyl glycol, 1,5-pentane diol, 1,6-hexane diol, decamethylene glycol, cyclohexane dimethanol, cyclohexane diol, and dimer diol; long-chain glycols having a molecular weight of 200 to 100,000, such as polyethylene glycol, poly-1,3-propylene glycol, and polytetramethylene glycol; and aromatic dioxy compounds such as 4,4'-dihydroxybiphenyl, hydroquinone, t-butylhydroquinone, bisphenol A, bisphenol S, and bisphenol F; and ester-forming derivatives thereof.

Examples of the homopolymer or copolymer containing, as structural units, a dicarboxylic acid or an ester-forming derivative thereof and a diol or an ester-forming derivative thereof include: aromatic polyester resins such as polyethylene terephthalate, polypropylene terephthalate, polybutylene terephthalate, polyethylene isophthalate, polypropylene isophthalate, polybutylene isophthalate, polyethylene naphthalate, polypropylene naphthalate, polybutylene naphthalate, polyethylene isophthalate/terephthalate, polypropylene isophthalate/terephthalate, polybutylene isophthalate/terephthalate, polyethylene terephthalate/naphthalate, polypropylene terephthalate/naphthalate, polybutylene terephthalate/naphthalate, polybutylene terephthalate/decane dicarboxylate, polyethylene terephthalate/5-sodium sulfoisophthalate, polypropylene terephthalate/5-sodium sulfoisophthalate, polybutylene terephthalate/5-sodium sulfoisophthalate, polyethylene terephthalate/polyethylene glycol, polypropylene terephthalate/polyethylene glycol, polybutylene terephthalate/polyethylene glycol, polyethylene terephthalate/polytetramethylene glycol, polypropylene terephthalate/polytetramethylene glycol, polybutylene terephthalate/polytetramethylene glycol, polyethylene terephthalate/isophthalate/polytetramethylene glycol, polypropylene terephthalate/isophthalate/polytetramethylene glycol, polybutylene terephthalate/isophthalate/polytetramethylene glycol, polyethylene terephthalate/succinate, polypropylene terephthalate//succinate, polybutylene terephthalate/succinate, polyethylene terephthalate/adipate, polypropylene terephthalate/adipate, polybutylene terephthalate/adipate, polyethylene terephthalate/sebacate, polypropylene terephthalate/sebacate, polybutylene terephthalate/sebacate.

Here, "/" denotes a copolymer. These homopolymers and copolymers may be used singly, or used in mixture of two or more kinds thereof in arbitrary amounts. Among others, a homopolymer or copolymer containing, as main raw materials, an aromatic dicarboxylic acid available through abundant production and for abundant consumption, or an ester-forming derivative thereof, and an aliphatic diol or an ester-forming derivative thereof is preferable for the purposes of recycling the thermoplastic polyester, and promoting recycling of fossil resources. Examples of the preferable polymer include a homopolymer or copolymer obtained by polycondensing the following: at least one selected from terephthalic acid, naphthalene dicarboxylic acid, and ester-forming derivatives thereof; and at least one selected from ethylene glycol, 1,3-propylene glycol, 1,4-butane diol, and ester-forming derivatives thereof. Among these, at least one selected from polyethylene terephthalate, polypropylene terephthalate, polybutylene terephthalate, and copolymers thereof is particularly preferably used as the thermoplastic polyester (A) in the composition containing the thermoplastic polyester (A).

Examples of the alkaline metal salt include lithium hydroxide, sodium hydroxide, potassium hydroxide, cesium hydroxide, lithium carbonate, sodium carbonate, potassium carbonate, cesium carbonate, lithium hydrogencarbonate, sodium hydrogencarbonate, potassium hydrogencarbonate, cesium hydrogencarbonate, lithium phosphate, sodium phosphate, potassium phosphate, cesium phosphate, dilithium hydrogenphosphate, dipotassium hydrogenphosphate, didicesium hydrogenphosphate, lithium monohydrogenphosphate, sodium monohydrogenphosphate, potassium monohydrogenphosphate, cesium monohydrogenphosphate, lithium borate, sodium borate, potassium borate, cesium borate, lithium citrate, sodium citrate, potassium citrate, cesium citrate, lithium formate, sodium formate, potassium formate, cesium formate, lithium acetate, sodium acetate, potassium acetate, cesium acetate, lithium oxalate, sodium oxalate, potassium oxalate, cesium oxalate, lithium maleate, potassium maleate, and cesium maleate. Among these, lithium hydroxide, sodium hydroxide, potassium hydroxide, cesium hydroxide, lithium carbonate, sodium carbonate, potassium carbonate, cesium carbonate, lithium hydrogencarbonate, sodium hydrogencarbonate, potassium hydrogencarbonate, and cesium hydrogencarbonate are more preferable.

Examples of the alkaline earth metal salt include magnesium hydroxide, calcium hydroxide, barium hydroxide, magnesium carbonate, calcium carbonate, barium carbonate, magnesium phosphate, calcium phosphate, barium phosphate, magnesium hydrogenphosphate, calcium hydrogenphosphate, barium hydrogenphosphate, dimagnesium hydrogenphosphate, dicalcium hydrogenphosphate, dibarium hydrogenphosphate, magnesium borate, calcium borate, barium borate, magnesium citrate, calcium citrate, barium citrate, magnesium formate, calcium formate, barium formate, magnesium acetate, calcium acetate, barium acetate, magnesium borate, calcium borate, barium borate, magnesium oxalate, calcium oxalate, barium oxalate, magnesium maleate, calcium maleate, and barium maleate. Among these, magnesium hydroxide, calcium hydroxide, barium hydroxide, magnesium carbonate, calcium carbonate, and barium carbonate are more preferable.

With 100 parts by weight of the thermoplastic polyester (A), 0.01 to 4.0 parts by weight of the alkaline metal salt and/or the alkaline earth metal salt is blended, making it possible to inhibit a product of depolymerization from overreaction, and to obtain a polyester monomer with a high yield. The amount of the alkaline metal salt and/or the alkaline earth metal salt blended in is more preferably 0.05 part by weight or more, still more preferably 0.25 part by weight or more. On the other hand, the amount is more preferably 2.5 parts by weight or less, still more preferably 1.0 part by weight or less.

In the second preferable aspect of the present invention, 100 to 1000 parts by weight of water is preferably blended with 100 parts by weight of the thermoplastic polyester (A) in the composition containing the thermoplastic polyester (A). Blending in 100 to 1000 parts by weight of water makes it possible both to inhibit an environmental burden by restraining the consumption of energy for heating water, and to obtain a polyester monomer with a high yield. The amount of water blended in is more preferably 150 parts by weight or more, still more preferably 200 parts by weight or more. On the other hand, the amount is more preferably 800 parts by weight or less, still more preferably 600 parts by weight or less.

In the second preferable aspect of the present invention, the temperature in a step (step 1) of blending the alkaline metal salt and/or the alkaline earth metal salt with the thermoplastic polyester (A), and depolymerizing the polyester is preferably 250 to 350°C. Bringing the temperature within this range promotes depolymerization, and inhibits the overreaction of a product, enabling a polyester monomer to be obtained with a high yield. The temperature is more preferably 260°C or more, still more preferably 270°C or more. On the other hand, the temperature is more preferably 320°C or less, still more preferably 300°C or less.

In the second preferable aspect of the present invention, the pressure in the step (step 1) of blending the alkaline metal salt and/or the alkaline earth metal salt with the thermoplastic polyester (A), and depolymerizing the polyester is preferably 3 to 30 MPa. Bringing the pressure within this range increases the ion concentration of water, thus enabling the thermoplastic polyester (A) to be depolymerized efficiently. The pressure is more preferably 4.5 MPa or more, still more preferably 6.0 MPa or more. On the other hand, the pressure is more preferably 25 MPa or less, still more preferably 20 MPa or less.

In the second preferable aspect of the present invention, a composition containing a thermoplastic polyester (A) may be used, in which composition the thermoplastic polyester (A) is blended with a dye, additive of any kind, fibrous filler, fibrous filler other than an organic filler and an inorganic filler, and/or the like. Examples of the additive include antioxidants, weather-resistant agents, mold release agents, and nucleating agents.

Examples of the antioxidant include hindered-phenol antioxidants, phosphorus antioxidants, and sulfur antioxidants.

Examples of the phenol antioxidant include 2,4-dimethyl-6-*t*-butylphenol, 2,6-di-*t*-butylphenol, 2,6-di-*t*-butyl-*p*-cresol, 2,6-di-*t*-butyl-4-ethyl phenol, 4,4'-butylidene bis(6-*t*-butyl-3-methylphenol), 2,2'-methylene-bis(4-methyl-6-*t*-butylphenol), 2,2'-methylene-bis(4-ethyl-6-*t*-butylphenol), octadecyl-3-(3',5'-di-*t*-butyl-4'-hydroxyphenyl)propionate, pentaerythritoltetrakis [3-(3,5-di-*t*-butyl-4-hydroxyphenyl)propionate], 1,1,3-tris(2-methyl-4-hydroxy-5-di-*t*-butylphenyl)butane, tris(3,5-di-*t*-butyl-4-hydroxybenzyl)isocyanurate, triethylene glycol-bis[3-(3-*t*-butyl-4-hydroxy-5-methylphenyl)propionate], 1,6-hexanediolbis[3-(3,5-di-*t*-butyl-4-hydroxyphenyl)propionate]], 2,4-bis-(*n*-octylthio)-6-(4-hydroxy-3,5-di-*t*-butylanilino)-1,3,5-triazine, 2,2-thio-diethylenebis[3-(3,5-di-*t*-butyl-4-hydroxyphenyl)propionate], *N,N'*-hexamethylene bis(3,5-di-*t*-butyl-4-hydroxyhydrocinnamide), 3,5-di-*t*-butyl-4-hydroxybenzyl phosphonate-diethyl ester, 1,3,5-trimethyl-2,4,6-tris(3,5-di-*t*-butyl-4-hydroxybenzyl)benzene, tris(3,5-di-*t*-butyl-4-hydroxybenzyl)-isocyanurate, 2,4-bis[(octylthio)methyl]-*o*-cresol, and isooctyl-3-(3,5-di-*t*-butyl-4-hydroxyphenyl)propionate.

Examples of the phosphorus antioxidant include triphenyl phosphite, triisodecyl phosphite, isodecyldiphenyl phosphite, 2-ethylhexyldiphenyl phosphite, 4,4'-isopropylidenediphenolalkyl ($C_{12}$-$C_{15}$) phosphite, tris(nonylphenyl) phosphite (Adekastab 1178), tris(2,4-di-*t*-butylphenyl) phosphite, 2,2'-methylenebis(4,6-di-*t*-butylphenyl)2-ethylhexyl phosphite, 3,9-bis(2,6-di-*t*-butyl-4-methylphenoxy)-2,4,8,10-tetraoxa-3,9-diphosphaspiro[5.5]undecane, and 3,9-bis(octadecyloxy)-2,4,8,10-tetraoxa-3,9-diphosphaspiro[5.5]undecane.

Examples of the sulfur antioxidant include dilauryl thiodipropionate, dimyristylthio dipropionate, distearylthio dipropionate, ditridecylthio dipropionate, pentaerythritol(3-laurylthiopropionate), and 2-mercaptobenzimidazole.

These antioxidants may be used singly. The antioxidants, when used in combination of two or more kinds thereof, afford a synergistic effect in some cases, and thus, may be used in combination of a plurality of kinds thereof.

Examples of the weather-resistant agent include ultraviolet absorbers and light stabilizers. Specific examples of the ultraviolet absorber include: benzophenone ultraviolet absorbers typified by 2-hydroxy-4-octyloxybenzophenone, 2,2'-dihydroxy-4,4'-dimethoxybenzophenone, and 2,2',4,4'-tetrahydroxybenzophenone; benzotriazole ultraviolet absorbers typified by condensates with 4,6-bis(1-methyl-1-phenylethyl)-2-(2*H*-benzotriazole-2-yl)phenol, 2-(2'-hydroxy-5'-methylphenyl)benzotriazole, 2-(2'-hydroxy-3',5'-di-*tert*-amylphenyl)benzotriazole, 2-[2'-hydroxy-3',5'-bis(α,α'-dimethylbenzyl)phenyl]benzotriazole, 2,2'-methylenebis[4-(1,1,3,3-tetramethylbutyl)-6-(2*H*-benzotriazole-2-yl)phenol], 2-(2*H*-benzotriazole-2-yl)-4-(1,1,3,3-tetramethylbutyl)phenol, 2-(2*H*-benzotriazole-2-yl)-p-cresol, 2-(5-chloro-2*H*-benzotriazole-2-yl)-6-t-butyl-4-methylphenol, 2-(4,6-diphenyl-1,3,5-triazine-2-yl)-5-[2-(2-ethylhexanoyloxy)ethoxy]phenol, or methyl-3-[3-*tert*-butyl-5-(2*H*-benzotriazole-2-yl)-4-hydroxyphenyl]propionate-polyethylene glycol; triazine ultraviolet absorbers typified by 2,4,6-tris(2-hydroxy-4-hexyloxy-3-methylphenyl)-1,3,5-triazine.

In addition, specific examples of the light stabilizer include hindered amine light stabilizers typified by bis(2,2,6,6-tetramethyl-4-piperidyl)sebacate, bis(1,2,2,6,6-pentamethyl-4-piperidyl)sebacate, tetrakis(2,2,6,6-tetramethyl-4-piperidyl)-1,2,3,4-butane tetracarboxylate, tetrakis(1,2,2,6,6-pentamethyl-4-piperidyl)-1,2,3,4-butane tetracarboxylate, poly{[6-(1,1,3,3-tetramethylbutyl)amino-1,3,5-triazine-2,4-diyl] [(2,2,6,6-tetramethylpiperidyl)imino]hexamethylene[(2,2,6,6-tetramethylpiperidyl)imino]}, polymethylpropyl 3-oxy-[4-(2,2,6,6-tetramethyl)piperidinyl]siloxane, esterified product mixtures of 1,2,3,4-butane tetracarboxylic acid with 1,2,2,6,6-pentamethyl-4-piperidinol and 3,9-bis(2-hydroxy-1,1-dimethyl-lethyl)-2,4,8,10-tetraoxaspiro[5.5]undecane, esterified product mixtures of 1,2,3,4-butane tetracarboxylic acid with 2,2,6,6-tetramethyl-4-piperidinol and 3,9-bis(2-hydroxy-1,1-dimethylethyl)-2,4,8,10-tetraoxaspiro[5.5]undecane, bis(1-undecane oxy-2,2,6,6-tetramethylpiperidine-4-yl)carbonate, 1,2,2,6,6-pentamethyl-4-piperidyl methacrylate), and 2,2,6,6-tetramethyl-4-piperidyl methacrylate.

Specific examples of the mold release agent include fatty acids, fatty acid metal salts, oxyfatty acids, fatty acid esters, aliphatic partly-saponified esters, paraffins, low-molecular-weight polyolefins, fatty acid amides, alkylene bisfatty acid amides, aliphatic ketones, and modified silicones.

Among these, a fatty acid ester-based mold release agent is composed of a trivalent to hexavalent aliphatic alcohol and a fatty acid. Examples of the trivalent to hexavalent aliphatic alcohol include glycerol, diglycerol, erythritol, pentaerythritol, sorbitol, triglycerol, dipentaerythritol, and tetraglycerol.

In addition, the fatty acid-based mold release agent is a $C_{5-30}$ linear or branched saturated fatty acid. Examples include pentanoic acid, hexanoic acid, octanoic acid, nonanoic acid, decanoic acid, lauric acid, myristic acid, palmitic acid, stearic acid, isostearic acid, arachidic acid, behenic acid, lignoceric acid, cerotinic acid, montanoic acid, and melissic acid. Specific examples of the nucleating agent include inorganic nucleating agents, such as synthesized mica, talc (the tradename, High toron or the like), clay, zeolite, magnesium oxide, calcium sulfide, boron nitride, and neodymium oxide.

In addition, examples of the organic nucleating agent include: organic carboxylic acid metal salts such as sodium benzoate, potassium benzoate, lithium benzoate, calcium benzoate, magnesium benzoate, barium benzoate, dilithium terephthalate, disodium terephthalate, dipotassium terephthalate, sodium toluate, sodium salicylate, potassium salicylate, zinc salicylate, aluminium dibenzoate, potassium dibenzoate, lithium dibenzoate, sodium β-naphthalate, and sodium cyclohexanecarboxylate; organic sulfoniates such as sodium p-toluenesulfonate and sodium sulfoisophthalate; and phosphorus compound metal salts such as sorbitol compounds, metal salts of phenylphosphonate, and sodium-2,2'-methylenebis(4,6-di*t*-butylphenyl)phosphate.

The amount of the above-described additive is preferably 0.01 to 3.0 parts by weight with respect to 100 parts by weight of the thermoplastic polyester (A) in the composition containing a thermoplastic polyester (A). Bringing the amount within this range makes it possible to maintain the effects of the additive, and inhibit the pyrolysis of the additive in a case where the additive is melt-kneaded with the thermoplastic polyester (A). The amount of additive blended in is more preferably 0.05 part by weight or more, still more preferably 0.1 part by weight or more. On the other hand, the amount is more preferably 2.0 parts by weight or less, still more preferably 1.0 part by weight or less.

The depolymerization of a composition containing a thermoplastic polyester (A) blended with the above-described additive has a problem in that the depolymerization is prone to facilitate the overreaction of a product, and decreases the yield of generation of a polyester monomer. In the second preferable aspect of the present invention, blending in an alkaline metal salt and/or an alkaline earth metal salt makes it possible to inhibit the overreaction, and to produce a polyester monomer with a high yield.

For the depolymerization of a composition containing a thermoplastic polyester (A) in the second preferable aspect of the present invention, any kind of known reaction method of a batch type, continuous type, or the like can be adopted. Examples of the batch type include: an autoclave and a vertical or horizontal type reactor, which each include a stirrer and a heating function; and a vertical or horizontal type reactor including a compression mechanism such as a cylinder in addition to a stirrer and a heating function. Examples of the continuous type include: an extruder and a tubular reactor which each include a heating function; a tubular reactor including a mixing mechanism such as a baffle; a line mixer; a vertical or horizontal type reactor; a vertical or horizontal type reactor including a stirrer; and a tower. In addition, the atmosphere in production is desirably a nonoxidizing atmosphere. The production is preferably performed under an inert atmosphere such as nitrogen, helium, and argon. A nitrogen atmosphere is preferable from the viewpoints of economical efficiency and easiness of handling.

A method of recovering a diol and a dicarboxylic acid generated using a method of depolymerizing a composition containing the thermoplastic polyester (A) in the second preferable aspect of the present invention is not particularly limited. For example, in a case where the depolymerization is performed in a batch manner, a reaction mixture is distilled out together with water after the termination of the depolymerization, and the reaction mixture can be obtained in the form of an aqueous diol solution in which a solid dicarboxylic acid is dispersed and/or precipitated. Additionally, in a case where the depolymerization is performed continuously, the reaction mixture can be obtained, with the progress of the reaction, in the form of an aqueous diol solution in which a solid dicarboxylic acid is dispersed. From the reaction mixture obtained, a solid dicarboxylic acid is separated using a known method such as solid-liquid separation, and a diol is separated from water through separation by distillation, whereby a diol and a dicarboxylic acid that have a high purity can be recovered. Additionally, if the reaction mixture has any component insoluble in water, the component is separated using a known method such as solid-liquid separation under a condition where a dicarboxylic acid is dissolved. Then, the reaction mixture can be used in a step of recovering a diol and a dicarboxylic acid.

A method of obtaining a diol having an even higher purity can be a method combined with a purification method such as the following: a method in which the diol recovered is precisely distilled; a method in which the diol, with a trace amount of sodium hydroxide added, is distilled under reduced pressure; a method in which the diol is treated with activated carbon; a method in which the diol is ion-exchanged; or a method in which the diol is recrystallized. Such a method can efficiently remove impurities that are difficult to separate through separation by distillation.

Examples of a method of obtaining a dicarboxylic acid having an even higher purity include: a method in which the dicarboxylic acid is dissolved and recrystallized in a solvent that dissolves a dicarboxylic acid; and a method in which the dicarboxylic acid is esterified to be converted into dialkyl dicarboxylate, and purified by distillation, and then, the dialkyl dicarboxylate is converted back into a dicarboxylic acid.

In a method of depolymerizing a composition containing a thermoplastic polyester (A) in the second preferable aspect of the present invention, the diol and the dicarboxylic acid can be used as polymerization raw materials for the thermoplastic polyester (A) in the same manner as a diol and a carboxylic acid that are produced from a petroleum-derived raw material. The thermoplastic polyester (A) can be produced through polycondensing a diol and/or a dicarboxylic acid directly or through allowing an ester-forming derivative(s) thereof to undergo a commonly known esterification reaction or ester interchange reaction. The second preferable aspect of the present invention makes it possible to chemically recycle a composition containing a spent, waste thermoplastic polyester (A).

In addition, the polyester obtained in this manner can be processed into and utilized as various products such as fibers, films, bottles, injection-molded articles and the like in the same manner as the thermoplastic polyester (A) produced from a petroleum-derived raw material. These products are useful for agricultural materials, gardening materials, fishing materials, civil engineering and construction materials, stationery, medical supplies, automobile components, electrical and electronic components, or other applications.

[2-3] third preferable aspect of the present invention

The third preferable aspect of the present invention is a method of producing a recycled monomer, including a step of bringing a polymer I and a polymer II into contact with subcritical water of 250 to 350°C to hydrolyze the polymers simultaneously; in which a composition contains the polymer I and the polymer II; in which the polymer I is a thermoplastic polyester (A), and the polymer II is the following: a polymer selected from the group consisting of a thermoplastic polyester (B) having a melting point different from the melting point of the thermoplastic polyester (A), a polycarbonate, and a polyurethane; or a combination of these polymers; and in which the amount of the polymer II (the total amount of the thermoplastic polyester (B), the polycarbonate, and the polyurethane) in the composition is 1 to 100 parts by weight with respect to 100 parts by weight of the polymer I.

In a method of producingng a recycled monomer in the third preferable aspect of the present invention, the recycled monomer is preferably a terephthalic acid and/or a diol. The recycled monomer being a terephthalic acid and/or a diol makes it possible to recycle a polyester available as a plastic through abundant production, thus contributing greatly to recycling resource.

The thermoplastic polyester (A) and the thermoplastic polyester (B) to be used in the third preferable aspect of the present invention are obtained by polycondensing a dicarboxylic acid or an ester-forming derivative thereof and a diol as main raw materials. Here, the main raw materials mean that the dicarboxylic acid or an ester-forming derivative thereof and the diol as the constituent units in the polymer amount to 80 mol% or more in total. The total of these constituent units is more preferably 90 mol% or more, still more preferably 95 mol% or more, most preferably 100 mol%.

Examples of the dicarboxylic acid or an ester-forming derivative thereof include, but are not particularly limited to: aromatic dicarboxylic acids such as terephthalic acid, isophthalic acid, 2,6-naphthalene dicarboxylic acid, diphenyl-4,4'-dicarboxylic acid, diphenyl ether-4,4'-dicarboxylic acid, diphenyl thio ether-4,4'-dicarboxylic acid, 5-tetrabutylphosphoniumisophthalic acid, and 5-sodiumsulfoisophthalic acid; aliphatic dicarboxylic acids such as oxalic acid, malonic acid, succinic acid, glutaric acid, adipic acid, pimelic acid, suberic acid, sebacic acid, azelaic acid, undecanedioic acid, and dodecanedioic acid; or ester-forming derivatives.

An ester-forming derivative as referred to here is a lower alkyl ester, acid anhydride, acid halide, or the like of the dicarboxylic acid described above. As a lower alkyl ester of the dicarboxylic acid, a methyl ester, ethyl ester, hydroxyethyl ester, hydroxybutyl ester, or the like is preferably used. As an acid anhydride of the dicarboxylic acid, an anhydride of dicarboxylic acids, an anhydride of a dicarboxylic acid and an acetic acid, or the like is preferably used. As a halide of the dicarboxylic acid, an acid chloride, acid bromide, acid iodide, or the like is preferably used.

The dicarboxylic acid or an ester-forming derivative is preferably an aromatic dicarboxylic acid or an ester-forming derivative thereof, which is available through abundant use as a thermoplastic polyester raw material to be used for fibers, resins, and films. The aromatic dicarboxylic acid or an ester-forming derivative is more preferably terephthalic acid, 2,6-naphthalenedicarboxylic acid, or a dimethyl ester thereof.

Examples of the diol include ethylene glycol, 1,3-propane diol, neopentyl glycol, 1,4-butane diol, 1,5-pentane diol, 1,6-hexane diol, decamethylene glycol, 2,2,4,4-tetramethyl-1,3-cyclobutane diol, cyclohexane dimethanol, xylylene glycol, polyethylene glycol, polypropylene glycol, polytetramethylene glycol, bisphenol A-ethylene oxide adduct. Among these, ethylene glycol, 1,3-propane diol and 1,4-butane diol more are more preferable from the viewpoint of the high versatility of a resulting thermoplastic polyester.

In addition, the dicarboxylic acid, an ester-forming derivative of the dicarboxylic acid, and the diol may each be used singly, or may be used in combination of two or more kinds thereof.

Examples of a thermoplastic polyester to be used in the third preferable aspect of the present invention include polyethylene terephthalate, polypropylene terephthalate, polybutylene terephthalate, polycyclohexane dimethylene terephthalate, polyethylene isophthalate, polypropylene isophthalate, polybutylene isophthalate, polycyclohexane dimethylene isophthalate, polyethylene naphthalate, polypropylene naphthalate, polybutylene naphthalate, polycyclohexane dimethylene terephthalate/polyethylene terephthalate, polyethylene isophthalate/terephthalate, polypropylene isophthalate/terephthalate, polybutylene isophthalate/terephthalate, polyethylene terephthalate/naphthalate, polypropylene terephthalate/naphthalate, polybutylene terephthalate/naphthalate, polyethylene terephthalate/5-sodium sulfoisophthalate, polypropylene terephthalate/5-sodium sulfoisophthalate, and polybutylene terephthalate/5-sodium sulfoisophthalate. Here, "/" denotes a copolymer. Among these, polyethylene terephthalate, polypropylene terephthalate, polybutylene terephthalate, and polyethylene naphthalate are preferable. Polyethylene terephthalate, polypropylene terephthalate, and polybutylene terephthalate have excellent versatility, are available through abundant recovery in the form of source materials for recycling, and thus, are more preferable.

In a method of producingng a recycled monomer according to the third preferable aspect of the present invention, the thermoplastic polyester (A) is preferably selected from polyethylene terephthalate, polypropylene terephthalate, polybutylene terephthalate, and copolymers thereof. These thermoplastic polyesters are available through abundant production, and involve a high need for recycling.

In a method of producing a recycled monomer according to the third preferable aspect of the present invention, the thermoplastic polyester (B) is preferably at least one selected from polyethylene terephthalate, polypropylene terephthalate, polybutylene terephthalate, and copolymers thereof, and is a thermoplastic polyester having a melting point different from the melting point of the thermoplastic polyester (A). These thermoplastic polyesters are available through abundant production, and involve a high need for recycling.

In a method of producing a recycled monomer according to the third preferable aspect of the present invention, both of the thermoplastic polyester (A) and the thermoplastic polyester (B) are each more preferably at least one selected from polyethylene terephthalate, polypropylene terephthalate, polybutylene terephthalate, and copolymers thereof. That the thermoplastic polyester (A) and the thermoplastic polyester (B) are both the above-described thermoplastic polyesters makes it easier to separate and purify a recycled monomer after both of the polyesters are depolymerized simultaneously.

The intrinsic viscosity of each of the thermoplastic polyester (A) and the thermoplastic polyester (B) to be used in the third preferable aspect of the present invention is preferably 0.4 to 2.0 dl/g. Here, the intrinsic viscosity of each of the thermoplastic polyester (A) and the thermoplastic polyester (B) is a value measured at 25°C, using o-chloro phenol as a solvent. In a case where the thermoplastic polyester to be used in the third preferable aspect of the present invention is polyethylene terephthalate, the intrinsic viscosity IV is preferably 0.4 to 1.5 dl/g, still more preferably 0.6 to 1.3 dl/g. In a case where the thermoplastic polyester to be used in the third preferable aspect of the present invention is polypropylene terephthalate, the intrinsic viscosity is more preferably 0.6 to 2.0 dl/g, still more preferably 0.7 to 1.6 dl/g. In a case where the thermoplastic polyester to be used in the third preferable aspect of the present invention is polybutylene terephthalate, the intrinsic viscosity is more preferably 0.6 to 2.0 dl/g, still more preferably 0.8 to 1.8 dl/g.

In a case where a composition in the third preferable aspect of the present invention contains the thermoplastic polyester (A) and the thermoplastic polyester (B), the thermoplastic polyester blended in in a larger amount is defined as the thermoplastic polyester (A). In a case where the blending amount of the thermoplastic polyester (A) and the blending amount of the thermoplastic polyester (B) are the same, the thermoplastic polyester having a higher melting point is defined as the thermoplastic polyester (A).

Examples of a polycarbonate to be used in the third preferable aspect of the present invention include: a polycarbonate obtained by interfacial polymerization of a dihydroxy compound and phosgene; a polycarbonate obtained by polymerizing a dihydroxy compound and diphenyl carbonate through molten ester interchange; a polycarbonate obtained by polymerizing a carbonate prepolymer through solid-phase ester interchange; and a polycarbonate obtained by ring-opening polymerization of a cyclic carbonate compound. Examples of the dihydroxy compound to be used here include one or more selected from 2,2'-bis(4-hydroxyphenyl)propane(bisphenol A), 4,4'-dihydroxy diphenyl alkane, 4,4'-dihydroxydiphenyl sulfone, 4,4'-dihydroxydiphenyl ether, 2,2'-bis(3,5-dimethyl-4-hydroxyphenyl)propane, 1,1'-bis(4-hydroxyphenyl)cyclohexane, and isosorbide (1,4:3,6-dianhydro-D-sorbitol). Among these, 2,2'-bis(4-hydroxyphenyl)propane(bisphenol A) is preferable.

In addition, a dihydroxy compound other than bisphenol A, for example, 4,4'-dihydroxydiphenyl alkane, 4,4'-dihydroxydiphenyl sulfone, 4,4'-dihydroxydiphenyl ether, or isosorbide (1,4:3,6-dianhydro-D-sorbitol)bisphenol A can be used simultaneously. The amount of the other dihydroxy compound to be used is preferably 10 mol% or less with respect to the total amount of the dihydroxy compounds.

The degree of polymerization of these polycarbonates is not particularly limited. The viscosity-average molecular weight (Mv) of a polycarbonate resin is preferably 10000 to 50000 from the viewpoints of excellent practical strength and facilitation of hydrolysis. The viscosity-average molecular weight is more preferably 15000 or more, still more preferably 18000 or more. On the other hand, the viscosity-average molecular weight is more preferably 40000 or less, still more preferably 35000 or less.

Here, the viscosity-average molecular weight (Mv) means a value obtained by determining a limiting viscosity [η] (unit dl/g) at a temperature of 20°C, using an Ubbelohde viscometer, and using dichloromethane as a solvent, and further by performing a calculation in accordance with the Schnell viscosity formula, i.e., $[η] = 1.23 \times 10^{-4} \times (Mv)^{0.83}$. Here, the limiting viscosity [η] is a value obtained by measuring the specific viscosity [ηsp] of each solution concentration [c] (g/dl), and performing a calculation in accordance with the following formula.

$$[η] = \lim η sp/c \ (c \rightarrow 0)$$

A polyurethane to be used in the third preferable aspect of the present invention can be any arbitrary polyurethane, subject to being a polyurethane the starting material of which is a polymer diol and diisocyanate, and is not particularly limited. In addition, the polyurethane may be the following: a polyurethane urea composed of a polymer diol, a diisocyanate, and a low-molecular-weight diamine as a chain extender; a polyurethane urethane composed of a polymer diol, a diisocyanate, and a low-molecular-weight diol as a chain extender; or a polyurethane urea obtained using, as a chain expander, a compound having a hydroxyl group and an amino group in the molecule.

The polymer diol to be used in the third preferable aspect of the present invention is preferably at least one selected from a polyether diol, a polyester diol, and a polycarbonate diol. In particular, a polyether diol is preferably used from the viewpoint of facilitation of separation after hydrolysis.

Examples of the polyether diol to be preferably used include: polyethylene oxide; polyethylene glycol; a derivative of polyethylene glycol; polypropylene glycol; polytetramethylene ether glycol (hereinafter abbreviated as PTMG); a modified PTMG that is a copolymer of tetrahydrofuran (hereinafter abbreviated as THF) and 3-methyl tetrahydrofuran; a modified PTMG that is a copolymer of THF and 2,3-dimethyl THF; a polyol having a side chain on both sides, as disclosed in JP2615131B and the like; and a random copolymer in which THF, ethylene oxide, and/or propylene oxide are arranged irregularly. These polyether diols may be used singly, or two or more kinds thereof, mixed or copolymerized, may be used.

In addition, it is also possible to use the following: a polyester diol such as butylene adipate, polycaprolactone diol, or a polyester polyol having a side chain and disclosed in JP61-26612A; a polycarbonate diol disclosed in JP02-289516B; or the like.

These polymer diols may be used singly, or two or more kinds thereof, mixed or copolymerized, may be used.

As the molecular weight of a polymer diol to be used in the third preferable aspect of the present invention, the number-average molecular weight is preferably 1000 or more and 8000 or less, more preferably 1800 or more and 6000 or less, from the viewpoint of retaining practical strength.

Examples of the diisocyanate to be used in the third preferable aspect of the present invention include: aromatic diisocyanates such as diphenylmethane diisocyanate, tolylene diisocyanate, 1,4-diisocyanate benzene, xylylene diisocyanate, and 2,6-naphthalene diisocyanate; alicyclic diisocyanates such as methylenebis(cyclohexylisocyanate), isophorone diisocyanate, methylcyclohexane 2,4-diisocyanate, methylcyclohexane 2,6-diisocyanate, cyclohexane 1,4-diisocyanate, hexahydroxylylene diisocyanate, hexahydrotolylene diisocyanate, and octahydro 1,5-naphthalene diisocyanate. An alicyclic diisocyanate can be used effectively to inhibit the yellowing of polyurethane yarn in particular. These diisocyanates may be used singly or in combination of two or more kinds thereof.

As the molecular weight of a polyurethane in the third preferable aspect of the present invention, the number-average molecular weight is preferably within the range of 30000 or more and 150000 or less from the viewpoints of retaining practical strength and facilitating depolymerization. In this regard, the molecular weight is measured by GPC (gel permeation chromatography), and calculated in terms of polystyrene.

In the third preferable aspect of the present invention, the polymer I composed of the thermoplastic polyester (A) and the polymer II composed of at least one selected from the thermoplastic polyester (B) having a melting point different from the melting point of the thermoplastic polyester (A), a polycarbonate, and a polyurethane are depolymerized simultaneously, thus bringing about an effect by which the yield of a dicarboxylic acid generated is higher than in a case where the thermoplastic polyester (A) alone is depolymerized. To achieve this effect, it is necessary to contain at least one or more polymers selected from the thermoplastic polyester (B), a polycarbonate, and a polyurethane in an amount of 1 part by weight or more with respect to 100 parts by weight of the thermoplastic polyester (A). On the other hand, the amount is more preferably 5 parts by weight or more, still more preferably 10 parts by weight or more, most preferably 20 parts by weight or more.

It is inferable that, in the hydrolysis of a polymer, the hydrolysis is promoted by the melting of the polymer or the dissolution of the polymer in water. The thermoplastic polyester (A) and at least one selected from the thermoplastic polyester (B) having a melting point different from the melting point of the thermoplastic polyester (A), a polycarbonate, and a polyurethane are heated simultaneously in the presence of water, whereby a polymer having a higher melting point is dissolved in a melt or solution of a polymer having a lower melting point (for a polymer that exhibits no melting point, the glass transition temperature thereof is defined as the melting point). It is considered that the simultaneous hydrolysis increases the yield of a monomer generated, because the hydrolysis of the polymer having a higher melting point is promoted, and/or because a depolymerized product of one polymer promotes the depolymerization of the other polymer.

Here, the melting point is defined as the temperature of an endothermic peak that appears when the polymer is cooled from a molten state to 0°C at a cooling rate of 20°C/minute, and then heated to the melting point + 40°C at a heating rate of 20°C/minute, under a nitrogen gas atmosphere, using a differential scanning calorimeter (DSC). However, in a case where two or more endothermic peaks are detected, the temperature of the endothermic peak having the largest peak intensity is regarded as the melting point. In addition, the glass transition temperature (the melting point in the third preferable aspect of the present invention) of a polymer that exhibits no melting point is defined as the temperature of the midpoint of a stepwise endothermic peak that appears when the polymer is cooled from a molten state to 0°C at a cooling rate of 20°C/minute, and then heated at a heating rate of 20°C/minute, under a nitrogen gas atmosphere. Furthermore, in the case of a polyurethane urea, the melting point thereof is higher than the pyrolytic temperature of the polymer, and thus, the melting point is difficult to accurately measure using a DSC. In this case, the intersection of the baseline of a starting weight and the tangent line of the point of maximum slope that are on a TGA curve is defined as a decomposition start temperature (the melting point in the third preferable aspect of the present invention), in which the TGA curve is obtained by heating the polymer at a heating rate of 20°C/minute under a nitrogen gas atmosphere, using a thermogravimetric analyzer (TGA), and in which the maximum slope is obtained after a weight decrease due to pyrolysis.

In the third preferable aspect of the present invention, the difference between the melting point of the thermoplastic polyester (A) and the melting point of the thermoplastic polyester (B), a polycarbonate, or a polyurethane is preferably 10°C or more. The difference in the melting point between the thermoplastic polyester (A) and the thermoplastic polyester (B) is more preferably 10°C or more. Combining polymers having a larger difference in the melting point therebetween makes it easier to dissolve the polymer having a higher melting point, and makes it possible to promote hydrolysis. The difference in the melting point is more preferably 20°C or more, still more preferably 30°C or more.

A polymer to be used in the third preferable aspect of the present invention may contain a polymer that is other than a thermoplastic polyester, a polycarbonate, and a polyurethane. Examples include a polyamide, polyolefin, modified polyphenylene ether, polysulfone, polyketone, polyether imide, polyalylate, polyether sulfone, polyether ketone, polythioether ketone, polyether ether ketone, polyimide, polyamide imide, polytetrafluoroethylene, and polyphenylene sulfide. Two or more kinds of these may be contained. The polymer other than a thermoplastic polyester, a polycarbonate, and a polyurethane is preferably in an amount of less than 50 parts by weight with respect to 100 parts by weight of the polymer components in the composition in the third preferable aspect of the present invention. The amount is more preferably 40 parts by weight or less, still more preferably 30 parts by weight or less.

In addition, a composition to be used in the third preferable aspect of the present invention may contain an additive such as a filler, nucleating agent, plasticizer, UV inhibitor, mold release agent, flame retardant, colorant (for example, a pigment or a dye), lubricant, antistatic agent, or antioxidant besides a polymer.

Examples of the filler include glass fiber, carbon fiber, potassium titanate whisker, zinc oxide whisker, aluminium borate whisker, aramid fiber, alumina fiber, silicon carbide fiber, ceramic fiber, asbestos fiber, gypsum fiber, metal fiber, glass sheet, wollastonite, zeolite, sericite, kaolin, mica, talc, clay, pyrophyllite, bentonite, montmorillonite, hectorite, synthesize mica, asbestos, graphite, aluminosilicate, alumina, silica, magnesium oxide, zirconium oxide, titanium oxide, iron oxide, calcium carbonate, magnesium carbonate, dolomite, calcium sulfate, barium sulfate, magnesium hydroxide, calcium hydroxide, aluminium hydroxide, glass beads, hollow glass beads, ceramic beads, boron nitride, silicon carbide, and wollastonite.

The fibrous filler is preferably glass fiber or carbon fiber from the viewpoint of separability after hydrolysis due to contact with subcritical water. The fibrous filler is not particularly limited to any cross-sectional shape, and may be a fiber having any of a circular shape and an oblate shape. In the third preferable aspect of the present invention, the amount of the fibrous filler in the polymer composition is preferably 50 wt% or less, more preferably 40 wt% or less, still more preferably 30 wt% or less, in the composition.

In the third preferable aspect of the present invention, the polymer I and the polymer II in the composition are brought into contact with subcritical water of 250 to 350°C to be hydrolyzed simultaneously. Water, when brought up to a pressure of 22.1 MPa and a temperature of 374.2°C, presents a state that is neither liquid nor gaseous. Water in this state is referred to as supercritical water. In addition, hot water in the near region of the critical point, i.e., water having a temperature and a pressure that are slightly lower than the critical point is referred to as subcritical water. Despite being water, the subcritical water is characterized by having (i) a low dielectric constant and (ii) a high ionic product. The dielectric constant and ionic product of the subcritical water depend on temperature and the partial pressure of the water, and can be controlled. Despite being water, the water having a low dielectric constant is allowed to be an excellent solvent for an organic compound. In addition, the high ionic product allows the hydrogen ion concentration and the hydroxide ion concentration to increase. Accordingly, the subcritical water has an excellent hydrolytic action.

Water to be used as a raw material for the subcritical water is not particularly limited, and may be any water, such as tap water, deionized water, distilled water, or well water. Deionized water or distilled water is preferably used from the viewpoint of inhibiting a side reaction due to the influence of the coexistent salt.

The hydrolysis in the third preferable aspect of the present invention needs to have a step of being conducted at 250°C or more and 350°C or less. Bringing the temperature within this range promotes hydrolysis, and inhibits the overreaction of a product, enabling the yield of a polyester monomer to be enhanced. The third preferable aspect of the present invention does not include a step of hydrolysis at a temperature higher than 350°C. The temperature is preferably 260°C or more, more preferably 270°C or more, still more preferably 280°C or more. On the other hand, the temperature is preferably 320°C or less, more preferably 310°C or less, still more preferably 300°C or less.

Another embodiment of a method of producing a recycled monomer in the third preferable aspect of the present invention includes a step of bringing a polymer I and a polymer II into contact with subcritical water of 200 to 350°C under a condition where an alkaline component is blended, to hydrolyze the polymers simultaneously; in which a composition containing a polymer contains the polymer I and the polymer II; in which the polymer I is a thermoplastic polyester (A), and the polymer II is the following: a polymer selected from the group consisting of a thermoplastic polyester (B) having a melting point different from the melting point of the thermoplastic polyester (A), a polycarbonate, and a polyurethane; or a combination of these polymers; and in which the amount of the polymer II (the total amount of the thermoplastic polyester (B), the polycarbonate, and/or the polyurethane) in the composition is 1 to 100 parts by weight with respect to 100 parts by weight of the polymer I. In another embodiment of a method of producing a recycled monomer in the third preferable aspect of the present invention, further blending in an alkaline component makes it possible to expand the temperature of hydrolysis to 200 to 350°C. That is, blending in an alkaline component enables the hydrolysis to be promoted at an even lower temperature. The temperature of hydrolysis in the case of blending in an alkaline component is preferably 210°C or more, still more preferably 220°C or more. On the other hand, the temperature of hydrolysis in the case of blending in an alkaline component is preferably 300°C or less, still more preferably 290°C or less.

In a method of producing a recycled monomer in the third preferable aspect of the present invention, the composition may include a composition containing the polymer I and the polymer II and/or include a mixture of a composition containing the polymer I and a composition containing the polymer II. That is, in the third preferable aspect of the present invention, a composition to be hydrolyzed may be a composition including any one of the following: a composition containing the polymer I and the polymer II; and a mixture of a composition containing the polymer I and a composition containing the polymer II; or may be a composition including both of them. Here, the "composition containing the polymer I and the polymer II" refers to a composition obtained, for example, by kneading the polymer I and the polymer II (encompassing the polymer I or/and the polymer II that contain(s) another arbitrary component) at a temperature not less than the melting point of any one of the polymers (for a polymer having no melting point, a temperature not lower than the glass transition temperature thereof). In addition, the "mixture of a composition containing the polymer I and a composition containing the polymer II" refers to, for example, a mixture obtained by mixing a composition (for example, pellets) containing the polymer I (that may contain another arbitrary component) and a composition (for example, pellets) containing the polymer II (that may contain another arbitrary component), in which the compositions are in a solid state at a temperature lower than the melting points of both polymers (for a polymer having no melting point, a temperature lower than the glass transition temperature thereof).

In addition, in the hydrolysis in the third preferable aspect of the present invention, 100 parts by weight or more and 900 parts by weight or less of water is preferably blended with 100 parts by weight of the polymer components in the composition. Using this range makes it possible to promote hydrolysis, and restrain energy consumption for heating water. The amount of water to be blended in is more preferably 150 parts by weight or more, still more preferably 200 parts by weight or more. On the other hand, the amount of water to be blended in is more preferably 500 parts by weight or less, still more preferably 350 parts by weight or less.

Furthermore, in the third preferable aspect of the present invention, the pressure in the step of bringing about the contact with the subcritical water of 250 to 350°C to cause simultaneous hydrolysis is preferably not lower than the saturated vapor pressure of water, specifically preferably 4.0 MPa to 30 MPa. Using this range makes it possible to increase the ion concentration of the water, and promote hydrolysis. The pressure is more preferably 5.0 MPa or more, still more preferably 6.0 MPa or more. On the other hand, the pressure is more preferably 25 MPa or less, still more preferably 22 MPa or less. To achieve the pressure ranges, for example, a method of pressurizing the inside of a pressure container can be used. To pressurize the inside of a pressure container, for example, a method of enclosing gas in addition to the water or a method of introducing high-pressure water can be used. Examples of such a gas include air, argon, and nitrogen. An inert gas such as nitrogen or argon is preferably used from the viewpoint of inhibiting a side reaction such as oxidation. The degree of pressurization with gas or high-pressure water is not particularly limited in being set at a pressure of interest, and is preferably 0.3 MPa or more. In a case where high-pressure water is used for pressurization, the inside of the pressure container is also enabled to be gas-free.

In the third preferable aspect of the present invention, it is preferable to further blending in an alkaline component in the above-described step. Blending in an alkaline component makes it possible to inhibit the overreaction of a product from hydrolysis, and enhance the yield of the monomer. Examples of the alkaline component include: alkaline metals such as lithium, sodium, a potassium; alkaline earth metals such as calcium; hydroxides such as ammonium; carbonates; bicarbonate; and amines. In particular, at least one selected from sodium hydroxide, potassium hydroxide, and calcium hydroxide is preferable. The amount of the alkaline component blending in is preferably 0.01 part by weight or more and 50 parts by weight or less with respect to 100 parts by weight of the polymer component in the composition. The amount is more preferably 20 parts by weight or more, still more preferably 30 parts by weight or more. On the other hand, the amount is more preferably 45 parts by weight or less, still more preferably 40 parts by weight or less. Blending in an alkaline component causes the hydrolysis of the thermoplastic polyester to generate a dicarboxylate. Through treatment with an acidic aqueous solution, a dicarboxylate can be converted into a dicarboxylic acid that is a thermoplastic polyester raw material.

For the hydrolysis of a composition containing a polymer in the third preferable aspect of the present invention, any kind of known reaction method of a batch type, continuous type, or the like can be adopted. Examples of the batch type include: an autoclave and a vertical or horizontal type reactor, which each include a stirrer and a heating function; and a vertical or horizontal type reactor including a compression mechanism such as a cylinder in addition to a stirrer and a heating function. Examples of the continuous type include: an extruder and a tubular reactor which each include a heating function; a tubular reactor including a mixing mechanism such as a baffle; a line mixer; a vertical or horizontal type reactor; a vertical or horizontal type reactor including a stirrer; and a tower. In addition, the atmosphere in production is desirably a nonoxidizing atmosphere. The production is preferably performed under an inert atmosphere such as nitrogen, helium, and argon. A nitrogen atmosphere is preferable from the viewpoints of economical efficiency and easiness of handling.

A method of recovering a diol and a dicarboxylic acid generated through the hydrolysis of a composition in the third preferable aspect of the present invention is not particularly limited. For example, in a case where terephthalic acid is used as a raw material for the thermoplastic polyester, solid terephthalic acid dispersed and/or precipitated in water is obtained in the form of a reaction mixture after the hydrolysis. The reaction mixture obtained undergoes solid-liquid separation, whereby solid terephthalic acid can be separated. Examples of a method of obtaining a terephthalic acid having an ever higher purity include: a method in which the terephthalic acid is dissolved and recrystallized in a solvent that dissolves a terephthalic acid; and a method in which the terephthalic acid is esterified to be converted into dialkyl terephthalate, and purified by distillation, and then, the dialkyl terephthalate is converted back into a terephthalic acid. Additionally, in a case where a water-soluble diol is used as a raw material for the thermoplastic polyester, the aqueous solution can undergo separation by distillation after the hydrolysis, and be separated into a diol and water. A method of obtaining a diol having an even higher purity can be a method combined with a purification method such as the following: a method in which the diol recovered is precisely distilled; a method in which the diol, with a trace amount of sodium hydroxide added, is distilled under reduced pressure; a method in which the diol is treated with activated carbon; a method in which the diol is ion-exchanged; or a method in which the diol is recrystallized. Such a method can efficiently remove impurities that are difficult to separate through separation by distillation.

In the third preferable aspect of the present invention, hydrolysates of a polycarbonate and a polyurethane can be generated in addition to hydrolysates of a thermoplastic polyester. Specifically, the hydrolysis of a thermoplastic polyester and a polycarbonate generates a diol plus a dicarboxylic acid and a dihydroxy compound, respectively. Thus, the products generated can be reused as raw materials for a polymer. On the other hand, the hydrolysis of a polyurethane generates a diamine, a polymer diol, and the like. A diamine can be converted into isocyanate to be reused as a raw material for a polyurethane. In addition, a polymer diol can be reused as a raw material for a polyurethane.

In a method of producing a thermoplastic polyester in the third preferable aspect of the present invention, a thermoplastic polyester is produced, using, as a polymerization raw material(s), a dicarboxylic acid and/or a diol obtained by using a method of producing a recycled monomer in the third preferable aspect of the present invention. The dicarboxylic acid and/or the diol obtained using a method of producing a recycled monomer in the third preferable aspect of the present invention are preferably a dicarboxylic acid and/or a diol obtained through the hydrolysis of the thermoplastic polyester (A) and/or the thermoplastic polyester (B). For example, a thermoplastic polyester can be produced through the first step composed of esterification of a dicarboxylic acid and a diol and the subsequent second step of polycondensation. In addition, a thermoplastic polyester can also be produced through the first step composed of ester interchange between a dialkyl dicarboxylate-obtained by chemically converting a dicarboxylic acid-and a diol and the subsequent second step of polycondensation.

In a method of producing a recycled monomer in the third preferable aspect of the present invention, the composition is preferably a waste. A monomer is regenerated through hydrolysis of a spent waste under a specific condition, repolymerized, and thus, can be reused as a thermoplastic polyester.

Furthermore, together with the thermoplastic polyester regenerated, a dye, additive of any kind, fibrous filler, non-fibrous filler, another polymer, and/or the like can be blended to produce a composition containing a thermoplastic polyester. Examples of the additive include antioxidants, weather-resistant agents, mold release agents, and nucleating agents.

A thermoplastic polyester and a composition containing a thermoplastic polyester that are obtained using a method of producing a thermoplastic polyester in the third preferable aspect of the present invention can be utilized in the production of various products of a thermoplastic polyester and various products of a composition containing a plastic polyester, examples of such products including: fibers, films, and bottles obtained through melt spinning, melt film-formation, melt extrusion molding, or the like; and molded articles obtained through injection molding, blow molding, extrusion molding, or the like. These products are useful for agricultural materials, gardening materials, fishing materials, civil engineering and construction materials, industrial films, sheets, stationery, medical supplies, clothing, automobile components, electrical and electronic components, or other applications.

**In** a method of producing a thermoplastic polyester product in the third preferable aspect of the present invention, a thermoplastic polyester product in the form of a fiber, a film, or a molded article is produced, using, as a raw material, a thermoplastic polyester obtained using a method of producing a thermoplastic polyester in the third preferable aspect of the present invention. Additionally, in a method of producing a product of a composition containing a thermoplastic polyester in the third preferable aspect of the present invention, a product of a composition containing a thermoplastic polyester obtained using a method of producing a thermoplastic polyester in the third preferable aspect of the present invention is produced. Examples of the thermoplastic polyester product and the product containing a thermoplastic polyester and examples of the method of producing the thermoplastic polyester product and the product containing the thermoplastic polyester are as described above.

Apparatus of producing recycled monomer

An apparatus of producing a recycled monomer according to the present invention includes: means (L) for heating, pressurizing, and supplying a composition containing a thermoplastic polymer; means (M) for generating and supplying subcritical water; means (N) for mixing the composition containing a thermoplastic polymer and supplied from the means (L) and the subcritical water supplied from the means (M); and a continuous reactor (O) for conducting hydrolysis of the thermoplastic polymer. Below, each constituent will be described.

(1) Composition containing thermoplastic polymer

In an apparatus of producing a recycled monomer according to the present invention, a composition containing a thermoplastic polymer is not limited to any kind, subject to containing a hydrolyzable polymer. Examples of the hydrolyzable polymer include thermoplastic polyamides and thermoplastic polyesters. Examples of the thermoplastic polyamide include polyamide 6 and polyamide 66. Examples of the thermoplastic polyester include polyethylene terephthalate and polybutylene terephthalate. A thermoplastic polyamide is widely used as an engineering plastic and a fiber product. In addition, a thermoplastic polyester is widely used as a general-purpose plastic bottle and a fiber product. Hence, these hydrolyzable polymers are preferable from the viewpoints of easiness of recovery of source materials and easiness of hydrolysis.

In an apparatus of producing a recycled monomer according to the present invention, a composition containing a thermoplastic polymer may be a waste of a resin molded article.

In a case where the thermoplastic polymer is a thermoplastic polyamide, examples of the waste of a resin molded article containing a thermoplastic polyamide include: polyamide products; industrial wastes generated in production processes of polyamide products; and spent wastes of polyamide products. Examples of the polyamide product include: clothing fiber structures such as old clothes, uniforms, sports wear, and inner wear; industrial fiber structures such as curtains, carpets, ropes, nets, belts, seats, an air bags; automobile components; molded components for housing materials; electrical and electronic molded components; aircraft components; industrial machine components; film products; extrusion-molded articles; in-situ polymerization molded articles; and RIM molded articles. Furthermore, product scraps, pellet scraps, lumpy scraps, cutoffs from cutting processing, and the like generated in production processes of these correspond to objects as wastes.

In a case where the thermoplastic polymer is a thermoplastic polyester, examples of the waste of a resin molded article containing a thermoplastic polyester include thermoplastic polyester products, industrial wastes generated in production processes of a thermoplastic polyester product, spent wastes of a thermoplastic polyester product, and the like. Examples of the thermoplastic polyester product include: containers such as drink bottles and seasoning bottles; sheet products such as food trays, blister packs, partitions for food, and industrial trays; film products such as packaging films, optical functional films, magnetic tapes, and insulating materials; clothing fiber structures such as old clothes, uniforms, sports wear, and inner wear; industrial fiber structures such as curtains, carpets, nets, belts, and seats; and molded articles such as automobile components, electrical and electronic components, construction components and materials, daily necessities, household sundries, and sanitary goods. Furthermore, product scraps, pellet scraps, lumpy scraps, and the like generated in production processes of these correspond to objects as wastes.

In an apparatus of producing a recycled monomer according to the present invention, a composition containing a thermoplastic polymer may contain a fibrous filler. Here, the fibrous filler may be any filler having a fibrous form. Specific examples include: glass fibers; polyacrylonitrile (PAN)-based or pitch-based carbon fibers; metal fibers such as stainless-steel fibers, aluminium fibers, and brass fibers; organic fibers such as polyester fibers and aromatic polyamide fibers; fibrous or whisker fillers such as gypsum fibers, ceramic fibers, asbestos fibers, zirconia fibers, alumina fibers, silica fibers, titanium oxide fibers, silicon carbide fibers, rock wool, titanic acid potassium whisker, silicon nitride whisker, wollastonite, and alumina silicate; glass fibers coated with one or more metals selected from the group consisting of nickel, copper, cobalt, silver, aluminium, iron, and alloys thereof; carbon fibers; aromatic polyamide fibers; and polyester fibers. Two or more kinds of these may be contained. The amount of the fibrous filler is preferably 1 to 200 parts by mass with respect to 100 parts by mass of the thermoplastic polymer as a main component.

In an apparatus of producing a recycled monomer according to the present invention, a composition containing a thermoplastic polymer may further contain a filler, additive of any kind, and/or the like other than the fibrous filler, to the extent that an object of the invention of an apparatus of producing a recycled monomer according to the present invention is not impaired. A filler other than the fibrous filler, i.e., a non-fibrous filler may be any one of an organic filler and an inorganic filler. Two or more of kinds thereof may be contained. Examples of the non-fibrous filler include: non-swellable silicates such as talc, wollastonite, zeolite, sericite, mica, kaolin, clay, pyrophyllite, bentonite, asbestos, alumina silicate, and calcium silicate; swellable layered silicates such as swellable mica of Li fluorine taeniolite, Na fluorine taeniolite, Na fluorotetrasilicic mica, and Li fluorotetrasilicic mica; metal oxides such as silicon oxide, magnesium oxide, alumina, silica, diatomaceous earth, zirconium oxide, titanium oxide, iron oxide, zinc oxide, calcium oxide, tin oxide, and antimony oxide; metal carbonates such as calcium carbonate, magnesium carbonate, zinc carbonate, barium carbonate, dolomite, and hydrotalcite; metal sulfates such as calcium sulfate and barium sulfate; metal hydroxides such as magnesium hydroxide, calcium hydroxide, aluminium hydroxide, and alkaline magnesium carbonate; smectite clay minerals and vermiculite such as montmorillonite, beidellite, nontronite, saponite, hectorite, and sauconite; various clay minerals such as halloysite, kanemite, kenyte, zirconium phosphate, and titanium phosphate; glass beads; glass flake; ceramic beads; boron nitride; aluminium nitride; silicon carbide; calcium phosphate; carbon black; and graphite. **In** the swellable layered silicate, an exchangeable cation present between layers may be exchanged with an organic onium ion. Examples of the organic onium ion include an ammonium ion, phosphonium ion, and sulfonium ion.

Specific examples of the various additives include: antioxidants and heat stabilizers (those which are hindered phenol-based, hydroquinone-based, or phosphite-based; substitution products thereof; copper halides; iodine compounds; or the like); weather-resistant agents (that are, for example, resorcinol-based, salicylatebased, benzotriazole-based, benzophenone-based, or hindered amine-based); mold release agents and lubricants (aliphatic alcohols, aliphatic amides, aliphatic bisamides, bisureas, polyethylene wax, and the like); pigments (cadmium sulfide, phthalocyanine, carbon black, and the like); dyes (nigrosin, aniline black, and the like); plasticizers (octyl *p*-oxybenzoate, *N*-butyl benzene sulfone amide, and the like); antistatic agents (alkyl sulfate type anion antistatic agents; quaternary ammonium salt type cation antistatic agents; nonionic antistatic agents such as polyoxyethylene sorbitan monostearate; betaine zwitterionic antistatic agent; and the like); and flame retardants (melamine cyanurate; hydroxides such as magnesium hydroxides and aluminium hydroxide; phosphorus flame retardants such as ammonium polyphosphate, melamine polyphosphate, and phosphinic acid metal salts; polystyrene bromide, brominated polyphenylene oxide, brominated polycarbonate, epoxy bromide resin, and combinations of these bromine flame retardants and antimony trioxide; and the like). In a case where such an additive is contained, the amount thereof is preferably 10 parts by mass or less, more preferably 1 part by mass or less with respect to 100 parts by mass of the thermoplastic polymer component as a main component.

(2) Means (L) for heating, pressurizing, and supplying composition containing thermoplastic polymer

In an apparatus of producing a recycled monomer according to the present invention, an apparatus of producing a recycled monomer includes means (L) (herein simply referred to as "means (L)" in some cases) for heating, pressurizing, and supplying a composition containing a thermoplastic polymer. Examples of the means (L) include: a device for performing melting under heating with an electric heater, and pressurizing with a gear pump; a device for simultaneously performing heating and pressurizing with an extruder; a device including a combination of an extruder and a gear pump; and a device for further heating, with an electric heater, a thermoplastic polymer discharged from an extruder. Among these, the means (L) is a preferably a device including an extruder. With the means (L) that is a device including an extruder, it is easier to stabilize a pressure at which a thermoplastic polymer is extruded.

A temperature at which the composition containing a thermoplastic polymer is heated is preferably raised to a temperature at which the composition containing a thermoplastic polymer is melted. Heating the composition up to a temperature at which the composition containing a thermoplastic polymer is melted makes it possible to increase the area of contact with subcritical water, and allows the reaction to progress rapidly.

(3) Means (M) for generating and supplying subcritical water

An apparatus of producing a recycled monomer according to the present invention includes means (M) for generating and supplying subcritical water (simply referred to as "means (M)" in some cases with an apparatus of producing a recycled monomer according to the present invention). Water, when brought up to a pressure of 22.1 MPa and a temperature of 374.2°C, presents a state that is neither liquid nor gaseous. Water in this state is referred to as supercritical water. In addition, hot water in the near region of the critical point, i.e., water having a temperature and a pressure that are slightly lower than the critical point is referred to as subcritical water. Despite being water, the subcritical water is characterized by having (i) a low dielectric constant and (ii) a high ionic product. The dielectric constant and ionic product of the subcritical water depend on temperature and the partial pressure of the water, and can be controlled. Despite being water, the subcritical water having a low dielectric constant is allowed to be an excellent solvent for an organic compound. In addition, the high ionic subcritical water allows the hydrogen ion concentration and the hydroxide ion concentration to increase. Accordingly, the subcritical water has an excellent hydrolytic action.

Water to be used as a raw material for the subcritical water is not particularly limited, and may be any water, such as tap water, deionized water, distilled water, or well water. Deionized water or distilled water is preferably used from the viewpoint of inhibiting a side reaction due to the influence of the coexistent salt.

Examples of the means (M) include a device with which water is introduced under pressure into high-pressure apparatus, using a diaphragm pump, Mohno Pump, gear pump, plunger pump, or the like, and then heated to a predetermined temperature, using a heat exchanger, electric heater, heating furnace, or the like. A device not taken for example here may be used.

The subcritical water in an apparatus of producing a recycled monomer according to the present invention is preferably heated to a temperature not lower than the melting point of the composition containing a thermoplastic polymer. Allowing the subcritical water to be heated to a temperature not lower than the melting point of the composition containing a thermoplastic polymer makes it possible to disperse, in droplets, the composition containing a thermoplastic polymer, increase the interfacial area, and enhance the reaction efficiency. The subcritical water is brought into contact with the composition containing a thermoplastic polymer, causing a hydrolytic reaction, the endothermic and exothermic actions of which reaction then cause the temperature of the reaction system to rise and fall. Thus, the temperature of the subcritical water is preferably controlled in accordance with a composition to be used as the composition containing a thermoplastic polymer. In a case where the temperature of the reaction system is low, the hydrolytic reaction is insufficient. In a case where the temperature of the reaction system is high, the reaction is prone to be overreaction. Thus, any of the cases leads to a decrease in the yield of a recycled monomer. Considering the degree of the endothermic and exothermic actions of the hydrolytic reaction, the supply temperature of the subcritical water is preferably less than $\pm 20$°C, more preferably less than $\pm 10$°C, still more preferably less than $\pm 5$°C, with respect to the temperature in the inlet of the continuous reactor (O).

It is preferable that the subcritical water is retained in liquid form in the continuous reactor (O). Thus, in a preferable example, the supply pressure of the subcritical water is a pressure higher than the saturated vapor pressure of water at the highest temperature of the subcritical water. The vapor pressure of water is, for example 6.4 MPa at 280°C, 8.6 MPa at 300°C, 11.3 MPa at 320°C, or 16.5 MPa at 350°C. Thus, for the subcritical water to be retained in liquid form, a pressure not lower than the vapor pressure needs to be applied.

(4) Means (N) for mixing composition containing thermoplastic polymer with subcritical water

An apparatus of producing a recycled monomer according to the present invention includes means (N) (simply referred to as "means (N)" in some cases with an apparatus of producing a recycled monomer according to the present invention) for mixing the following: the composition containing a thermoplastic polymer, supplied from the means (L); and the subcritical water supplied from the means (M). Examples of the means (N) include mixing vessels, flow paths with a screw, jet mixers, homogenizers, and static mixers. The means (N) is preferably a static mixer particularly from the viewpoints of having no movable part, and causing a smaller pressure loss.

An example of the characteristics of an apparatus of producing a recycled monomer according to the present invention is that subcritical water pressurized and heated preliminarily and a composition containing a thermoplastic polymer are mixed rapidly. An apparatus of producing a recycled monomer according to the present invention can control the temperature during the time of contact between a composition containing a thermoplastic polymer and subcritical water, thus enabling the reaction starting temperature to be precisely controlled, compared with the following conventional apparatuses: an apparatus of producing a recycled monomer, including a batch type reactor that heats a reaction vessel in which water and a composition containing a thermoplastic polymer are well mixed preliminarily and then allowed to react under heating; and a continuous type apparatus of producing a recycled monomer, in which apparatus slurry of a composition containing a thermoplastic polymer is dispersed in water, and allowed to react under pressurization and heating. As a result, it is possible to inhibit the overdecomposition of a monomer, simultaneously allow the depolymerization of a thermoplastic polymer to progress in a short time, and thus enhance the yield of the monomer. In addition, the apparatus can start the reaction instantly, and thus, can decrease the retention time in the continuous reactor (O). This results in making it possible to decrease the capacity of the continuous reactor (O), leading to a decrease in the apparatus cost.

(5) Continuous reactor (O); device (P) for monitoring inside temperature of upstream section and downstream section of continuous reactor (O); and temperature control mechanism (Q) for heating and/or cooling continuous reactor (O)

An apparatus of producing a recycled monomer according to the present invention includes: a device (P) (simply referred to as a "device (P)" in some cases with an apparatus of producing a recycled monomer according to the present invention) for monitoring the inside temperature of each of the upstream section and downstream section of the continuous reactor (O); and a temperature control mechanism (Q) (simply referred to as a "temperature control mechanism (Q)" in some cases with an apparatus of producing a recycled monomer according to the present invention) for heating and/or cooling the continuous reactor (O). The temperature control mechanism (Q) is preferably operated in accordance with the device (P) to control the inside temperature of the continuous reactor (O).

An apparatus of producing a recycled monomer according to the present invention includes the continuous reactor (O) for conducting hydrolysis of a thermoplastic polymer. The continuous reactor (O) can continuously treat a composition containing a thermoplastic polymer, and thus, makes it possible to reduce the time for preliminary preparation and withdrawal both necessary for batch treatment, and to decrease the capacity of the continuous reactor (O).

Examples of the continuous reactor (O) include tubular reactors, tower reactors, and multistage mixing vessel reactors. The continuous reactor (O) may be provided with a stirrer therein. The continuous reactor (O) may have a plurality of containers arranged in a multistage manner, or may have a plurality of flow paths arranged in parallel as a multitubular continuous reactor does. The continuous reactor (O) is preferably a tubular continuous reactor among these. A tubular continuous reactor has no movable part, and is simple, making it easy to control a reaction temperature that is important for using subcritical water to depolymerize a composition containing a thermoplastic polymer.

In one embodiment of an apparatus of producing a recycled monomer according to the present invention, it is preferable to include the device (P) for monitoring the inside temperature of the upstream section and downstream section of the continuous reactor (O). Examples of the device for monitoring the inside temperature include thermocouples, platinum resistance thermometers, bimetallic thermometers, radiation thermometers, thermographs, and thermistors. The upstream section of the continuous reactor (O) means the position of the upstream 1/2 or smaller portion of the total reaction volume of the continuous reactor (O). The upstream section of the continuous reactor (O) is preferably the position of the upstream 1/3 or smaller portion of the total reaction volume of the continuous reactor (O), more preferably the position of the upstream 1/4 or smaller portion, still more preferably the position of the upstream 1/5 or smaller portion. In addition, the downstream section of the continuous reactor means the position of the downstream 1/2 or smaller portion of the total reaction volume of the continuous reactor (O). The downstream section of the continuous reactor is preferably the position of the downstream 1/3 or smaller portion of the total reaction volume of the continuous reactor (O), more preferably the position of the downstream 1/4 or smaller portion, still more preferably the position of the downstream 1/5 or smaller portion. A plurality of devices for monitoring the inside temperature may be installed in the reactor. The total reaction volume means the total of the reaction capacity from the inlet to the outlet of the continuous reactor (O). In this regard, in a case where the continuous reactor (O) has a plurality of containers arranged in a multistage manner, the total reaction volume is the volume of the total of the plurality of containers.

On the basis of the inside temperature of the upstream section of the continuous reactor (O), the temperature for the means (L) and the means (M) may be controlled. Controlling a supply device on the basis of the inside temperature of the upstream section of the continuous reactor (O) makes it possible to precisely control the temperature in the upstream section of the continuous reactor. Accordingly, it is possible to prevent a monomer from being overdecomposed by overheating in the initial stage of reaction, and prevent the reaction rate of depolymerization from being decreased by insufficient heating.

It is preferable that an apparatus of producing a recycled monomer according to the present invention includes a temperature control mechanism (Q) for heating and/or cooling the continuous reactor (O), and that the temperature control mechanism (Q) is operated in accordance with the device (P) to control the inside temperature of the continuous reactor (O). Controlling the temperature in the continuous reactor on the basis of the inside temperature of each of the upstream section and downstream section of the continuous reactor (O) in such a manner makes it possible to precisely control the temperature in the continuous reactor (O). Accordingly, it is possible to prevent a monomer particularly from being overdecomposed by overheating in the later stage of reaction, prevent an oligomer from being generated by repolymerization, and prevent the reaction rate of depolymerization from being decreased by insufficient heating.

It is preferable that, in an apparatus of producing a recycled monomer according to the present invention, a plurality of temperature control mechanisms (Q) are installed, and that the plurality of temperature control mechanisms (Q) are operated in accordance with the device (P). Installing the plurality of temperature control mechanisms (Q) makes it possible to achieve finer temperature control in accordance with the extent of reaction of hydrolysis in the upstream section and downstream section of the continuous reactor (O).

It is preferable that, in an apparatus of producing a recycled monomer according to the present invention, the temperature control mechanism (Q) is controlled in such a manner that the inside temperature of the downstream section of the continuous reactor (O) is 10°C or more lower than the inside temperature of the upstream section. By adopting such a scheme, the temperature is made higher in the initial stage of reaction than in the later stage of reaction in the hydrolysis of a thermoplastic polymer, thus promoting the hydrolysis of the thermoplastic polymer contained in a large amount in a reaction solution in the initial stage of reaction. On the other hand, the reaction solution in the later stage of reaction contains a large amount of the recycled monomer hydrolyzed, and thus, making the temperature in the later stage lower than in the initial stage of reaction can make it easier to inhibit the repolymerization of the recycled monomer. That is, adopting such a scheme can make it easier to achieve both hydrolyzing a thermoplastic polymer with high efficiency and obtaining a recycled monomer with a high yield. The inside temperature of the downstream section of the continuous reactor (O) is more preferably 20°C or more lower, still more preferably 30°C or more lower, than the inside temperature of the upstream section. In addition, the inside temperature of the downstream section of the continuous reactor (O) is not particularly limited, and is usually 100°C or less lower, preferably 80°C or less lower, more preferably 60°C or less lower, than the inside temperature of the upstream section, from the viewpoint of keeping the reaction rate of the hydrolysis of a thermoplastic polymer.

In a case where a composition that contains a thermoplastic polymer, and is to be depolymerized is a waste, it is usually difficult for the waste to have a constant quality. Thus, the variation of the composition of a waste results in external disturbance, and affects the temperature in the reactor in some cases. Even in such a case, heating and/or cooling the continuous reactor (O) to control the reaction temperature on the basis of the inside temperature of the continuous reactor (O) makes it possible to instantly control the reaction temperature, as described in the present invention, even if external disturbance such as the variation of the composition of a composition containing a thermoplastic polymer has occurred.

Examples of the means for heating the continuous reactor (O) include, but are not particularly limited to: a method in which the continuous reactor (O) is provided with a jacket or a shell to be heated using a heating medium such as steam; and a method in which heating is performed using an electric heater. Examples of the method of cooling the continuous reactor (O) include, but are not particularly limited to: a method in which the continuous reactor (O) is provided with a jacket or a shell to be cooled with a heating medium/cooling medium having a temperature lower than the inside temperature of the continuous reactor (O); and a method in which the continuous reactor (O) is cooled by aerating the surface thereof with air.

In a case where the continuous reactor (O) is a tubular continuous reactor, the tubular continuous reactor having a larger inner diameter makes it possible to increase the rate of a flow treatable per surface area of the tubular continuous reactor, but decreases the area of heat transfer, and thus, is prone to cause a temperature distribution in the axial direction or the radial direction. Accordingly, it becomes more important that a device for monitoring the inside temperature of the continuous reactor and a temperature control device for heating and/or cooling the continuous reactor (O), both described in the present invention, are installed. The inner diameter of the tubular continuous reactor is preferably 1 cm or more, more preferably 2 cm or more.

Assuming that the mass flow ratio of the subcritical water to a thermoplastic polymer component in the composition containing a thermoplastic polymer is X: 1, X is preferably less than 6. Water has a specific heat capacity of 4.3 kJ/kg·K and a heat of vaporization of 2250 kJ/kg, which are very high, compared with another organic solvent. From the viewpoint of energy saving, it is preferable to bring X to less than 6, and to decrease the amount of water to be used. In addition, bringing X to less than 6 increases a temperature change caused inside the continuous reactor (O) by the heat of reaction of the depolymerization of a composition containing a thermoplastic polymer, thus makes it more important to perform temperature control on the basis of the inside temperature of the continuous reactor (O) as described in the present invention, and hence, is preferable. Furthermore, bringing X to less than 6 can increase the amount of treatment of a composition containing a thermoplastic polymer per unit volume of the continuous reactor (O), leading to a decrease in the apparatus cost. In addition, bringing X to less than 6 causes the concentration of the recycled monomer to rise in the downstream section of the continuous reactor (O), thus promotes the repolymerization of the recycled monomer, makes it more important to perform temperature control on the basis of the inside temperature of the continuous reactor (O) as described in the present invention, and hence, is preferable.

Assuming that the highest temperature indicated on the device (P) for monitoring the inside temperature in the upstream section and downstream section of the continuous reactor (O) for conducting hydrolysis is Y°C, it is preferable to control the product of X and Y at 2000 or less. In this regard, the highest temperature Y°C refers to the higher temperature of the highest temperature value indicated as the inside temperature of the upstream section and the highest temperature value indicated as the inside temperature of the downstream section. The product of X and Y is more preferably conditioned to be 1600 or less, still more preferably conditioned to be 1300 or less, particularly preferably conditioned to be 1200 or less. In addition, the lower limit of the product of X and Y is not particularly limited, and is preferably 300, more preferably 320, particularly preferably 340. Bringing the product of X and Y within these ranges of condition can make it easier to achieve both the efficiency of depolymerization treatment of a composition containing a thermoplastic polymer and energy saving.

In addition, assuming that the average retention time of a mixture between a composition containing a thermoplastic polymer and subcritical water in the continuous reactor (O) is Z minute(s), it is preferable to control the product of X, Y, and Z at 60000 or less. In this regard, the average retention time is a value obtained by dividing "the capacity of the continuous reactor (O)" by "the volumetric rate of flow of a mixture between a composition containing a thermoplastic polymer and subcritical water". For example, the product of X, Y, and Z is more preferably 40000 or less, still more preferably 30000 or less. In addition, the lower limit of the product of X, Y, and Z is not particularly limited, and is preferably 5000, more preferably 8000, particularly preferably 9000. Bringing the product of X, Y, and Z within these preferable ranges of condition can make it easier to achieve both the efficiency of depolymerization treatment of a composition containing a thermoplastic polymer and energy saving.

(6) Cooler and back-pressure valve

The reaction solution discharged from the continuous reactor (O) is preferably introduced into a device for stopping hydrolysis. Examples of the device for stopping hydrolysis include coolers and flash vessels. The device for stopping hydrolysis is preferably a cooler among these. Examples of the cooler include a known means such as a heat exchanger. The reaction solution discharged from a cooler usually has a high pressure in many cases, and thus, is preferably released from pressure by a known means such as a back-pressure valve.

(7) Monomer purification apparatus

The reaction solution after the stop of reaction is preferably purified. Examples of the purification method include a known method such as distillation or crystallization.

The monomer purified is preferably repolymerized and reused as a composition containing a thermoplastic polymer. As the repolymerization method, a known polymerization method can be used.

(8) Method of producing recycled monomer (fourth preferable aspect of present invention)

A fourth preferable aspect of the present invention is a method of producing a recycled monomer, including a step of bringing a composition containing a thermoplastic polymer into contact with subcritical water to hydrolyze the thermoplastic polymer. The definitions, examples, and suitable aspects of the composition containing a thermoplastic polymer and the subcritical water are as described above. In addition, the reaction conditions for the step of hydrolysis, i.e., a suitable mixing ratio, temperature, pressure, and the like are as described above.

In the fourth preferable aspect of the present invention, the thermoplastic polymer is preferably a thermoplastic polyester or a thermoplastic polyamide. The thermoplastic polymer being a thermoplastic polyester or a thermoplastic polyamide makes it easier to recover an unpolymerized monomer through hydrolysis.

In the fourth preferable aspect of the present invention, the reaction temperature in the later stage of hydrolysis is preferably 10°C or more lower than the reaction temperature in the initial stage of hydrolysis in the step of hydrolyzing a thermoplastic polymer. The temperature is made higher in the initial stage of hydrolysis than in the later stage of hydrolysis, thus promoting the hydrolysis of the thermoplastic polymer contained in a large amount in a reaction solution in the initial stage of reaction. On the other hand, the reaction solution in the later stage of reaction contains a large amount of the recycled monomer hydrolyzed, and thus, making the temperature in the later stage lower than in the initial stage of reaction can make it easier to inhibit the repolymerization of the recycled monomer. That is, it is possible to make it easier to achieve both hydrolyzing a thermoplastic polymer with high efficiency and obtaining a recycled monomer with a high yield. The reaction temperature in the later stage of hydrolysis is more preferably 20°C or more lower, still more preferably 30°C or more lower, than the reaction temperature in the initial stage of hydrolysis. In addition, the reaction temperature in the later stage of hydrolysis with respect to the reaction temperature in the initial stage of hydrolysis is not particularly limited, and is usually 100°C or less lower, preferably 80°C or less lower, more preferably 60°C or less lower, from the viewpoint of keeping the reaction rate of the hydrolysis of a thermoplastic polymer.

Examples

The present invention will be described below with reference to Examples, but the present invention is not limited to these Examples.

The following raw materials were used in the first to fourth preferable aspects of the present invention and in Examples of an apparatus of producing a recycled monomer.

[Raw materials used in description of first preferable aspect]

Thermoplastic polyester (A-1): commercially available polyethylene terephthalate resin pellets; the melting point, 254°C

Thermoplastic polyester (A-2): PET bottle flakes washed and dried; the melting point, 255°C

Thermoplastic polyester (A-3): commercially available polybutylene terephthalate resin pellets; the melting point, 224°C

Here, the melting point was defined as the temperature of an endothermic peak that appeared when each resin molded article was cooled from a molten state to 30°C at a cooling rate of 20°C/minute, and then heated to the melting point + 40°C at a heating rate of 20°C/minute under a nitrogen gas atmosphere, using a differential scanning calorimeter. However, in a case where two or more endothermic peaks were detected, the temperature of the endothermic peak having the largest peak intensity was regarded as the melting point.

[Raw materials used in description of second preferable aspect]

PET: a polyethylene terephthalate resin (the intrinsic viscosity, 0.62 dl/g) manufactured by Toray Industries, Inc.

PBT1: a polybutylene terephthalate resin (the intrinsic viscosity, 0.85 dl/g) manufactured by Toray Industries, Inc.

PBT2: a polyester resin composition obtained by blending pentaerythritoltetrakis[3-(3,5-di-*t*-butyl-4-hydroxyphenyl) propionate] (Irganox 1010 [tradename] manufactured by BASF SE) (0.5 part by weight) with PBT1 (100 parts by weight), and melt-kneading the resulting mixture at 250°C

PBT3: a polyester resin composition obtained by blending 2,2'-methylenebis[4-(1,1,3,3-tetramethylbutyl)-6-(2*H*-benzotriazole-2-yl) phenol] (Adekastab LA-31G) (0.5 part by weight) with PBT1 (100 parts by weight), and melt-kneading the resulting mixture at 250°C

PBT4: a polyester resin composition obtained by blending 3,9-bis(2,6-di-*tert*-butyl-4-methylphenoxy)-2,4,8,10-tetraoxa-3,9-diphosphaspiro[5.5] undecane (Adekastab PEP-36) (0.5 part by weight) with PBT1 (100 parts by weight), and melt-kneading the resulting mixture at 250°C

PBT5: a polyester resin composition obtained by blending Irganox 1010 [tradename] manufactured by BASF (0.5 part by weight), Adekastab LA-31G (0.5 part by weight), and Adekastab PEP-36 (0.5 part by weight) with PBT1 (100 parts by weight), and melt-kneading the resulting mixture at 250°C

[Raw materials used in description of third preferable aspect]

Polyethylene terephthalate [PET] (manufactured by Toray Industries, Inc.; the intrinsic viscosity, 0.62 dl/g; the melting point, 254°C)

Polybutylene terephthalate [PBT] (manufactured by Toray Industries, Inc.; the intrinsic viscosity, 0.85 dl/g; the melting point, 224°C)

Polycarbonate [PC] ("TARFLON" (registered trademark) A2200, manufactured by Idemitsu Kosan Co., Ltd.; the viscosity-average molecular weight, 22000; no melting point; the glass transition temperature, 150°C)

Polyurethane [PU] (polyurethane urea composed of PTMG having a molecular weight of 1800, diphenylmethane diisocyanate, ethylenediamine, and diethyl amine as an end-capping agent; the pyrolysis start temperature, 277°C)

[Apparatus of producing recycled monomer, and raw materials used in description of fourth preferable aspect]

Polyethylene terephthalate (PET-A)]

Commercially available polyethylene terephthalate resin pellets (PET-A); the melting point, 254°C

Waste of a polyethylene terephthalate resin (PET-B): PET bottle flakes washed and dried; the melting point, 255°C

Here, the melting point was defined as the temperature of an endothermic peak that appeared when the thermoplastic polyester was cooled from a molten state to 30°C at a cooling rate of 20°C/minute, and then heated to the melting point + 40°C at a heating rate of 20°C/minute under a nitrogen gas atmosphere, using a differential scanning calorimeter. However, in a case where two or more endothermic peaks were detected, the temperature of the endothermic peak having the largest peak intensity was regarded as the melting point.

<<Evaluation methods>>

[Diol yield (GC)]

The diol yield (GC) in the present invention was calculated in accordance with a gas-chromatographic measurement. The measurement conditions are as follows.

Device: GC-2010, manufactured by Shimadzu Corporation
Column: DB-5 manufactured by Agilent Technologies, Inc.; 0.32 mm $\times$ 30 m (0.25 $\mu$m)
Carrier gas: helium; Detector: flame ionization detector (FID)
Sample: approximately 1.2 g of a reaction mixture was weighed out, diluted with approximately 5 g of methanol, and filtrated for separation and removal of a component insoluble in methanol to prepare a gas-chromatographic measurement sample.

Quantitation of diol: the amount of diol was quantitated using the absolute calibration curve method.

[Dicarboxylic acid yield (HPLC)]

The dicarboxylic acid yield (HPLC) in the present invention was calculated in accordance with a high-performance-liquid-chromatographic measurement. The measurement conditions are as follows.

Device: LC-10Avp Series, manufactured by Shimadzu Corporation
Column: Mightysil RP-18GP150-4.6
Detector: a photodiode array detector (UV = 254 nm)
Flow rate: 1 mL/min
Column temperature: 40°C
Eluent: an aqueous solution of 0.1% acetic acid/acetonitrile
Sample: approximately 0.1 g of a reaction mixture was weighed out, diluted with approximately 10 g of dimethylformamide, and filtrated for separation and removal of a component insoluble in dimethylformamide to prepare a high-performance-liquid-chromatographic measurement sample.

Quantitation of dicarboxylic acid: the amount of dicarboxylic acid was quantitated using the absolute calibration curve method (with the proviso that, in Examples 9, 12 to 25, 35, 36 and Comparative Examples 8 to 10, a disodium terephthalate salt or a diammonium terephthalate salt was generated, and converted into terephthalic acid by virtue of an acid contained in the eluent).

[Examples in first preferable aspect, and Comparative Examples]

[Example 1]

In a SUS316L autoclave including a stirrer, 20.0 g of the thermoplastic polyester (A-1) and 60.0 g of deionized water were preliminarily well mixed. The mass ratio of the water to the thermoplastic polyester (Z:1) was 3:1.
The reactor was purged with nitrogen, and hermetically sealed under a nitrogen pressure of 0.5 MPa. Then, the resulting mixture was retained with stirring at 200 rpm at 260°C for 15 minutes to react. During the reaction, the pressure in the system was 4.5 MPa. Upon completion of the reaction, the reaction mixture was cooled to room temperature, and collected. The reaction temperature X°C was 260°C; the retention time at the reaction temperature of 260°C was 15 minutes; thus, the product of X, Y, and Z was 11,700.
The diol yield calculated on the basis of the gas-chromatographic measurement of the reaction mixture collected was 95%; the dicarboxylic acid yield calculated on the basis of the high-performance-liquid-chromatographic measurement was 83%; and the diol concentration of the reaction mixture was 7.63 mass%. In addition, the reaction mixture was white.

[Examples 2 to 7 and Comparative Examples 1 to 5]

Using the thermoplastic polyester (A-1) as a raw material, depolymerization was performed using the same method as in Example 1 except that the amount of the water with respect to the thermoplastic polyester, the reaction temperature, and the reaction time were changed. The reaction conditions, the diol yields, the dicarboxylic acid yields, and the color tones of the reaction mixtures are shown in Tables 1 and 2.

[Example 8]

Depolymerization was performed using the same method as in Example 1 except that the thermoplastic polyester (A-2) was used as a raw material. The diol yields, the dicarboxylic acid yields, and the color tones of the reaction mixtures are shown in Table 1.

[Example 9]

Depolymerization was performed using the same method as in Example 1 except that sodium hydroxide was further added. The diol yields, the dicarboxylic acid yields, and the color tones of the reaction mixtures are shown in Table 1.

[Example 10]

Depolymerization was performed using the same method as in Example 2 except that the thermoplastic polyester (A-3) was used as a raw material. The diol yields, the cyclic ether yields, the dicarboxylic acid yields, and the color tones of the reaction mixtures are shown in Table 1.

[Table 1]

| | | Example 1 | Example 2 | Example 3 | Example 4 | Example 5 | Example 6 | Example 7 | Example 8 | Example 9 | Example 10 |
|---|---|---|---|---|---|---|---|---|---|---|---|
| (A-1) PET | g | 20 | 20 | 30 | 30 | 12 | 50 | 20 | 0 | 20 | 0 |
| (A-2) PET bottle flakes | g | 0 | 0 | 0 | 0 | 0 | 0 | 0 | 20 | 0 | 0 |
| (A-3) PBT | g | 0 | 0 | 0 | 0 | 0 | 0 | 0 | 0 | 0 | 20 |
| (B) water | g | 60 | 60 | 60 | 60 | 60 | 50 | 60 | 60 | 60 | 60 |
| Sodium hydroxide | g | 0 | 0 | 0 | 0 | 0 | 0 | 0 | 0 | 8.4 | 0 |
| X: reaction temperature | °C | 260 | 280 | 260 | 280 | 260 | 260 | 290 | 260 | 260 | 280 |
| Y: reaction time | minute(s) | 15 | 15 | 15 | 15 | 15 | 15 | 3 | 15 | 15 | 15 |
| Z: Weight ratio of water to thermoplastic polyester, (B)/(A) | - | 3 | 3 | 2 | 2 | 5 | 1 | 3 | 3 | 3 | 3 |
| Product of X, Y, and Z | - | 11700 | 12600 | 7800 | 8400 | 19500 | 3900 | 2610 | 11700 | 11700 | 12600 |
| Pressure in system | MPa | 4.5 | 6.0 | 4.5 | 5.9 | 4.6 | 4.0 | 7.0 | 4.5 | 4.5 | 6.5 |
| Diol concentration of reaction mixture (C) | mass% | 7.63 | 6.54 | 9.48 | 8.57 | 4.93 | 13.58 | 6.71 | 7.45 | 7.02 | 0.818 |
| Cyclic ether concentration of reaction mixture (C) | mass% | 0 | 0 | 0 | 0 | 0 | 0 | 0 | 0 | 0 | 6.63 |
| Diol yield (GC) | % | 95 | 81 | 87 | 79 | 91 | 84 | 82 | 93 | 96 | 2 |
| Cyclic ether yield (GC) | % | 0 | 0 | 0 | 0 | 0 | 0 | 0 | 0 | 0 | 81 |
| Dicarboxylic acid yield (LC) | % | 83 | 100 | 74 | 74 | 100 | 72 | 87 | 84 | 98 | 98 |
| Color tone of reaction mixture (C) | - | white | white | white | white | white | white | white | white | white | white |

EP 4 545 584 A1

**[Table 2]**

| | | Comparative Example 1 | Comparative Example 2 | Comparative Example 3 | Comparative Example 4 | Comparative Example 5 |
|---|---|---|---|---|---|---|
| (A-1) PET | g | 20 | 20 | 20 | 20 | 6 |
| (A-2) PET bottle flakes | g | 0 | 0 | 0 | 0 | 0 |
| (A-3)PBT | g | 0 | 0 | 0 | 0 | 0 |
| (B) water | g | 60 | 60 | 60 | 60 | 60 |
| Sodium hydroxide | g | 0 | 0 | 0 | 0 | 0 |
| X: reaction temperature | °C | 240 | 300 | 260 | 260 | 260 |
| Y: reaction time | minute(s) | 15 | 15 | 1 | 30 | 15 |
| Z: Weight ratio of water to thermoplastic polyester, (B)/(A) | - | 3 | 3 | 3 | 3 | 10 |
| Product of X, Y, and Z | - | 10800 | 13500 | 780 | 23400 | 39000 |
| Pressure in system | MPa | 3.5 | 8.1 | 4.5 | 4.5 | 4.6 |
| Diol concentration of reaction mixture (C) | mass% | 3.62 | 6.38 | 3.38 | 7.43 | 2.34 |
| Cyclic ether concentration of reaction mixture (C) | mass% | 0 | 0 | 0 | 0 | 0 |
| Diol yield (GC) | % | 44 | 79 | 42 | 91 | 79 |
| Cyclic ether yield (GC) | % | 0 | 0 | 0 | 0 | 0 |
| Dicarboxylic acid yield (LC) | % | 12 | 100 | 36 | 100 | 79 |
| Color tone of reaction mixture (C) | - | white | light orange | white | light orange | white |

Examples 1 to 8 have revealed that bringing X to a temperature not lower than the melting points of the thermoplastic polyester (A-1) and the thermoplastic polyester waste (A-2) and lower than 300°C, and bringing the product of X, Y, and Z to 2,000 or more and 20,000 or less made it possible to significantly restrain the amount of energy necessary for the depolymerization of the thermoplastic polyester, obtain a diol and a dicarboxylic acid with a high yield, and furthermore, to obtain the diol and the dicarboxylic acid as a reaction mixture having an increased diol concentration and not colored.

In Comparative Example 1 in which the reaction was performed at 240°C that was lower than the melting point of the thermoplastic polyester (A-1), namely 254°C, the yields of a diol and a dicarboxylic acid were markedly decreased to less than half of the yields in Example 1 in which the reaction was performed under the same conditions except the reaction temperature. This is considered to be because the depolymerization is limited to the surface of the solid thermoplastic polyester (A-1), and was insufficient. Also in Comparative Example 3 in which X and Z were in the preferable ranges, but the reaction time was short, and the product of X, Y, and Z was less than 2,000, the progress of the reaction was insufficient, and the yields of a diol and a dicarboxylic acid were decreased, compared with Example 1. In this manner, the condition where at least any one of X or the product of X, Y, and Z does not satisfy the preferable range causes the thermoplastic polyester and/or the oligomer to remain, causes the amount of a waste as a residue to increase, and thus, is not preferable.

Comparative Example 2 in which the reaction was performed at 300°C required a large amount of energy for heating water having a large specific heat capacity, but caused the diol yield to be lower than the diol yield in Example 1, and in addition, gave a reaction mixture containing a large amount of overreaction products such as coloring impurities. In Comparative Example 4 in which the product of X, Y, and Z exceeded 20,000, X and Z were within the preferable ranges, but the heating was performed for a long time, whereby a decrease in the diol yield and the coloration of the reaction mixture were presented in the same manner as in Comparative Example 2. In this manner, the condition where at least any one of X or the product of X, Y, and Z exceeded the preferably range caused the diol generated (for example, acetaldehyde, diethylene glycol, or coloring impurities in the case of ethylene glycol) to turn into overreaction products, and thus, caused the yield to decrease. In particular, acetaldehyde that is one kind of overreaction product cannot be removed only by using a method of heavy component removal by distillation, thus requires an additional treatment such as the addition of hydrogen, complicates the apparatus in a collection step, and further requires energy for the treatment. Thus, the depolymerization is desirably performed under a condition where overreaction is inhibited.

In Comparative Example 5 in which the amount of water with respect to the amount of the thermoplastic polyester (A-1) exceeded the preferable range, the yield was lower than in Example 1. Allowing the amount of water with respect to the amount of the thermoplastic polyester to exceed the preferable range increases the amount of energy for heating water having a large specific heat capacity during depolymerization, requires a larger size of apparatus for use for depolymerization, and thus, is not preferable.

Comparison between Example 1 and Example 9 has revealed that, in a case where the reaction temperature was 260°C, blending in an alkaline component allowed the yield to increase.

According to Example 10, using a polybutylene terephthalate as the thermoplastic polyester allowed a terephthalic acid to be obtained with a high yield.

[Reference Example 1]

Over a period of 4 hours, slurry of 100 parts by mass of high-purity terephthalic acid (manufactured by Mitsui Chemicals Inc.) and 45 parts by mass of ethylene glycol (manufactured by Nippon Shokubai Co., Ltd.) was supplied in installments into an esterification reactor retained at a temperature of 250°C and a pressure of $1.2 \times 10^5$ Pa, in which reactor 123 parts by mass of bis(hydroxyethyl) terephthalate preliminarily produced through ester interchange between dimethyl terephthalate (manufactured by Kanto Chemical Co., Inc.) and ethylene glycol (manufactured by Nippon Shokubai Co., Ltd.) had been preliminarily prepared. Upon completion of the supply, the resulting mixture was esterified for another 1 hour to afford an esterification product.

From the esterification product obtained, 100 parts by mass of the product was measured off, fed into a polycondensation reactor, and melted with stirring at a temperature of 250°C. To the product, tetraisopropyl titanate was added at 15 ppm in terms of titanium atoms with respect to the theoretical yield of the polyester to be obtained after polycondensation. The reaction system was gradually heated from 250°C to 285°C with stirring at 30 rpm, and the pressure was decreased to 40 Pa. The times taken to reach the final temperature and the final pressure respectively were set at 60 minutes. At a point of time when a predetermined stirring torque was achieved, the reaction system was purged with nitrogen, and brought back to an ordinary pressure. Then, the polycondensation was stopped. The product was discharged in strand form into cold water, and immediately cut to obtain polymer pellets.

[Example 11]

A reaction mixture obtained in the same manner as in Example 1 was cooled to room temperature to precipitate a regenerated terephthalic acid, which was filtrated off. On the other hand, water was removed under reduced pressure from the filtrate obtained filtrating the regenerated terephthalic acid off. Then, the resulting filtrate was distilled to obtain a regenerated ethylene glycol. A polyethylene terephthalate containing a regenerated monomer at 55 mass% was produced in the same manner as in Reference Example 1 except that the regenerated ethylene glycol and the regenerated terephthalic acid that were obtained in this manner were used as raw materials for esterification. The regenerated polyethylene terephthalate had a melting point of 256°C, and also had the same color tone as the polyethylene terephthalate obtained in Reference Example 1.

[Comparative Example 6]

A regenerated polyethylene terephthalate was produced in the same manner as in Reference Example 1 except that the regenerated terephthalic acid and the regenerated ethylene glycol that were obtained in the same manner as in Comparative Example 2 were used. The regenerated polyethylene terephthalate obtained had a melting point of 256°C, and was light orange.

Accordingly, Example 9 and Comparative Example 6 have revealed that the depolymerization according to the present invention makes it possible to obtain a diol and a dicarboxylic acid both having a high purity and not colored, and thus, can produce the same thermoplastic polyester as in a case where a commercially available diol and dicarboxylic acid are used as raw materials.

[Examples in second preferable aspect, and Comparative Examples]

[Example 12]

To a SUS316L autoclave including a stirrer, 20.0 g of a polyethylene terephthalate resin (PET), 40.0 g of deionized water, and 0.01 g of sodium hydroxide (manufactured by Fujifilm Wako Pure Chemical Corporation) were added. The reactor was purged with nitrogen, and hermetically sealed under a nitrogen pressure of 0.5 MPa. The resulting mixture was stirred at 200 rpm at 300°C for 15 minutes. During the reaction, the pressure in the system was 8.0 MPa. After the reaction, the reactant was cooled to room temperature, and collected. The reactant was filtrated, and then, the resulting solid was dried with a vacuum pump (80°C). A terephthalic acid in an amount of 16.2 g (the yield, 94%) and a benzoic acid in an amount of 0.1 g (the yield, 0.8%) were obtained. The results are shown in Table 3.

[Examples 13 and 14 and Comparative Examples 7 to 8]

A polyethylene terephthalate resin was depolymerized using exactly the same method as in Example 12 except that the amount of sodium hydroxide added was changed. The results are shown in Table 3.

[Table 3]

| | | | Example 12 | Example 13 | Example 14 | Comparative Example 7 | Comparative Example 8 |
|---|---|---|---|---|---|---|---|
| | Kind | | PET | PET | PET | PET | PET |
| Polyester resin composition | Blending amount | g | 20 | 20 | 20 | 20 | 20 |
| | | part(s) by weight | 100 | 100 | 100 | 100 | 100 |
| water | Blending amount | g | 40 | 40 | 40 | 40 | 40 |
| | | part(s) by weight | 200 | 200 | 200 | 200 | 200 |
| Sodium hydroxide | Blending amount | g | 0.01 | 0.1 | 0.5 | 0 | 0.0008 |
| | | part(s) by weight | 0.05 | 0.5 | 2.5 | 0 | 0.004 |
| Temperature | °C | | 300 | 300 | 300 | 300 | 300 |
| Pressure | MPa | | 8 | 8 | 8 | 8 | 8 |
| Terephthalic acid | yield (%) | | 94 | 96 | 91 | 69 | 70 |
| Benzoic acid | yield (%) | | 0.8 | 0.5 | 0.5 | 16 | 15 |

Comparison between Examples 12 to 14 and Comparative Examples 7 and 8 has revealed that depolymerizing PET with the addition of a specific amount of sodium hydroxide made it possible to inhibit the generation of a benzoic acid (a terephthalic acid overreaction product), and obtain a terephthalic acid with a high yield.

[Comparative Example 9]

To a SUS316L autoclave including a stirrer, 20.0 g of a polyethylene terephthalate resin (PET), 40.0 g of deionized water, and 0.36 mL of 28% ammonia water (manufactured by Fujifilm Wako Pure Chemical Corporation) were added. The reactor was purged with nitrogen, and hermetically sealed under a nitrogen pressure of 0.5 MPa. The resulting mixture was stirred at 200 rpm at 300°C for 15 minutes. During the reaction, the pressure in the system was 8.0 MPa. After the reaction, the reactant was cooled to room temperature, and collected. The reactant was filtrated, and then, the resulting solid was dried and hardened using a vacuum pump (80°C). A terephthalic acid in an amount of 10.7 g (the yield, 62%) and a benzoic acid in an amount of 2.3 g (the yield, 18%) were obtained. On the other hand, the filtrate was analyzed by GC with the result that the yield of the ethylene glycol was 78%. The results together with the results of Example 2 are shown in Table 4.

[Comparative Example 10]

To a SUS316L autoclave including a stirrer, 20.0 g of a polyethylene terephthalate resin (PET), 40.0 g of deionized water, and 10.0 g of sodium hydroxide were added. The reactor was purged with nitrogen, and hermetically sealed under a nitrogen pressure of 0.5 MPa. The resulting mixture was stirred at 200 rpm at 300°C for 15 minutes. During the reaction, the pressure in the system was 8.0 MPa. After the reaction, the reactant was cooled to room temperature, and collected. The reactant was filtrated, and then, the resulting solid was dried and hardened using a vacuum pump (80°C). A terephthalic acid in an amount of 15.6 g (the yield, 90%) and a benzoic acid in an amount of 0.11 g (the yield, 0.9%) were obtained. On the other hand, the filtrate was analyzed by GC with the result that the yield of the ethylene glycol was 52%. The results are shown in Table 4.

[Table 4]

| | | | Example 13 | Comparative Example 9 | Comparative Example 10 |
|---|---|---|---|---|---|
| Polyester resin composition | Kind | | PET | PET | PET |
| | Blending amount | g | 20 | 20 | 20 |
| | | part(s) by weight | 100 | 100 | 100 |
| Water | Blending amount | g | 40 | 40 | 40 |
| | | part(s) by weight | 200 | 200 | 200 |
| Base | Kind | | NaOH | $NH_3$ | NaOH |
| | Blending amount | g | 0.1 | 0.1 | 10 |
| | | part(s) by weight | 0.5 | 0.5 | 50 |
| Temperature | °C | | 300 | 300 | 300 |
| Pressure | MPa | | 8 | 8 | 8 |
| Terephthalic acid | yield (%) | | 96 | 62 | 78 |
| Benzoic acid | yield (%) | | 0.5 | 18 | 0.9 |
| Ethylene glycol | yield (%) | | 90 | 78 | 52 |

Example 13 and Comparative Example 9 have revealed that the formulation of addition of sodium hydroxide produced a benzoic acid with a lower yield, and produced a terephthalic acid and ethylene glycol with a higher yield, than the formulation of addition of ammonia. In addition, Example 13 and Comparative Example 10 have revealed that, in a case where the amount of sodium hydroxide added was large, the yield of each of a terephthalic acid and ethylene glycol was low.

[Example 15]

To a SUS316L autoclave including a stirrer, 20.0 g of a polybutylene terephthalate resin (PBT1), 60.0 g of deionized water, and 0.1 g of sodium hydroxide were added. The reactor was purged with nitrogen, and hermetically sealed under a nitrogen pressure of 0.5 MPa. The resulting mixture was stirred at 200 rpm at 280°C for 15 minutes. During the reaction, the pressure in the system was 6.3 MPa. After the reaction, the reactant was cooled to room temperature, and collected. The reactant was filtrated, and then, the resulting solid was dried and hardened using a vacuum pump (80°C). A terephthalic acid in an amount of 16.9 g (the yield, 98%) and a benzoic acid in an amount of 0.03 g (the yield, 0.2%) were obtained. The results are shown in Table 5.

[Examples 16 to 21 and Comparative Examples 11 to 15]

A polybutylene terephthalate resin composition was depolymerized using exactly the same method as in Example 15 except that the kind of the polybutylene terephthalate resin composition and the amount of sodium hydroxide added were changed. The results are shown in Table 5.

[Table 5]

| | | | Example 15 | Example 16 | Example 17 | Example 18 | Example 19 | Example 20 | Example 21 | Comparative Example 11 | Comparative Example 12 | Comparative Example 13 | Comparative Example 14 | Comparative Example 15 |
|---|---|---|---|---|---|---|---|---|---|---|---|---|---|---|
| Polyester resin composition | Kind | | PBT1 | PBT2 | PBT3 | PBT4 | PBT5 | PBT5 | PBT5 | PBT1 | PBT2 | PBT3 | PBT4 | PBT5 |
| | Blending amount | g | 20 | 20 | 20 | 20 | 20 | 20 | 20 | 20 | 20 | 20 | 20 | 20 |
| | | part(s) by weight | 100 | 100 | 100 | 100 | 100 | 100 | 100 | 100 | 100 | 100 | 100 | 100 |
| Water | Blending amount | g | 60 | 60 | 60 | 60 | 60 | 60 | 60 | 60 | 60 | 60 | 60 | 60 |
| | | part(s) by weight | 300 | 300 | 300 | 300 | 300 | 300 | 300 | 300 | 300 | 300 | 300 | 300 |
| Sodium hydroxide | Blending amount | g | 0.1 | 0.1 | 0.1 | 0.1 | 0.01 | 0.1 | 0.5 | 0 | 0 | 0 | 0 | 0 |
| | | part(s) by weight | 0.5 | 0.5 | 0.5 | 0.5 | 0.05 | 0.5 | 2.5 | 0 | 0 | 0 | 0 | 0 |
| Temperature | | °C | 280 | 280 | 280 | 280 | 280 | 280 | 280 | 280 | 280 | 280 | 280 | 280 |
| Pressure | | MPa | 6.3 | 6.3 | 6.3 | 6.3 | 6.3 | 6.3 | 6.3 | 6.3 | 6.3 | 6.3 | 6.3 | 6.3 |
| Terephthalic acid | | yield (%) | 98 | 97 | 98 | 97 | 98 | 99 | 95 | 94 | 91 | 92 | 91 | 89 |
| Benzoic acid | | yield (%) | 0.2 | 0.2 | 0.1 | 0.2 | 0.1 | 0.1 | 0.2 | 1.6 | 3.8 | 2.3 | 2.2 | 6.1 |

Comparative Example 11 and Comparative Examples 12 to 15 have revealed that blending an antioxidant and a weather-resistant agent with PBT increased the yield of a benzoic acid, and decreased the yield of a terephthalic acid. In addition, Comparative Examples 12 to 15 and Examples 16 to 21 have revealed that adding sodium hydroxide to PBT blended with the additives decreased the yield of a benzoic acid, and increased the yield of a terephthalic acid.

[Example 22]

To a SUS316L autoclave including a stirrer, 20.0 g of a polybutylene terephthalate resin composition (PBT5), 60.0 g of deionized water, and 0.1 g of sodium hydroxide were added. The reactor was purged with nitrogen, and hermetically sealed under a nitrogen pressure of 0.5 MPa. The resulting mixture was stirred at 200 rpm at 260°C for 15 minutes. During the reaction, the pressure in the system was 5.0 MPa. After the reaction, the reactant was cooled to room temperature, and collected. The reactant was filtrated, and then, the resulting solid was dried and hardened using a vacuum pump (80°C). A terephthalic acid in an amount of 15.6 g (the yield, 90%) and a benzoic acid in an amount of 0.03 g (the yield, 0.2%) were obtained. The results together with the results of Example 20 are shown in Table 6.

[Example 23]

A polybutylene terephthalate resin composition was depolymerized using exactly the same method as in Example 22 except that the temperature was changed. The results are shown in Table 6.

[Table 6]

| | | | Example 22 | Example 20 | Example 23 |
|---|---|---|---|---|---|
| Polyester resin composition | Kind | | PBT5 | PBT5 | PBT5 |
| | Blending amount | g | 20 | 20 | 20 |
| | | part(s) by weight | 100 | 100 | 100 |
| Water | Blending amount | g | 60 | 60 | 60 |
| | | part(s) by weight | 300 | 300 | 300 |
| Sodium hydroxide | Blending amount | g | 0.1 | 0.1 | 0.1 |
| | | part(s) by weight | 0.5 | 0.5 | 0.5 |
| Temperature | °C | | 260 | 280 | 300 |
| Pressure | MPa | | 5 | 6.3 | 8 |
| Terephthalic acid | yield (%) | | 90 | 99 | 97 |
| Benzoic acid | yield (%) | | 0.2 | 0.1 | 0.5 |

Comparison between Example 22 and Example 20 has revealed that raising the temperature from 260°C to 280°C increased the yield of a terephthalic acid. Comparison between Example 20 and Example 23 has revealed that raising the temperature from 280 to 300°C increased the yield of a benzoic acid, and decreased the yield of a terephthalic acid. Accordingly, it is understood that, when the temperature was 280°C, the yield of a terephthalic acid was the highest.

[Example 24]

To a SUS316L autoclave including a stirrer, 20.0 g of a polybutylene terephthalate resin (PBT5), 40.0 g of deionized water, and 0.1 g of sodium hydroxide were added. The reactor was purged with nitrogen, and hermetically sealed under a nitrogen pressure of 0.5 MPa. The resulting mixture was stirred at 200 rpm at 280°C for 15 minutes. During the reaction, the pressure in the system was 6.3 MPa. After the reaction, the reactant was cooled to room temperature, and collected. The reactant was filtrated, and then, the resulting solid was dried and hardened using a vacuum pump (80°C). A terephthalic acid in an amount of 14.3 g (the yield, 83%) and a benzoic acid in an amount of 0.01 g (the yield, 0.1%) were obtained. The results together with the results of Example 20 are shown in Table 7.

[Example 25]

A polybutylene terephthalate resin composition was obtained using exactly the same method as in Example 24 except that the amount of the polybutylene terephthalate resin composition blended in and the amount of water blended in were changed. The results are shown in Table 7.

[Table 7]

| | | | Example 24 | Example 20 | Example 25 |
|---|---|---|---|---|---|
| Polyester resin composition | Kind | | PBT5 | PBT5 | PBT5 |
| | Blending amount | g | 20 | 20 | 7.5 |
| | | part(s) by weight | 100 | 100 | 100 |
| Water | Blending amount | g | 40 | 60 | 60 |
| | | part(s) by weight | 200 | 300 | 800 |
| Sodium hydroxide | Blending amount | g | 0.1 | 0.1 | 0.1 |
| | | part(s) by weight | 0.5 | 0.5 | 0.5 |
| Temperature | °C | | 280 | 280 | 280 |
| Pressure | MPa | | 6.3 | 6.3 | 6.3 |
| Terephthalic acid | yield (%) | | 83 | 99 | 95 |
| Benzoic acid | yield (%) | | 0.1 | 0.1 | 0.2 |

Comparison between Example 24 and Example 20 has revealed that increasing the amount of water blended in from 200 parts by weight to 300 parts by weight increased the yield of a terephthalic acid. Comparison between Example 20 and Example 25 has revealed that increasing the amount of water blended in from 300 parts by weight to 800 parts by weight decreased the yield of a terephthalic acid. Accordingly, it is understood that, when the amount of water blended in was 300 parts by weight, the yield of a terephthalic acid was the highest.

[Examples in third preferable aspect, and Comparative Examples]

[Example 26]

In a SUS316L autoclave including a stirrer, a polyester mixture of 10.0 g of polyethylene terephthalate and 10.0 g of polybutylene terephthalate and 50.0 g of deionized water were preliminarily well mixed. The reactor was purged with nitrogen, and hermetically sealed under a nitrogen pressure of 0.3 MPa. Then, the resulting mixture was retained with stirring at 200 rpm at 260°C for 15 minutes to react. During the reaction, the pressure in the system was 4.8 MPa. Upon completion of the reaction, the reactant was cooled to room temperature, and a white precipitate component was collected through filtration. The terephthalic acid yield calculated on the basis of a high-performance-liquid-chromatographic measurement of the precipitate component collected is shown in Table 8. The terephthalic acid yield was 90%, and the ethylene glycol yield was 83%.

[Example 27]

A polyester mixture was hydrolyzed in exactly the same manner as in Example 26 except that the amount of deionized water was changed to 30.0 g. The results are shown in Table 8. The terephthalic acid yield was 88%, and the ethylene glycol yield was 82%.

[Example 28]

A polyester mixture was hydrolyzed in exactly the same manner as in Example 26 except that the amount of deionized water was changed to 100 g. The results are shown in Table 8. The terephthalic acid yield was 95%, and the ethylene glycol yield was 88%.

[Example 29]

Hydrolysis was conducted in exactly the same manner as in Example 26 except that the composition containing a polymer was changed to a polyester mixture containing 19.0 g of polyethylene terephthalate and 1.0 g of polybutylene terephthalate. The results are shown in Table 8. The terephthalic acid yield was 85%, and the ethylene glycol yield was 78%.

[Example 30]

Hydrolysis was conducted in exactly the same manner as in Example 26 except that the composition containing a polymer was changed to a polyester mixture containing 19.8 g of polyethylene terephthalate and 0.2 g of polybutylene terephthalate. The results are shown in Table 8. The terephthalic acid yield was 82%, and the ethylene glycol yield was 76%.

[Example 31]

A polyester mixture was hydrolyzed in exactly the same manner as in Example 26 except that the reaction temperature was changed to 290°C. The results are shown in Table 8. The terephthalic acid yield was 98%, and the ethylene glycol yield was 90%.

[Example 32]

A polyester mixture was hydrolyzed in exactly the same manner as in Example 26 except that the nitrogen pressure was changed to 8.5 MPa. The results are shown in Table 8. The terephthalic acid yield was 97%, and the ethylene glycol yield was 90%.

[Example 33]

Hydrolysis was conducted in exactly the same manner as in Example 26 except that the composition containing a polymer was changed to a polyester mixture containing 16.0 g of polybutylene terephthalate and 4.0 g of polycarbonate, and that the reaction temperature was changed to 250°C. The results are shown in Table 8. The terephthalic acid yield was 85%.

[Example 34]

Hydrolysis was conducted in exactly the same manner as in Example 26 except that the composition containing a polymer was changed to a polyester mixture containing 18.0 g of polyethylene terephthalate and 2.0 g of polyurethane urea. The results are shown in Table 8. The terephthalic acid yield was 84%, and the ethylene glycol yield was 74%.

[Example 35]

Hydrolysis was conducted in exactly the same manner as in Example 34 except that 7.7 g of sodium hydroxide was further added. To the hydrolysate obtained, 100 g of deionized water was added. In the resulting mixture, all the amount of disodium terephthalate generated was dissolved. This was filtrated, and then, hydrochloric acid was added to the aqueous solution to precipitate a terephthalic acid, which was collected. The results are shown in Table 8. The terephthalic acid yield was 95%, and the ethylene glycol yield was 93%.

[Example 36]

Hydrolysis was conducted in exactly the same manner as in Example 35 except that the reaction temperature was changed to 230°C. The results are shown in Table 8. The terephthalic acid yield was 92%, and the ethylene glycol yield was 90%.

[Comparative Example 16]

A polyester mixture was hydrolyzed in exactly the same manner as in Example 26 except that the reaction temperature was changed to 240°C. The results are shown in Table 9. The terephthalic acid yield was 10%, and the ethylene glycol yield was 8%.

[Comparative Example 17]

Hydrolysis was conducted in exactly the same manner as in Example 26 except that the composition containing a polymer was changed to 20.0 g of polyethylene terephthalate alone. The results are shown in Table 9. The terephthalic acid yield was 78%, and the ethylene glycol yield was 72%.

[Comparative Example 18]

Hydrolysis was conducted in exactly the same manner as in Example 26 except that the polymer was changed to 20.0 g of polyethylene terephthalate alone, and that the amount of deionized water was changed to 30 g. The results are shown in Table 9. The terephthalic acid yield was 73%, and the ethylene glycol yield was 68%.

[Comparative Example 19]

Hydrolysis was conducted in exactly the same manner as in Example 26 except that the polymer was changed to 20.0 g of polybutylene terephthalate alone, and that the reaction temperature was changed to 250°C. The results are shown in Table 9. The terephthalic acid yield was 75%.

[Comparative Example 20]

Hydrolysis was conducted in exactly the same manner as in Example 26 except that the composition containing a polymer was changed to a polyester mixture containing 19.92 g of polyethylene terephthalate and 0.08 g of polybutylene terephthalate. The results are shown in Table 9. The terephthalic acid yield was 79%, and the ethylene glycol yield was 72%.

[Table 8]

| | | Example 26 | Example 27 | Example 28 | Example 29 | Example 30 | Example 31 | Example 32 | Example 33 | Example 34 | Example 35 | Example 36 |
|---|---|---|---|---|---|---|---|---|---|---|---|---|
| Polymer | Polyester (A) | PET | PET | PET | PET | PET | PET | PET | PBT | PET | PET | PET |
| | part(s) by weight | 100 | 100 | 100 | 100 | 100 | 100 | 100 | 100 | 100 | 100 | 100 |
| | Polyester (B) | PBT | PBT | PBT | PBT | PBT | PBT | PBT | | | | |
| | part(s) by weight | 100 | 100 | 100 | 5.3 | 1.0 | 100 | 100 | | | | |
| | Another polymer | | | | | | | | PC | PU | PU | PU |
| | part(s) by weight | | | | | | | | 25 | 11 | 11 | 11 |
| Sodium hydroxide | part(s) by weight (vs 100 parts by weight of polymer) | | | | | | | | | | 39 | 39 |
| Water | part(s) by weight (vs 100 parts by weight of polymer) | 250 | 150 | 500 | 250 | 250 | 250 | 250 | 250 | 250 | 250 | 250 |
| Temperature | °C | 260 | 260 | 260 | 260 | 260 | 290 | 260 | 250 | 260 | 260 | 230 |
| Pressure | MPa | 4.8 | 4.8 | 4.8 | 4.8 | 4.8 | 7.5 | 20 | 4.1 | 4.8 | 4.8 | 4.8 |
| Difference in melting point between two polymers | °C | 30 | 30 | 30 | 30 | 30 | 30 | 30 | 74 | 23 | 23 | 23 |
| Terephthalic acid yield | % | 90 | 88 | 95 | 85 | 82 | 98 | 97 | 85 | 84 | 95 | 92 |
| Ethylene elvcol yield | % | 83 | 82 | 88 | 78 | 76 | 90 | 90 | - | 74 | 93 | 90 |

EP 4 545 584 A1

EP 4 545 584 A1

[Table 9]

| | | Comparative Example 16 | Comparative Example 17 | Comparative Example 18 | Comparative Example 19 | Comparative Example 20 |
|---|---|---|---|---|---|---|
| Polymer | Polyester (A) | PET | PET | PET | PBT | PET |
| | part(s) by weight | 100 | 100 | 100 | 100 | 100 |
| | Polyester (B) | PBT | | | | PBT |
| | part(s) by weight | 100 | | | | 0.4 |
| Water | part(s) by weight (vs 100 parts by weight of polymer) | 250 | 250 | 150 | 250 | 250 |
| Temperature | °C | 240 | 260 | 260 | 250 | 260 |
| Pressure | MPa | 3.5 | 4.8 | 4.8 | 4.1 | 4.8 |
| Difference in melting point between two polymers | °C | 30 | - | - | - | 30 |
| Terephthalic acid yield | % | 10 | 78 | 73 | 75 | 79 |
| Ethylene glycol yield | % | 8 | 72 | 68 | - | 72 |

Comparison between Examples 26, 29, and 30 and Comparative Examples 17 and comparison between Example 27 and Comparative Example 18 have revealed that simultaneously hydrolyzing PET and PBT increased the yield of the recycled monomer.

Comparison between Example 33 and Comparative Example 19 has revealed that simultaneously hydrolyzing PBT and PC increased the yield of a terephthalic acid.

Comparison between Examples 26 and 31 and Comparative Example 16 has revealed that raising the reaction temperature increased the yield of the recycled monomer.

Comparison between Examples 26 and 28 has revealed that suitably increasing the amount of water blended in in the hydrolysis increased the yield of the recycled monomer.

Comparison between Examples 26 and 32 has revealed that suitably increasing the pressure in the hydrolysis increased the yield of the recycled monomer.

Comparison between Comparative Example 17 and Comparative Example 20 has revealed that the yield of the recycled monomer in a case where the polymer mixture of 100 parts by weight of PET and 0.4 parts by weight of PBT was simultaneously hydrolyzed was substantially the same as in a case where the PET was hydrolyzed singly.

Comparison between Example 34 and Comparative Example 17 has revealed that simultaneously hydrolyzing PET and PU increased the yield of the recycled monomer.

Comparison between Example 34 and Example 35 have revealed that hydrolysis with the addition of sodium hydroxide afforded the recycled monomer with a high yield.

Comparison between Example 35 and Example 36 has revealed that blending in an alkaline component made it possible to afford a recycled monomer with a high yield, even if the reaction temperature was decreased.

[Reference Example 2]

Bis(hydroxyethyl) terephthalate preliminarily produced through ester interchange between dimethyl terephthalate (manufactured by Kanto Chemical Co., Inc.) and ethylene glycol (manufactured by Nippon Shokubai Co., Ltd.) was preliminarily prepared in an amount of 123 parts by mass. Over a period of 4 hours, slurry of 100 parts by weight of high-purity terephthalic acid (manufactured by Mitsui Chemicals Inc.) and 45 parts by weight of ethylene glycol (manufactured by Nippon Shokubai Co., Ltd.) was supplied in installments into an esterification reactor retained at a temperature of 250°C and a pressure of $1.2 \times 10^5$ Pa. Upon completion of the supply, the resulting mixture was esterified for another 1 hour to afford an esterification product.

From the esterification product obtained, 100 parts by weight of the product was measured off, fed into a polycondensation reactor, and melted with stirring at a temperature of 250°C. To the product, tetraisopropyl titanate was added at 15 ppm in terms of titanium atoms with respect to the theoretical yield of the polyester to be obtained after polycondensation. The reaction system was gradually heated from 250°C to 285°C with stirring at 30 rpm, and the pressure was decreased to 40 Pa. The times taken to reach the final temperature and the final pressure respectively were set at 60 minutes. At a point of time when a predetermined stirring torque was achieved, the reaction system was purged with nitrogen, and brought back to an ordinary pressure. Then, the polycondensation was stopped. The product was discharged in strand form into cold water, and immediately cut to obtain polymer pellets. The melting point of this polymer was 254°C.

[Example 37]

After the hydrolysis in Example 1, a terephthalic acid, monohydroxyethyl terephthalate, and a benzoic acid were detected as components precipitated at room temperature. Monohydroxyethyl terephthalate is a polymerizable precursor of polyethylene terephthalate, and thus, is utilizable as a polymerization raw material. On the other hand, a benzoic acid can inhibit polymerization as an end-capping agent during polymerization, and thus, the precipitate component was washed in hot ethanol three times to remove a benzoic acid. Using this method, a mixture of terephthalic acid and monohydroxyethyl terephthalate (at a weight ratio of 89:11) was collected.

In addition, the water-soluble component obtained through the hydrolysis in Example 26 was distilled to collect ethylene glycol.

Bis(hydroxyethyl) terephthalate preliminarily produced through ester interchange between dimethyl terephthalate (manufactured by Kanto Chemical Co., Inc.) and ethylene glycol (manufactured by Nippon Shokubai Co., Ltd.) was preliminarily prepared in an amount of 123 parts by mass. Over a period of 4 hours, slurry of 102 parts by weight of a mixture (recycled monomer) of terephthalic acid and monohydroxyethyl terephthalate collected using the above-described method and 42 parts by weight of ethylene glycol (recycled monomer) was supplied in installments into an esterification reactor retained at a temperature of 250°C and a pressure of $1.2 \times 10^5$ Pa. Upon completion of the supply, the resulting mixture was esterified for another 1 hour to afford an esterification product.

From the esterification product obtained, 100 parts by weight of the product was measured off, fed into a polycondensation reactor, and melted with stirring at a temperature of 250°C. To the product, tetraisopropyl titanate was added at 15 ppm in terms of titanium atoms with respect to the theoretical yield of the polyester to be obtained after polycondensation. The reaction system was gradually heated from 250°C to 285°C with stirring at 30 rpm, and the pressure was decreased to 40 Pa. The times taken to reach the final temperature and the final pressure respectively were set at 60 minutes. At a point of time when a predetermined stirring torque was achieved, the reaction system was purged with nitrogen, and brought back to an ordinary pressure. Then, the polycondensation was stopped. The product was discharged in strand form into cold water, and immediately cut to obtain polymer pellets. The melting point of this polymer was 254°C, the same as in Reference Example 2.

[Apparatus of producing recycled monomer, Examples in fourth preferable aspect, and Comparative Examples]

[Example 38]

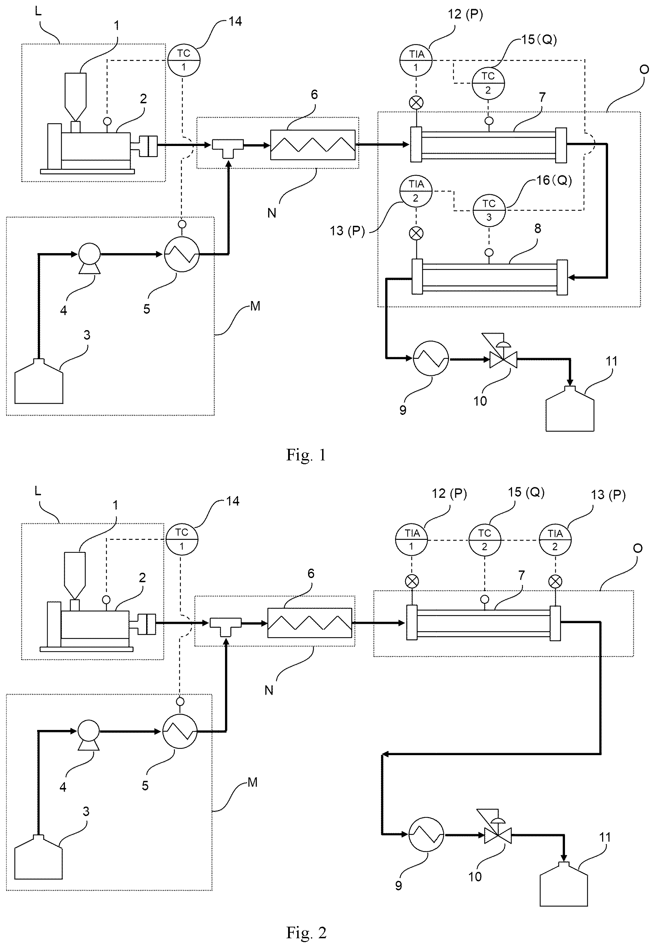

The apparatus of producing a recycled monomer, shown in FIG. 1, was used. As the means (L) for heating, pressurizing, and supplying a composition containing a thermoplastic polymer, an extruder 2 was used. As the means (M) for generating and supplying subcritical water, a high-pressure pump 4 and an electric heater 5 were used. The heating temperature of each of the extruder 2 and the electric heater 5 was controlled so as to be a preset temperature, using an extruder/electric heater temperature control device 14. As the means (N) for mixing the composition containing a thermoplastic polymer and the subcritical water supplied from the means (M), a static mixer 6 was used, in which the composition was supplied from the means (L). As a continuous reactor (O) connected to the means (N) and for conducting hydrolysis, a tubular continuous reactor 7 and a tubular continuous reactor 8 that were arranged in series, and capable of running a heating medium in the form of a counterflow into an external jacket was used.

As a raw-material composition containing a thermoplastic polymer, polyethylene terephthalate (PET-A) was introduced into a raw material hopper 1, and PET-A was melted and pressurized, using the extruder 2. With the PET-A, the temperature was 281°C, the pressure was 20 MPa, and the supply amount was 13.0 g/min. Deionized water was stored in a water tank 3, pressurized using a high-pressure pump 4, and heated using an electric heater 5, to be turned into subcritical water. With the subcritical water, the temperature was 281°C, the pressure was 20 MPa, and the supply amount was 39.0 g/min. The mass flow ratio (X:1) of the subcritical water to the thermoplastic polymer component in the composition containing the thermoplastic polymer was 3:1. The composition containing a thermoplastic polymer, heated and melted under pressure, was allowed to join the flow of the subcritical water, and supplied into a static mixer 6 having 24 elements. The solution discharged from the static mixer 6 was supplied into a tubular continuous reactor 7 having an inner diameter of 2.3 cm and a length of 250 cm. The solution discharged from a tubular continuous reactor 7 was supplied into a tubular continuous reactor 8 having an inner diameter of 2.3 cm and a length of 250 cm.

At a position 5 cm from the inlet of the tubular continuous reactor 7, a continuous-reactor-upstream-section temperature monitoring device 12 for monitoring the temperature in the upstream section of the continuous reactor (O) was inserted. At a position 5 cm from the outlet of the tubular continuous reactor 8, a continuous-reactor-downstream-section temperature monitoring device 13 for monitoring the temperature in the downstream section of the continuous reactor (O) was inserted. On the basis of a signal from the continuous-reactor-upstream-section temperature monitoring device 12, the flow rate and temperature of the heating medium supplied into the jacket of the tubular continuous reactor 7 were adjusted by the continuous reactor temperature control mechanism 15 of the tubular continuous reactor 7. On the basis of a signal from each of the continuous-reactor-upstream-section temperature monitoring device 12 and the continuous-reactor-downstream-section temperature monitoring device 13, the flow rate and temperature of the heating medium supplied into the jacket of the tubular continuous reactor 8 were adjusted by the continuous reactor temperature control mechanism 16 of the tubular continuous reactor 8. The temperature of the continuous-reactor-upstream-section temperature monitoring device 12 became 280°C, and the temperature of the continuous-reactor-downstream-section temperature monitoring device 13 became 240°C.

In addition, the average retention time Z of the continuous reactor (O) was 30 minutes. The product of X and Y and the product of X, Y, and Z were as in Table 10.

The reaction solution discharged from the tubular continuous reactor 8 was heat-exchanged with a cooling water by a heat exchanger 9 to be cooled to 40°C. Then, the reaction solution was released from a pressure of 20 MPa to the atmospheric pressure by a back-pressure valve 10, and stored in a reaction solution tank 11.

The reaction solution stored in the reaction solution tank 11 was cooled to room temperature, filtrated by suction to collect a solid component (terephthalic acid), and separated from the solution (an aqueous solution of ethylene glycol). From the weight of the solid component and the HPLC analysis results, the molar yield of the terephthalic acid was found to be 98%. On the other hand, from the weight of the solution and the GC analysis results, the molar yield of ethylene glycol was found to be 80%.

[Table 10]

| | | Example 38 | Example 39 | Example 40 | Example 41 | Example 42 | Example 43 |
|---|---|---|---|---|---|---|---|
| Apparatus | | FIG. 1 | FIG. 1 | FIG. 1 | FIG. 2 | FIG. 2 | FIG. 1 |
| Composition containing thermoplastic polymer | | PET-A | PET-A | PET-A | PET-A | PET-A | PET-B |
| Flow rate of composition containing thermoplastic polymer | g/min | 13.0 | 13.0 | 13.0 | 13.0 | 6.5 | 13.0 |
| Flow rate of subcritical water | g/min | 39.0 | 39.0 | 39.0 | 39.0 | 19.5 | 39.0 |
| Mass flow ratio of subcritical water to thermoplastic polymer component in composition, (X:1) | - | 3:1 | 3:1 | 3:1 | 3:1 | 3:1 | 3:1 |
| Inside temperature of upstream section of continuous reactor, (12) | °C | 280 | 280 | 280 | 280 | 277 | 280 |
| Inside temperature of downstream section of continuous reactor, (13) | °C | 240 | 260 | 220 | 280 | 240 | 240 |
| Average retention time (Z) | min | 30 | 30 | 30 | 15 | 30 | 30 |
| Pressure in continuous reactor | MPa | 20 | 20 | 20 | 20 | 20 | 20 |
| Product of X and Y | - | 840 | 840 | 840 | 840 | 831 | 840 |
| Product of X, Y, and Z | - | 25200 | 25200 | 25200 | 12600 | 24930 | 25200 |
| Terephthalic acid, molar yield | % | 98 | 92 | 90 | 85 | 93 | 97 |

(continued)

|  |  | Example 38 | Example 39 | Example 40 | Example 41 | Example 42 | Example 43 |
|---|---|---|---|---|---|---|---|
| Ethylene glycol, molar yield | % | 80 | 80 | 78 | 75 | 78 | 79 |

[Example 39]

An apparatus of producing a recycled monomer was operated in the same manner as in Example 38 except the setting of the temperature continuous-reactor-downstream-section temperature monitoring device 13. The results are as shown in Table 10.

[Example 40]

An apparatus of producing a recycled monomer was operated in the same manner as in Example 38 except the setting of the temperature continuous-reactor-downstream-section temperature monitoring device 13. The results are as shown in Table 10.

[Example 41]

The apparatus of producing a recycled monomer, shown in FIG. 2, was used. The apparatus shown in FIG. 2, compared with the apparatus shown in FIG. 1, is characterized by having one tubular continuous reactor 7 and one continuous reactor temperature control mechanism 15. At a position 5 cm from the inlet of the tubular continuous reactor 7, a continuous-reactor-upstream-section temperature monitoring device 12 for monitoring the temperature in the upstream section of the continuous reactor (O) was inserted. At a position 5 cm from the outlet of the tubular continuous reactor 7, a continuous-reactor-downstream-section temperature monitoring device 13 for monitoring the temperature in the downstream section of the continuous reactor (O) was inserted. Using the apparatus shown in FIG. 2, the continuous reactor temperature control mechanism 15 was set so as to keep the inside temperature of each of the upstream section and downstream section of the tubular continuous reactor 7 at 280°C, thereby controlling the flow rate and temperature of the heating medium to be run into the tubular continuous reactor 8. The other conditions were set as shown in Table 10, and the apparatus of producing a recycled monomer, the apparatus shown in FIG. 2, was operated, bringing about the results as shown in Table 10. An inference is made as follows: operation with the temperature kept at 280°C caused hydrolysis to progress promptly, and thus enabled the treatment to be conducted in a shorter retention time than in Example 38, but it was difficult to inhibit the repolymerization of a terephthalic acid in particular in the later stage of reaction, and hence, the molar yield of the terephthalic acid was decreased. In this regard, the molar yield of ethylene glycol was approximately the same as in other Examples.

[Example 42]

The apparatus of producing a recycled monomer, shown in FIG. 2, was used. With the apparatus shown in FIG. 2, the temperature in the upstream section of the tubular continuous reactor 7 was kept at approximately 280°C, and the continuous reactor temperature control mechanism 15 was set in such a manner that the temperature in the downstream section of the tubular continuous reactor would become 240°C. When this was performed, control was performed so as to decrease the flow rate of the heating medium in such a manner that a temperature gradient would be generated in tubular continuous reactor 8. The other conditions were set as shown in Table 10, and the apparatus of producing a recycled monomer, the apparatus shown in FIG. 2, was operated, bringing about the results as shown in Table 10. It is estimated that having one temperature control mechanism shortens the highest temperature keeping time, thus the molar yield of the terephthalic acid to be decreased, compared with Example 38.

[Example 43]

An apparatus of producing a recycled monomer was operated in the same manner as in Example 38 except the composition containing a thermoplastic polymer was changed to PET-B. The results are as shown in Table 10. It has been revealed that using a waste of a polyethylene terephthalate resin as a target composition containing a thermoplastic polymer made it possible to produce a recycled monomer in the same manner as in Example 38.

Reference Signs List

1 Raw material hopper
2 Extruder
3 Water tank
4 High-pressure pump
5 Electric heater
6 Static mixer
7 Tubular continuous reactor
8 Tubular continuous reactor
9 Heat exchanger
10 Back-pressure valve
11 Reaction solution tank
12 Continuous-reactor-upstream-section temperature monitoring device
13 Continuous-reactor-downstream-section temperature monitoring device
14 Extruder/electric heater temperature control device
15 Continuous reactor temperature control mechanism
16 Continuous reactor temperature control mechanism

**Claims**

1. A recycled monomer produced by bringing a composition containing a polymer into contact with subcritical water, wherein the recycled monomer is produced under a condition where X and X·Y·Z which is the product of X, Y, and Z satisfy the following (I) and (II) simultaneously, assuming that the melting point of the polymer in the composition is Mp °C, as measured using a differential scanning calorimeter, that a reaction temperature is X°C, that a reaction time is Y minute(s), and that the mass ratio of the water to the polymer is Z:1;

$$\text{(I) } Mp \leq X < 300$$

$$\text{(II) } 2000 \leq X \cdot Y \cdot Z \leq 20000.$$

2. The recycled monomer according to claim 1, wherein the polymer is a thermoplastic polyester or a thermoplastic polyamide.

3. A recycled polymer obtained by repolymerizing the recycled monomer according to claim 1.

4. A clothing fiber, industrial fiber, film, sheet, automobile component, or electrical and electronic component produced using the recycled polymer according to claim 3.

5. A method of producing a recycled monomer, comprising a step of bringing a composition containing a polymer into contact with subcritical water.

6. The method of producing a recycled monomer according to claim 5, wherein the polymer is a thermoplastic polyester (A), and wherein X and X·Y·Z which is the product of X, Y, and Z satisfy the following (I) and (II) simultaneously, assuming that the melting point of the thermoplastic polyester (A) is Mp°C, as measured using a differential scanning calorimeter, that a reaction temperature is X°C, that a reaction time is Y minute(s), and that the mass ratio of the water to the thermoplastic polyester (A) is Z:1;

$$\text{(I) } Mp \leq X < 300$$

$$\text{(II) } 2000 \leq X \cdot Y \cdot Z \leq 20000$$

7. The method of producing a recycled monomer according to claim 6, wherein the concentration of a diol in a reaction mixture obtained by bringing the composition containing the thermoplastic polyester (A) into contact with the subcritical water to hydrolyze the thermoplastic polyester (A) is 4.9 mass% or more, wherein the diol is derived from the thermoplastic polyester (A).

8. The method of producing a recycled monomer according to claim 6, wherein the concentration of a cyclic ether derived from the thermoplastic polyester (A) in a reaction mixture obtained by bringing the composition containing the thermoplastic polyester (A) into contact with the subcritical water to hydrolyze the thermoplastic polyester (A) is 3.0 mass% or more.

9. The method of producing a recycled monomer according to claim 6, wherein the mass ratio Z of the subcritical water to the thermoplastic polyester (A) is less than 6.

10. The method of producing a recycled monomer according to claim 6, wherein the thermoplastic polyester (A) comprises, as a main component, at least one selected from polyethylene terephthalate, polypropylene terephthalate, polybutylene terephthalate, and copolymers thereof.

11. The method of producing a recycled monomer according to claim 6, wherein the thermoplastic polyester (A) is a waste containing at least a polyester.

12. The method of producing a recycled monomer according to claim 6, wherein the recycled monomer is a dicarboxylic acid and/or a diol.

13. A method of producing a thermoplastic polyester using, as a raw material, the dicarboxylic acid and/or the diol obtained using the producing method according to claim 12.

14. A method of producing a thermoplastic polyester product, wherein the thermoplastic polyester obtained using the producing method according to claim 12 is used as a raw material to produce a product containing the thermoplastic polyester in the form of a fiber, a film, or a molded article.

15. A method of producing a product of a composition containing a thermoplastic polyester, wherein the composition containing a thermoplastic polyester, obtained using the producing method according to claim 12, is used as a raw material to produce a product of the composition containing a thermoplastic polyester in the form of a fiber, a film, or a molded article.

16. The method of producing a recycled monomer according to claim 5, wherein the polymer is the thermoplastic polyester (A), and wherein 0.01 to 4.0 parts by weight of an alkali metal salt and/or an alkaline earth metal salt is blended with 100 parts by weight of the thermoplastic polyester (A).

17. The method of producing a recycled monomer according to claim 16, wherein 100 to 1000 parts by weight of the subcritical water is blended with 100 parts by weight of the thermoplastic polyester (A).

18. The method of producing a recycled monomer according to claim 16, wherein a temperature in the step of bringing about contact with the subcritical water is 250 to 350°C.

19. The method of producing a recycled monomer according to claim 16, wherein a pressure in the step of bringing about contact with the subcritical water is 3 to 30 MPa.

20. The method of producing a recycled monomer according to claim 16, wherein the alkaline metal salt and/or the alkaline earth metal salt is/are a metal hydroxide and/or a metal carbonate.

21. The method of producing a recycled monomer according to claim 16, wherein the thermoplastic polyester (A) is at least one selected from polyethylene terephthalate, polypropylene terephthalate, polybutylene terephthalate, and copolymers thereof.

22. The method of producing a recycled monomer according to claim 16, wherein the composition containing the thermoplastic polyester (A) comprises at least one selected from antioxidants, heat-resistant agents, weather-resistant agents, mold release agents, and nucleating agents.

**23.** The method of producing a recycled monomer according to claim 16, wherein the composition containing the thermoplastic polyester (A) is a waste.

**24.** The method of producing a recycled monomer according to claim 16, wherein the recycled monomer is a dicarboxylic acid and/or a diol.

**25.** A method of producing a thermoplastic polyester using, as a raw material, a dicarboxylic acid and/or a diol obtained using the producing method according to claim 24.

**26.** A method of producing a composition containing a thermoplastic polyester, wherein at least one selected from antioxidants, weather-resistant agents, mold release agents, and nucleating agents is blended with the thermoplastic polyester obtained using the producing method according to claim 25.

**27.** A method of producing a thermoplastic polyester product, wherein the thermoplastic polyester obtained using the producing method according to claim 25 is used as a raw material to produce a product containing the thermoplastic polyester in the form of a fiber, a film, or a molded article.

**28.** A method of producing a product of a composition containing a thermoplastic polyester, wherein the composition containing a thermoplastic polyester, obtained using the producing method according to claim 26, is used as a raw material to produce a product of the composition containing a thermoplastic polyester in the form of a fiber, a film, or a molded article.

**29.** The method of producing a recycled monomer according to claim 5,

wherein the composition containing a polymer comprises a polymer I and a polymer II,
wherein the polymer I is a thermoplastic polyester (A),
wherein the polymer II is a polymer selected from the group consisting of a thermoplastic polyester (B) having a melting point different from the melting point of the thermoplastic polyester (A), a polycarbonate, and a polyurethane, or a combination of these polymers,
wherein the amount of the polymer II contained in the composition (the total amount of the thermoplastic polyester (B), the polycarbonate, and the polyurethane) is 1 to 100 parts by weight with respect to 100 parts by weight of the polymer I, and
wherein the method includes a step of bringing the polymer I and the polymer II in the composition simultaneously into contact with the subcritical water of 250 to 350°C.

**30.** The method of producing a recycled monomer according to claim 5,

wherein the composition containing a polymer comprises a polymer I and a polymer II,
wherein the polymer I is a thermoplastic polyester (A),
wherein the polymer II is a polymer selected from the group consisting of a thermoplastic polyester (B) having a melting point different from the melting point of the thermoplastic polyester (A), a polycarbonate, and a polyurethane, or a combination of these polymers,
wherein the amount of the polymer II contained in the composition (the total amount of the thermoplastic polyester (B), the polycarbonate, and the polyurethane) is 1 to 100 parts by weight with respect to 100 parts by weight of the polymer I, and
wherein the method includes a step of bringing the polymer I and the polymer II in the composition into contact with the subcritical water of 200 to 350°C to hydrolyze the polymer I and the polymer II simultaneously, under a condition where an alkaline component is blended.

**31.** The method of producing a recycled monomer according to claim 29 or 30, wherein the composition containing a polymer comprises the composition containing the polymer I and the polymer II and/or comprises a mixture of a composition containing the polymer I and a composition containing the polymer II.

**32.** The method of producing a recycled monomer according to claim 29 or 30, wherein 100 to 900 parts by weight of water is blended with 100 parts by weight of the polymer component in the composition containing the polymer.

**33.** The method of producing a recycled monomer according to claim 29 or 30, wherein a pressure in the step of bringing about contact with the subcritical water is 4.0 MPa to 30 MPa.

34. The method of producing a recycled monomer according to claim 29, wherein an alkaline component is further blended with the composition containing a polymer.

35. The method of producing a recycled monomer according to claim 29 or 30, wherein the thermoplastic polyester (A) is selected from polyethylene terephthalate, polypropylene terephthalate, polybutylene terephthalate, and copolymers thereof.

36. The method of producing a recycled monomer according to claim 29 or 30, wherein the thermoplastic polyester (B) is at least one selected from polyethylene terephthalate, polypropylene terephthalate, polybutylene terephthalate, and copolymers thereof, and has a melting point different from the melting point of the polyester (A).

37. The method of producing a recycled monomer according to claim 29 or 30, wherein a difference in the melting point between the thermoplastic polyester (A) and the thermoplastic polyester (B) is 10°C or more.

38. The method of producing a recycled monomer according to claim 29 or 30, wherein the composition containing a polymer is a waste.

39. The method of producing a recycled monomer according to claim 29 or 30, wherein the recycled monomer is a terephthalic acid and/or a diol.

40. A method of producing a thermoplastic polyester, wherein the dicarboxylic acid and/or the diol obtained using the producing method according to claim 29 or 30 is/are used as a polymerization raw material to produce the thermoplastic polyester.

41. A method of producing a thermoplastic polyester product, wherein the thermoplastic polyester obtained using the producing method according to claim 40 is used as a raw material to produce a thermoplastic polyester product in the form of a fiber, a film, or a molded article.

42. A method of producing a product of a composition containing a thermoplastic polyester, wherein the composition containing a thermoplastic polyester, obtained using the producing method according to claim 40, is used as a raw material to produce a product of the composition containing a thermoplastic polyester in the form of a fiber, a film, or a molded article.

43. An apparatus of producing a recycled monomer, comprising: means (L) for heating, pressurizing, and supplying a composition containing a polymer; means (M) for generating and supplying subcritical water; means (N) for mixing the composition containing a thermoplastic polymer and supplied from the means (L) and the subcritical water supplied from the means (M); and a continuous reactor (O) for conducting hydrolysis of the polymer.

44. The apparatus of producing a recycled monomer according to claim 43, comprising: a device (P) for monitoring the inside temperature of each of the upstream section and downstream section of the continuous reactor (O); and a temperature control mechanism (Q) for heating and/or cooling the continuous reactor (O);
wherein the temperature control mechanism (Q) is operated in accordance with the device (P) to control the inside temperature of the continuous reactor (O).

45. The apparatus of producing a recycled monomer according to claim 43, comprising a plurality of temperature control mechanisms (Q) that are installed, wherein the plurality of temperature control mechanisms (Q) are operated in accordance with the device (P).

46. The apparatus of producing produce a recycled monomer according to claim 44 or 45, wherein the temperature control mechanism (Q) is controlled in such a manner that the inside temperature of the downstream section of the continuous reactor (O) is 10°C or more lower than the inside temperature of the upstream section.

47. The apparatus of producing a recycled monomer according to claim 44 or 45, wherein the continuous reactor (O) is a tubular continuous reactor.

48. The apparatus of producing a recycled monomer according to claim 44 or 45, wherein the means (L) is an extruder.

49. The apparatus of producing a recycled monomer according to claim 44 or 45, wherein the means (N) is a static mixer.

50. The apparatus of producing a recycled monomer according to claim 44 or 45, wherein the composition containing a polymer is a waste of a resin molded article.

51. The apparatus of producing a recycled monomer according to claim 44 or 45, wherein the polymer is a thermoplastic polyamide or a thermoplastic polyester.

Fig. 1

Fig. 2

## INTERNATIONAL SEARCH REPORT

| International application No. |
| --- |
| **PCT/JP2023/022870** |

**A. CLASSIFICATION OF SUBJECT MATTER**

*C08G 63/88*(2006.01)i; *C07B 61/00*(2006.01)i; *C07C 27/02*(2006.01)i; *C07C 29/09*(2006.01)i; *C07C 31/20*(2006.01)i; *C07C 51/09*(2006.01)i; *C07C 63/26*(2006.01)i; *C08G 63/16*(2006.01)i; *C08G 63/183*(2006.01)i; *C08J 11/14*(2006.01)i; *C08J 11/16*(2006.01)i; *C08K 3/22*(2006.01)i; *C08K 3/26*(2006.01)i; *C08L 67/02*(2006.01)i

FI: C08G63/88; C07B61/00 300; C07C27/02 ZAB; C07C29/09; C07C31/20 A; C07C31/20 B; C07C51/09; C07C63/26; C07C63/26 A; C07C63/26 Z; C08G63/16; C08G63/183; C08J11/14 ZAB; C08J11/16; C08K3/22; C08K3/26; C08L67/02

According to International Patent Classification (IPC) or to both national classification and IPC

**B. FIELDS SEARCHED**

Minimum documentation searched (classification system followed by classification symbols)

C08G63/88; C07B61/00; C07C27/02; C07C29/09; C07C31/20; C07C51/09; C07C63/26; C08G63/16; C08G63/183; C08J11/14; C08J11/16; C08K3/22; C08K3/26; C08L67/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 09-077905 A (KOBE STEEL, LTD.) 25 March 1997 (1997-03-25) claims, paragraphs [0001], [0010], [0015], [0026], [0034], [0038] | 1-28, 43-51 |
| A | | 29-42 |
| X | JP 2021-130766 A (TOPPAN PRINTING CO., LTD.) 09 September 2021 (2021-09-09) claims | 5 |
| A | | 1-4, 6-51 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| **30 August 2023** | **12 September 2023** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

## INTERNATIONAL SEARCH REPORT
### Information on patent family members

International application No.

**PCT/JP2023/022870**

| Patent document cited in search report | | | Publication date (day/month/year) | Patent family member(s) | Publication date (day/month/year) |
|---|---|---|---|---|---|
| JP | 09-077905 | A | 25 March 1997 | (Family: none) | |
| JP | 2021-130766 | A | 09 September 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000309663 A
- JP 2007332361 A
- JP 6072922 A
- JP 11502870 A
- JP 8302061 A
- WO 2007148353 A1
- WO 2004041917 A1

- US 4605762 B
- JP 2012106244 A
- JP 9077905 A
- JP 2615131 B
- JP 61026612 A
- JP 2289516 B

**Non-patent literature cited in the description**

- *Waste Management*, 2017, vol. 68, 24-31